# FASCICULE DE BREVET EUROPEEN

(11) **EP 1 828 240 B1**
(45) Date de publication et mention de la délivrance du brevet: **29.07.2009**
(21) Numéro de dépôt: 05826561.2
(22) Date de dépôt: 15.12.2005
(51) Int. Cl.: C07K 14/525, C07K 14/705, A61K 38/04

(54) **NOUVEAUX LIGANDS MULTIMERIQUES DE CD40, LEUR PROCEDE DE PREPARATION ET LEUR UTILISATION POUR LA PREPARATION DE MEDICAMENTS**
NEUE MULTIMERE CD40-LIGANDEN, VERFAHREN ZU IHRER HERSTELLUNG UND IHRE VERWENDUNG ZUR HERSTELLUNG VON ARZNEIMITTELN
NOVEL MULTIMERIC CD40 LIGANDS, METHOD FOR PREPARING SAME AND USE THEREOF FOR PREPARING DRUGS

(30) Priorité: 15.12.2004 FR 0413331
(43) Date de publication de la demande: 05.09.2007
(73) Titulaire: Centre National de la Recherche Scientifique, 75794 Paris Cedex 16 (FR)
(72) Inventeur: GUICHARD, Gilles, F-67202 Wolfisheim (FR); FOURNEL, Sylvie, Victorine, Lucienne, F-67200 Strasbourg (FR); CHALOIN, Olivier, F-67200 Strasbourg (FR); TROUCHE, Nathalie, F-67150 KRAFFT (FR); WIECKOWSKI, Sébastien, F-74100 Ambilly (FR); HOEBEKE, Johan, B-3010 Kessel-Lo (BE)
(74) Mandataire: Grosset-Fournier, Chantal Catherine
(86) Numéro de dépôt international: PCT/FR2005/003146
(87) Numéro de publication internationale: WO 2006/064133

(56) Documents cités:
- WO-A-01/49866
- WO-A-02/00893
- WO-A-99/52877
- WO-A-03/102207
- SEMINARS IN IMMUNOLOGY., vol. 6, 1994, pages 295-301, XP002237774 US W.B. SAUNDERS COMPANY, PA.
- L K KIESSLING ET AL.: "Synthetic multivalent ligands in the exploration of cell-surface interactions" CURRENT OPINION IN CHEMICAL BIOLOGY, vol. 4, 2000, pages 696-703, XP002382114 GB CURRENT BIOLOGY LTD, LONDON
- S FOURNEL ET AL.: "C3-symmetric peptide scaffolds are functional mimetics of trimeric CD40L" NATURE CHEMICAL BIOLOGY, vol. 1, no. 7, décembre 2005 (2005-12), pages 377-382, XP002382115 US NATURE PUBLISHING GROUP, NEW YORK, NY

## Description

La présente invention a pour objet de nouveaux ligands multimériques de CD40, leur procédé de préparation, ainsi que leur utilisation pour la préparation de médicaments.

L'invention a également pour objet des molécules capables d'activer ou d'inhiber la réponse immunitaire.

L'importance du couple CD40/CD40L dans la réponse immunitaire a amené de nombreux groupes à utiliser des anticorps dirigés contre ces deux molécules à des fins thérapeutiques, de manière à inhiber ou activer le système immunitaire. L'administration d'anticorps anti-CD40L a donné des résultats encourageants dans le traitement de maladies auto-immunes comme l'encéphalomyélite allergique expérimentale murine (un modèle de la sclérose en plaques humaine)(Howard et al., 1999) ou dans le traitement de rejet d'allogreffes rénales chez les singes (Kirk et al., 1999). Dans ces deux cas, les anticorps ont inhibé une activité néfaste du système immunitaire. Inversement, l'utilisation d'anticorps anti-CD40 agonistes a permis d'une part, d'améliorer fortement la réponse à des vaccins anti-tumoraux peptidiques chez la souris (Diehl et al., 1999) et d'autre part, d'augmenter l'efficacité des cellules T CD4⁺ dans la lutte contre des tumeurs murines (Sotomayor et al., 1999 ; Lode et al., 2000). Une régression de tumeur dans des modèles murins a été mise en évidence après injection de cellules dendritiques (CDs) transformées par un adénovirus codant le CD40L (Kikuchi et al., 2000). Enfin, une activation des cellules dendritiques par l'interaction de leur molécule CD40 avec CD40L est capable de protéger des souris d'une infection par un parasite, *Trypanosoma Cruzi* (Chaussabel et al., 1999). Dans tous ces travaux, la valence particulière de la molécule CD40L, qui s'associe sous forme de trimère pour former avec le CD40 des complexes hexavalents, rend difficile la production d'anticorps fonctionnels capables d'interférer avec les fonctions du couple CD40/CD40L. Le développement des adénovirus codant pour CD40L répond en partie à cet inconvénient. Cependant, leur utilisation n'est pas sans poser de problèmes chez l'homme. Enfin, la valence particulière du système rend difficile la découverte de molécules de synthèse capables d'interférer avec l'interaction CD40/CD40L. A ce titre, il convient de noter les travaux décrivant des molécules multimériques capables d'agir sur des récepteurs et notamment sur le système CD40/CD40L (Guichard et al. WO-A-2003102207) (Rosenberg et al. WO-A-9952877) (Kiessling et al, Curr. Opin. Chem. Biol., 2000, 4, 696-703).

L'invention a pour but de fournir des ligands multimériques conçus pour interférer dans des interactions protéine-protéine.

La présente invention a également pour but la préparation de molécules pouvant interférer avec des interactions multivalentes protéines-protéines.

La présente invention a pour but de fournir une molécule de synthèse agissant sur le système CD40/CD40L, en fournissant de nouveaux ligands trimériques de CD40.

La présente invention a également pour but de fournir des molécules pouvant agir comme adjuvants ou immunosuppresseurs.

La présente invention concerne des composés répondant à la formule suivante (I) : 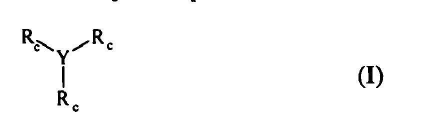 dans laquelle :
- Y représente un macrocycle dont le cycle comprend de 9 à 36 atomes, et est fonctionnalisé par trois fonctions amines ou COOH,
- R_{c} représente un groupe de formule H-Xₐ-X_{b}-X_{c}-X_{d}-Xₑ-(X_{f})ᵢ- ou H-X'ₐL-X'_{b}-X_{c}-X_{d}-Xₑ(X_{f})ᵢ-, dans laquelle :
   - i représente 0 ou 1,
   - Xₐ est choisi parmi les résidus d'acides aminés suivants :
      - lysine ;
      - arginine ;
      - ornithine ;
      - les β-acides aminés correspondant à la lysine, l'arginine ou l'ornithine, portant la substitution en position α ou β ;
      - acide tranéxamique ;
      - acide N-méthyl-tranéxamique ;
      - acide 8-amino-3,6-dioxaoctanoïque ;
      - acide 4(pipéridin-4-yl)butanoïque ;
      - acide 3(pipéridin-4-yl)propionique ;
      - N-(4-aminobutyl)-glycine ;
      - NH₂-(CH₂)ₙ-COOH, n variant de 1 à 10 ;
      - NH₂-(CH₂-CH₂-O)ₘ-CH₂CH₂COOH, m variant de 3 à 6 ;
      - 4-carboxyméthyl-pipérazine ;
      - acide 4-(4-aminophényl)butanoïque ;
      - acide 3-(4-aminophényl)propanoïque ;
      - acide 4-aminophénylacétique ;
      - 4-(2aminoéthyl)-1-carboxyméthyl-pipérazine ;
      - acide trans-4-aminocyclohexanecarboxylique ;
      - acide cis-4-aminocyclohexanecarboxylique ;
      - acide cis-4-aminocyclohexane acétique ;
      - acide trans-4-aminocyclohexane acétique ;
      - 4-amino-1-carboxyméthyl pipéridine ;
      - acide 4-aminobenzoïque ;
      - acide 4(2-aminoéthoxy)benzoïque ;
   - X_{b} est choisi parmi les résidus d'acides aminés suivants :
      - D-alanine ;
      - D-valine ;
      - L-proline substituée en position β, γ ou δ ;
      - D-proline substituée ou non en position β, γ ou δ ;
      - acides aminés naturels N-alkylés, le groupe alkyle étant un groupe méthyle, éthyle ou benzyle ;
      - acides aminés dialkylés acycliques de formule suivante : 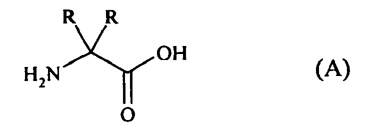 R représentant H, Me, Et, Pr ou Bu ;
      - acides aminés dialkylés cycliques de formule suivante : 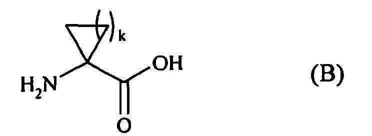 k représentant 1, 2, 3 ou 4 ;
   - X_{c} est choisi parmi les résidus d'acides aminés suivants :
      - tyrosine ;
      - phénylalanine ;
      - 3-nitro-tyrosine ;
      - 4-hydroxyméthyl-phénylalanine ;
      - 3,5-dihydroxy-phénylalanine ;
      - 2,6-diméthyl-tyrosine ;
      - 3,4-dihydroxy-phénylalanine (DOPA) ;
   - X_{d} est choisi parmi les résidus d'acides aminés suivants :
      - tyrosine ;
      - phénylalanine ;
      - 3-nitro-tyrosine ;
      - 4-hydroxyméthyl-phénylalanine ;
      - 3,5-dihydroxy-phénylalanine ;
      - 2,6-diméthyl-tyrosine ;
      - 3,4-dihydroxy-phénylalanine ;
   - Xₑ est choisi parmi les résidus d'acides aminés suivants :
      - NH₂-(CH₂)ₙ-COOH, n variant de 2 à 10 ;
      - NH₂-(CH₂-CH₂-O)ₘ-CH₂CH₂COOH, m variant de 3 à 6 ;
      - acide 8-amino-3,6-dioxaoctanoïque ;
      - acide tranéxamique ;
      - acide N-méthyl-tranéxamique ;
      - acide 4(pipéridin-4-yl)butanoïque ;
      - acide 3(pipéridin-4-yl)propionique ;
      - N-(4-aminobutyl)-glycine ;
      - 4-carboxyméthyl-pipérazine ;
      - acide 4-(4-aminophényl)butanoïque ;
      - acide 3-(4-aminophényl)propanoïque ;
      - acide 4-aminophénylacétique ;
      - 4-(2aminoéthyl)-1-carboxyméthyl-pipérazine ;
      - acide trans-4-aminocyclohexanecarboxylique ;
      - acide cis-4-aminocyclohexanecarboxylique ;
      - acide cis-4-aminocyclohexane acétique ;
      - acide trans-4-aminocyclohexane acétique ;
      - 4-amino-1-carboxyméthyl pipéridine ;
      - acide 4-aminobenzoïque ;
      - acide 4(2-aminoéthoxy)benzoïque ;
   - X_{f} est choisi parmi les résidus d'acides aminés suivants :
      - NH₂-(CH₂)ₙ-COOH, n variant de 2 à 10 ;
      - NH₂-(CH₂-CH₂-O)ₘ-CH₂CH₂COOH, m variant de 3 à 6 ;
      - acide 8-amino-3,6-dioxaoctanoïque ;
      - acide tranéxamique ;
      - acide N-méthyl-tranéxamique ;
      - acide 4(pipéridin-4-yl)butanoïque ;
      - acide 3(pipéridin-4-yl)propionique ;
      - N-(4-aminobutyl)-glycine ;
      - 4-carboxyméthyl-pipérazine ;
      - acide 4-(4-aminophényl)butanoïque ;
      - acide 3-(4-aminophényl)propanoïque ;
      - acide 4-aminophénylacétique ;
      - 4-(2aminoéthyl)-1-carboxyméthyl-pipérazine ;
      - acide trans-4-aminocyclohexanecarboxylique ;
      - acide cis-4-aminocyclohexanecarboxylique ;
      - acide cis-4-aminocyclohexane acétique ;
      - acide trans-4-aminocyclohexane acétique ;
      - 4-amino-1-carboxyméthyl pipéridine ;
      - acide 4-aminobenzoïque ;
      - acide 4(2-aminoéthoxy)benzoïque ;
   - -L- représente une liaison de nature pseudopeptidique entre les résidus X'ₐ et X'_{b}, choisie notamment dans la liste ci-dessous :
- L- = -ψ(CH₂CH₂)- ; -ψ(CH(Fₖ)=CH(Fₖ'))-; -ψ(CH₂NH)-; -ψ(NHCO)- ; -ψ(NHCONH)- ; -ψ(CO-O)-; -ψ(CS-NH)- ; -ψ(CH(OH)-CH(OH) )- ; -ψ(S-CH₂)- ; -ψ(CH₂-S)- ; -ψ(CH(CN)-CH₂)-; -ψ(CH(OH))- ; -ψ(COCH₂)- ; -ψ(CH(OH)CH₂)- ; -ψ(CH(OH)CH₂NH)-; -ψ(CH₂)- ; -ψ(CH(Fₖ))- ; -ψ(CH₂O)- ; -ψ(CH₂-NHCONH)- ; -ψ(CH(Fₖ)NHCONFₖ')- ; -ψ(CH₂-CONH)- ; -ψ(CH(Fₖ)CONH)- ; -ψ(CH(Fₖ)CH(Fₖ')CONH)- ;
ou -ψ(CH₂N)- ; -ψ(NHCON)- ; -ψ(CS-N)-; -ψ(CH(OH)CH₂N)- ; -ψ(CH₂-NHCON)- ; -ψ(CH₂-CON)-; -ψ(CH(Fₖ)CON)- ; -ψ(CH(Fₖ)CH(Fₖ')CON)-lorsque X'_{b} représente la chaîne latérale d'une proline,
ces liaisons pseudopeptidiques étant notamment décrites dans l'article de Spatola (1983),
Fₖ et Fₖ' représentant, indépendamment l'un de l'autre, un hydrogène, un halogène, un groupe alkyle de 1 à 20 atomes de carbone, ou un groupe aryle dont la structure du cycle contient de 5 à 20 atomes de carbone,
- X'ₐ représente la chaîne latérale de la lysine, de l'arginine ou de l'ornithine ; et
- X'_{b} représente la chaîne latérale de l'un des résidus d'acides aminés suivants :
   - glycine ;
   - asparagine ;
   - D-alanine ;
   - D-valine ;
   - L-proline substituée ou non en position β, γ ou δ ;
   - D-proline substituée ou non en position β, γ ou δ ;
   - acides aminés naturels N-alkylés, le groupe alkyle étant un groupe méthyle, éthyle ou benzyle ;
   - acides aminés dialkylés acycliques de formule suivante : 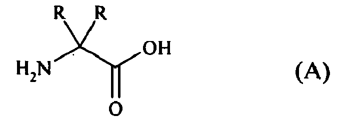 R représentant H, Me, Et, Pr ou Bu ;
   - acides aminés dialkylés cycliques de formule suivante : 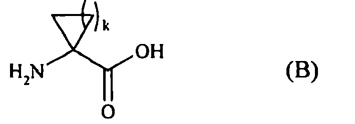 k représentant 1, 2, 3 ou 4.

Ainsi, le groupe H-X'ₐ-L-X'_{b}- peut par exemple être représenté par l'une des formules suivantes :

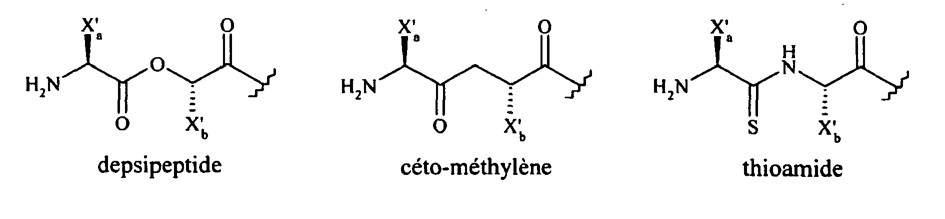

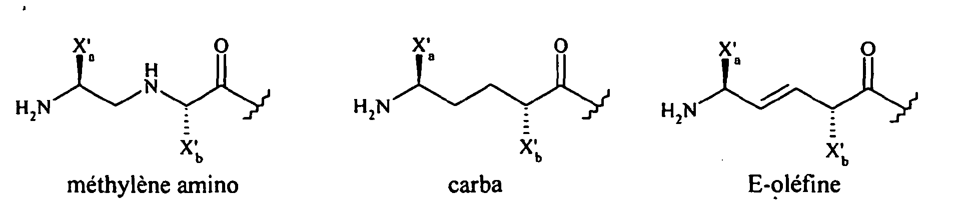

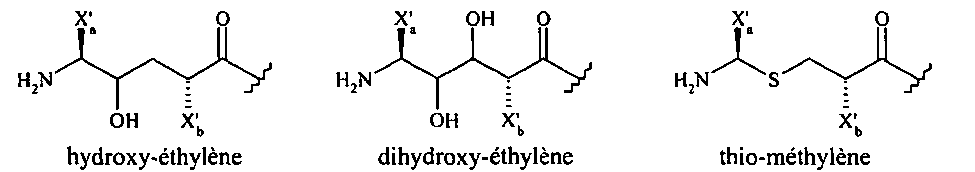

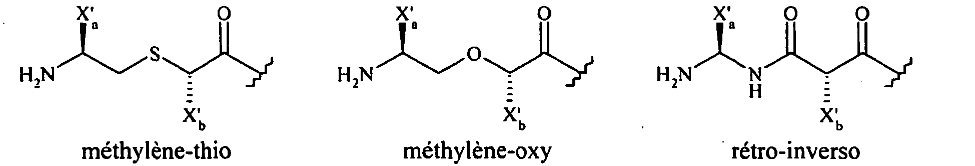

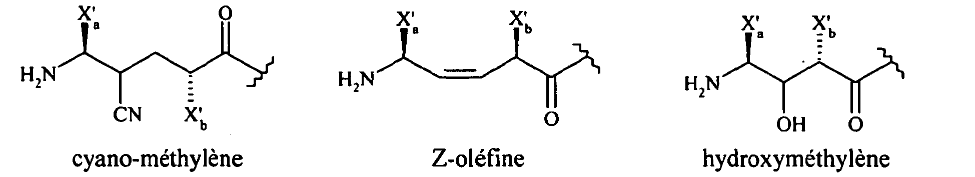

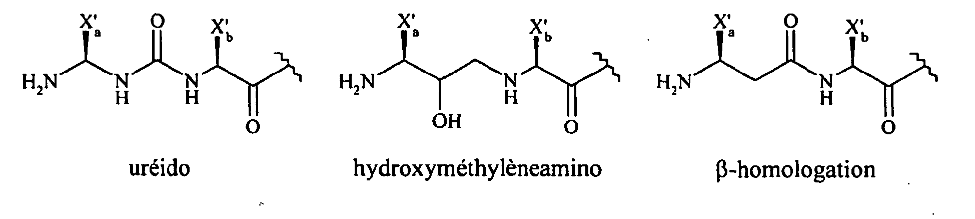

Les composés de l'invention répondent à la formule (I) telle que définie ci-dessus dans laquelle l'enchaînement Xₐ-X_{b}-X_{c}-X_{d} est différent de l'enchaînement Lys-Gly-Tyr-Tyr.

Lorsque le groupe Y est fonctionnalisé par trois fonctions COOH, Y est relié à R_{c} par un groupe -CO-, et lorsque Y est fonctionnalisé par trois fonctions amines, Y est reliée à R_{c} par un groupe -NH-.

Les composés de l'invention peuvent également être représentés par la formule suivante :

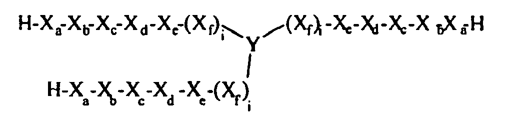

Y, Xₐ, X_{b}, X_{c}, X_{d}, Xₑ, X_{f} et i étant tels que définis ci-dessus.

Le groupe Xₑ-(X_{f})ᵢ- sert de bras pour relier H-Xₐ-X_{b}-X_{c}-X_{d}- à Y.

Les composés de l'invention sont des composés de symétrie C₃ et correspondent à de nouveaux ligands trimériques de CD40.

Dans le cadre de la présente invention, l'expression "acides aminés" désigne notamment des acides aminés naturels ou non naturels, notamment des acides aminés alpha-, beta-, gamma-, delta-aminé, ou des ω-aminoacides.

Le terme "macrocycle" désigne un cycle comprenant des atomes d'azote, de carbone ou d'oxygène, dont le nombre total d'atomes est supérieur ou égal à 9.

L'expression "β-acides aminés correspondant à la lysine, l'arginine ou l'ornithine, portant la substitution en position α ou β" désigne des composés répondant à l'une des formules suivantes : H₂N-CH₂-CH(R_{b})-COOH (position α) ou H₂N-CH(R_{b})-CH₂COOH (position β), R_{b} correspondant au résidu de la lysine, de l'arginine ou de l'ornithine.

Plus exactement, Xₐ est choisi parmi les groupes suivants :

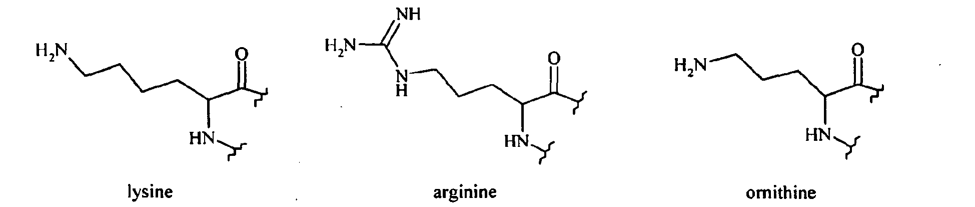

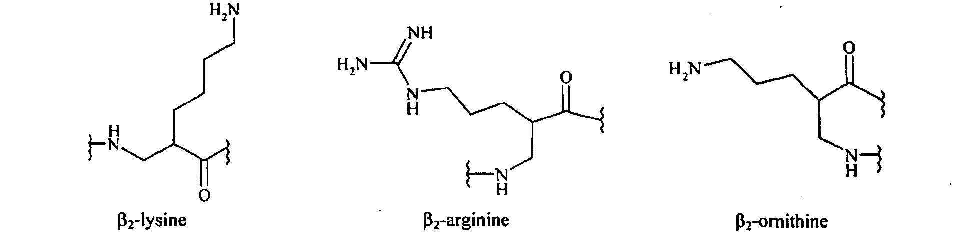

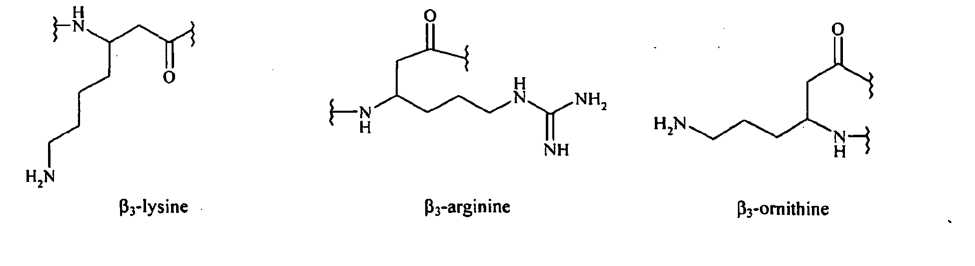

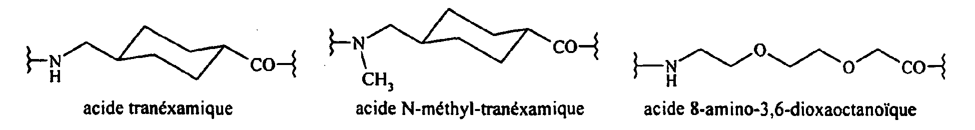

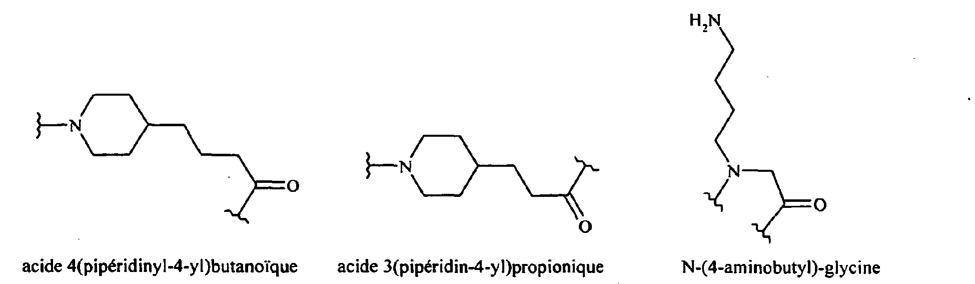

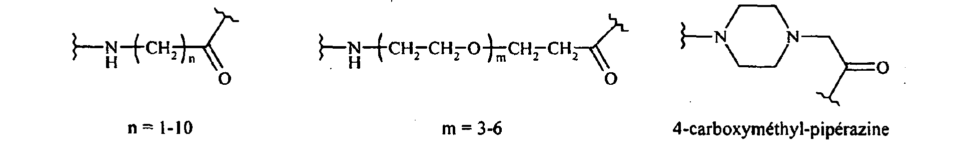

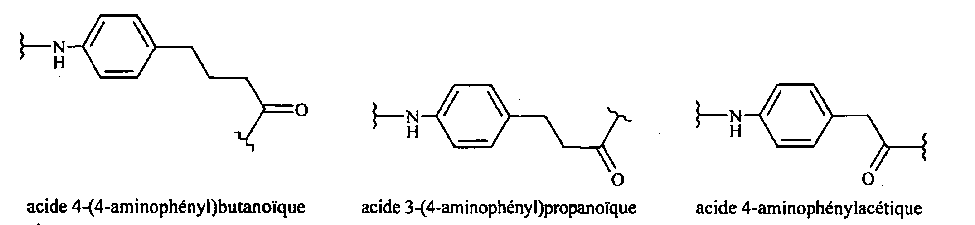

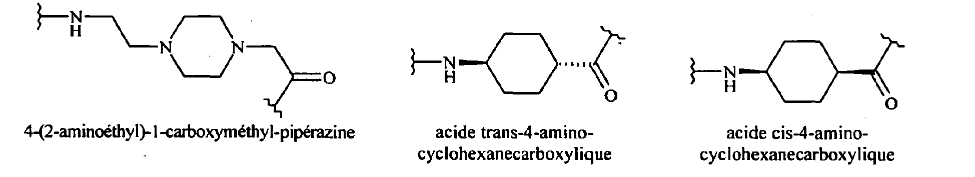

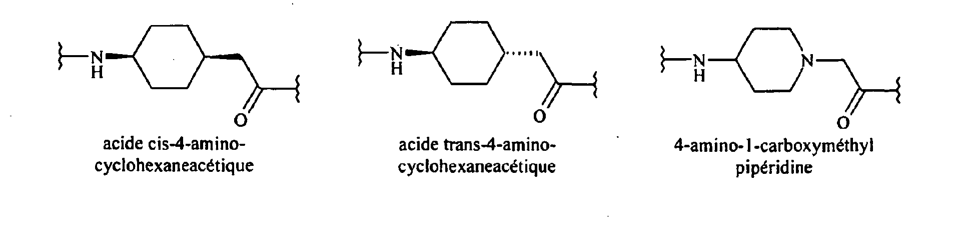

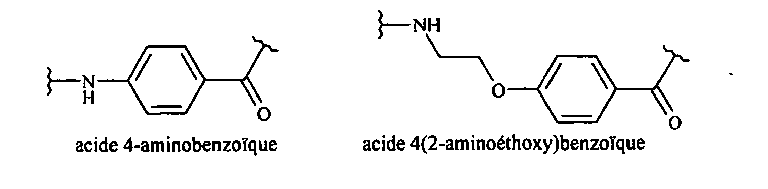

X_{b} est choisi parmi les groupes suivants :

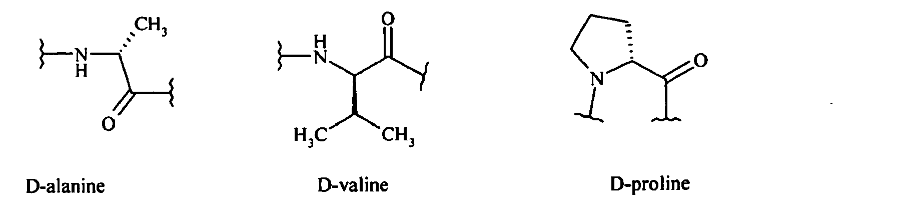

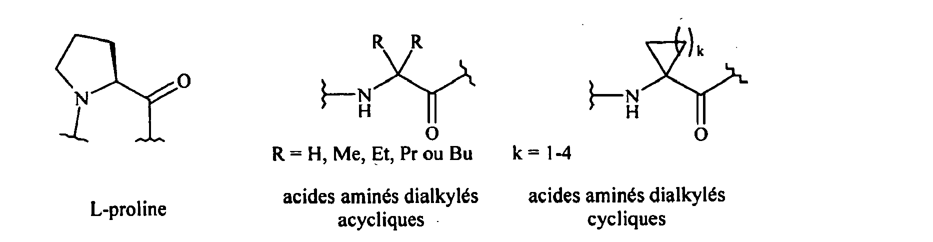

X_{c} et X_{d} sont choisis parmi les groupes suivants :

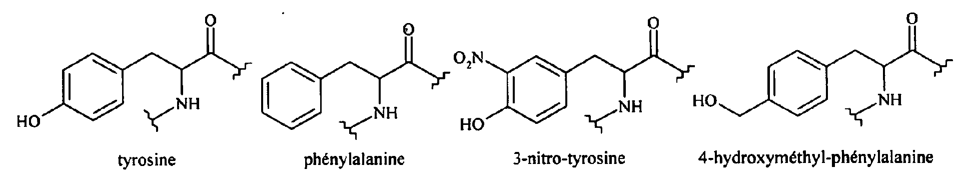

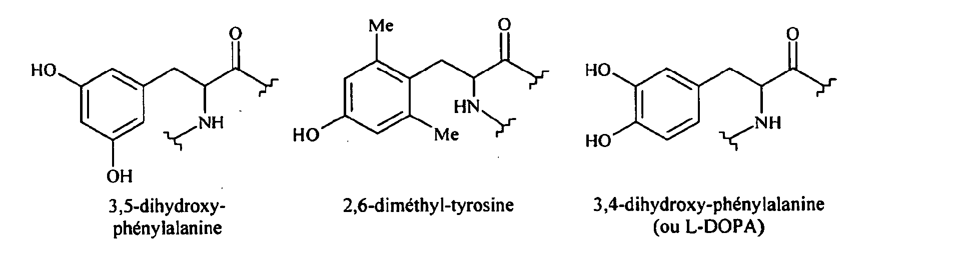

Xₑ et X_{f} sont choisis parmi les groupes suivants :

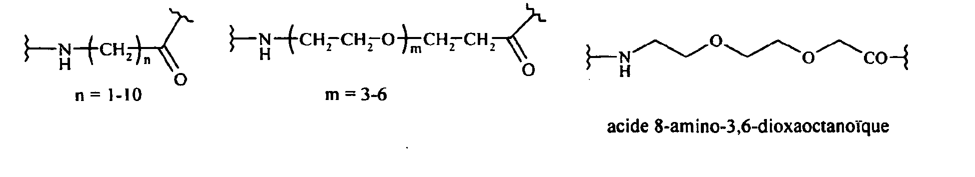

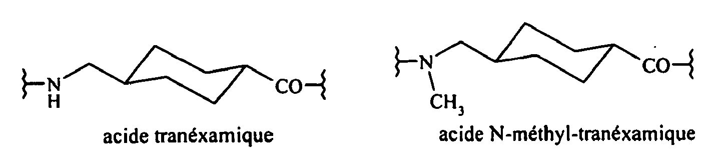

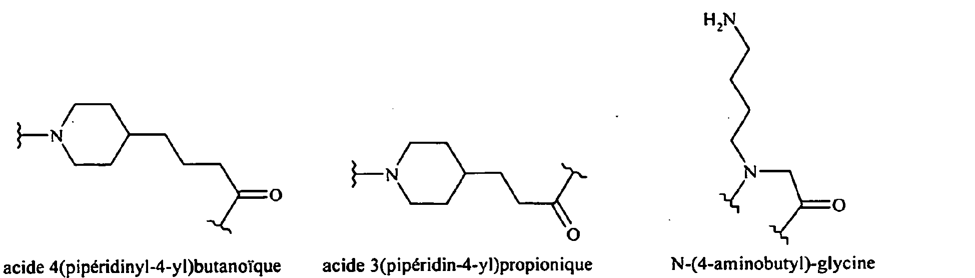

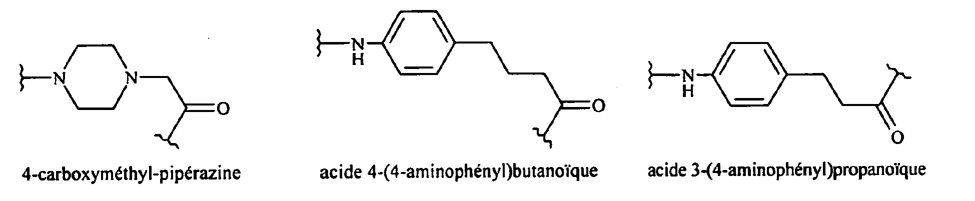

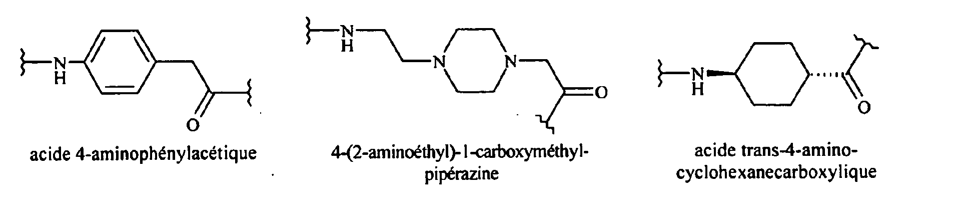

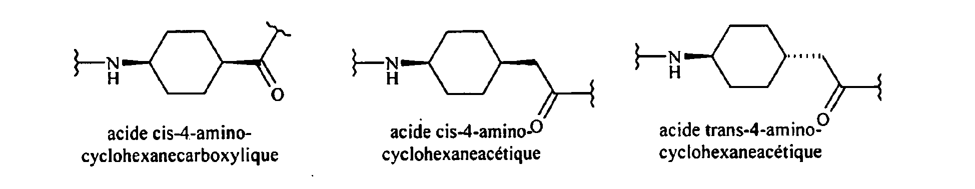

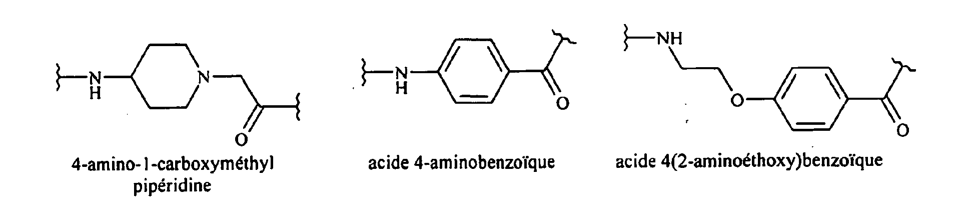

Un composé préféré selon l'invention est un composé de formule (I) susmentionnée dans laquelle :
- Xₐ n'est pas un résidu de lysine.

Le remplacement du résidu lysine permet, d'une part, d'optimiser la liaison du ligand avec CD40, c'est-à-dire augmenter l'affinité de la liaison dudit ligand avec CD40 et, d'autre part, de modifier la lipophilie de ce ligand.

Le remplacement du résidu glycine permet de stabiliser la conformation bioactive de l'enchaînement Xₐ-X_{b}-X_{c}-X_{d}.

Selon un mode de réalisation préféré, le composé de l'invention est un composé de formule (I) telle que définie ci-dessus, dans laquelle Xₐ n'est pas un résidu de lysine.

Ainsi, un composé préféré selon la présente invention est caractérisé en ce que Xₐ représente un résidu d'un acide aminé de formule NH₂-(CH₂)ₙ-COOH, n variant de 1 à 10, et de préférence en ce que Xₐ représente un résidu de l'acide aminohexanoïque.

De tels composés correspondent à des composés dans lesquels le résidu lysine du fragment ¹⁴³Lys-Gly-Tyr-Tyr¹⁴⁶ de CD40 naturel est remplacé par un résidu d'un acide aminé de formule NH₂-(CH₂)ₙ-COOH, n variant de 1 à 10.

Un composé préféré de l'invention est un composé répondant à l'une des formules suivantes : 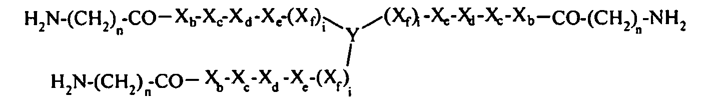 n étant tel que défini ci-dessus

Le composé préféré correspondant à n = 5 répond à la formule suivante :

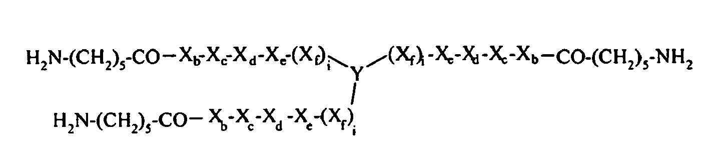

Le remplacement dans ce composé dudit résidu lysine par l'acide aminohexanoïque permet de moduler la lipophilie du ligand de l'invention tout en conservant une partie des fonctions effectrices dudit ligand, résultant de son interaction avec CD40 (test d'apoptose sur lymphomes, voir partie expérimentale).

De préférence, le composé de l'invention est un composé de formule (I) telle que définie ci-dessus, dans laquelle Xₐ n'est pas un résidu de lysine et dans laquelle les résidus X_{b}, X_{c} et X_{d} sont identiques aux résidus de la séquence naturelle de CD40L.

Ainsi, un composé préféré selon la présente invention est caractérisé en ce que X_{b} représente un résidu de D-proline. Un tel composé répond à la formule suivante :

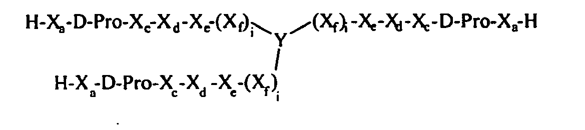

Le remplacement dans ce composé du résidu glycine du fragment ¹⁴³Lys-Gly-Tyr-Tyr¹⁴⁶ de CD40 naturel par un résidu de D-proline permet de stabiliser ledit ligand en stabilisant le repliement de type II' formé par la boucle ¹⁴³Lys-Gly-Tyr-Tyr¹⁴⁶ de CD40 naturel. Ainsi, une telle stabilisation permet d'obtenir des ligands plus sélectifs, notamment par leur capacité à induire une apoptose plus spécifique des lymphomes B par rapport aux ligands naturels de CD40, notamment utiles dans le cadre de l'apoptose des lymphomes.

De préférence, le composé de l'invention est un composé de formule (I) telle que définie ci-dessus, dans laquelle les résidus Xₐ, X_{c} et X_{d} sont identiques aux résidus de la séquence naturelle de CD40L.

Un composé préféré selon la présente invention est un composé dans lequel X_{b} représente un résidu de L-proline. Le remplacement dans ce composé du résidu glycine du fragment ¹⁴³Lys-Gly-Tyr-Tyr¹⁴⁶ de CD40 naturel par un résidu de L-proline permet d'obtenir un ligand qui présente les mêmes propriétés de liaison que le ligand naturel avec CD40, mais qui ne présente pas les propriétés biologiques associées : ce ligand n'a donc aucun effet sur l'apoptose des lymphomes B.

De préférence, les composés susmentionnés sont caractérisés en ce que
- Xₐ représente de préférence un résidu d'un acide aminé de formule NH₂-(CH₂)ₙ-COOH, n variant de 1 à 10, et notamment un résidu d'acide aminohexanoïque, et
- X_{b} représente de préférence un résidu de D-proline.

De tels composés répondent à l'une des formules générales suivantes :

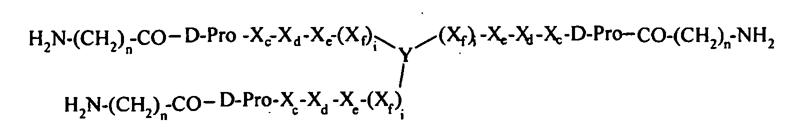

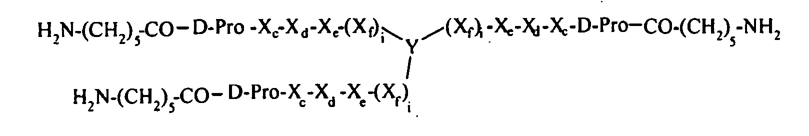

La présente invention concerne également les composés, répondant à la formule (I) telle que définie ci-dessus marqués par de la biotine.

Ces composés sont utilisés pour préparer des ligands fonctionnalisés permettant de multimériser les ligands de l'invention à partir d'un polymère. A cet égard, on peut notamment utiliser la streptavidine qui se peut se lier à plusieurs biotines.

Ainsi, la présente invention concerne également les ligands de l'invention sous forme multimérique, c'est-à-dire correspondant à une molécule polymérique sur laquelle sont liés plusieurs ligands selon l'invention, ainsi que l'utilisation de ces ligands multimérisés en tant que marqueurs.

Ces composés peuvent également permettre de purifier le récepteur CD40 par l'utilisation de colonnes greffées par ces composés ou de détecter facilement le récepteur CD40 à la surface des cellules au moyen de ces composés marqués, notamment dans le cadre d'une immunothérapie des tumeurs avec des cellules dendritiques.

La présente invention concerne des composés de formule (I) telle que définie ci-dessus, caractérisés en ce que Y répond à la formule suivante (II) : 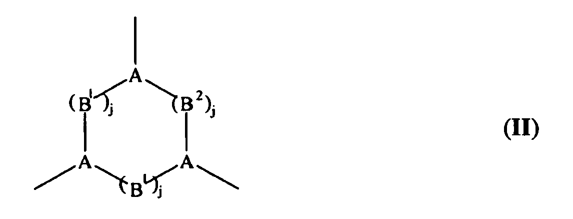 dans laquelle :
- j est 0 ou 1 ;
- A représente un résidu d'acide aminé ou un dérivé d'acide aminé choisi indifféremment parmi : 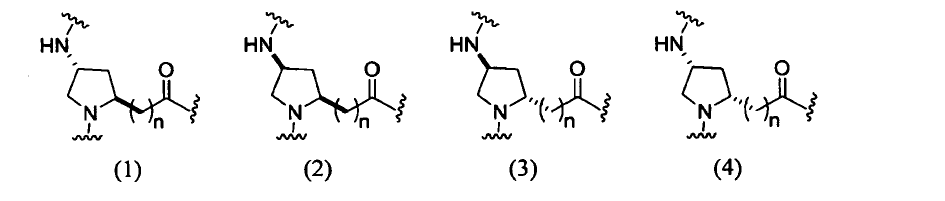 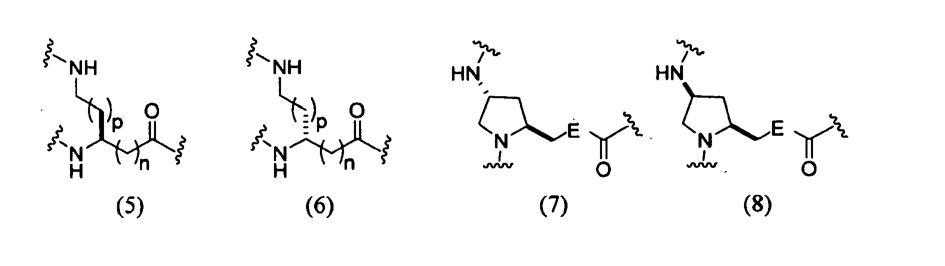 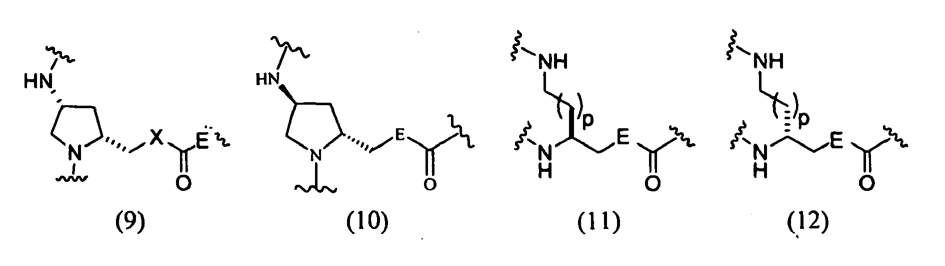
   - n représentant 0, 1, 2 ou 3 ;
   - p représentant 0, 1, 2 ou 3 ;
   - E représentant NH ou O ;
- B¹ est un résidu d'acide aminé ou un dérivé d'acide aminé choisi de manière indépendante parmi : 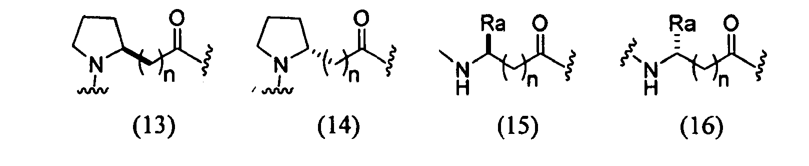 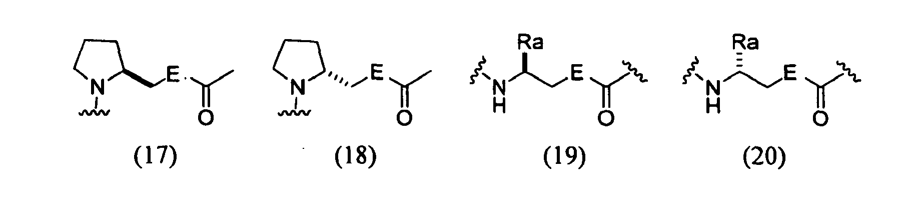
   - n représentant 0, 1, 2 ou 3 ;
   - p représentant 0, 1, 2 ou 3 ;
   - E représentant NH ou O ;
   - Ra représentant la chaîne d'un acide aminé protéinogénique, C1-C8 alkylé ;
- B² peut être identique à B¹ ou choisi de manière indépendante parmi les groupes suivants : 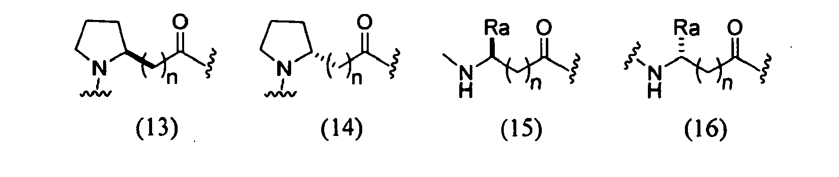 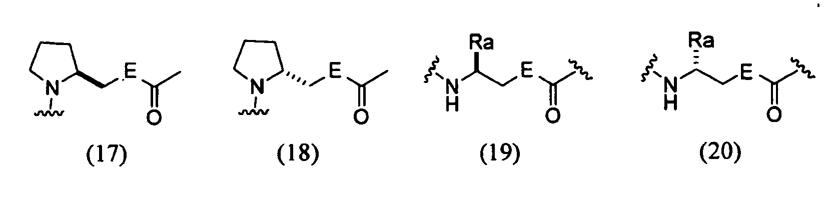 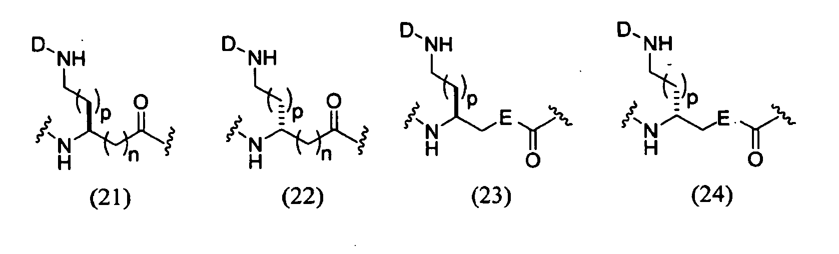
   . n représentant 0, 1, 2 ou 3 ;
   . p représentant 0, 1, 2 ou 3 ;
   . E représentant NH ou O ;
   . Ra représentant la chaîne d'un acide aminé protéinogénique, C1-C8 alkylé ;
   . D représentant l'un des groupes suivants : (+)-Biotinyl-, (+)-Biotinyl-X_{g}-, HS-(CH₂)_{q}-CO-, Pys-S-(CH₂)_{q}-CO-, Npys-S-(CH₂)_{q}-CO-, HS-(CH₂)_{q}-CO-X_{g}-, Pys-S-(CH₂)_{q}-CO-X_{g}-, Npys-S-(CH₂)_{q}-CO-X_{g}-
q représentant un nombre entier variant de 2 à 6 ;
X_{g} correspondant au résidu de l'un des groupes suivants :
-NH₂-(CH₂)ₙ-COOH, n variant de 1 à 10 ;
-NH₂-(CH₂-CH₂-O)ₘ-CH₂CH₂COOH, m variant de 3 à 6 ;
acide 8-amino-3,6-dioxaoctanoïque ;
acide tranéxamique ;
acide N-méthyl-tranéxamique ;
acide 4(pipéridin-4-yl)butanoïque ;
acide 3(pipéridin-4-yl)-propionique ;
N-(4-aminobutyl)-glycine ;
4-carboxyméthyl-pipérazine ;
acide 4-(4-aminophenyl)butanoïque ;
acide 3-(4-aminophenyl)propanoïque ;
acide 4-aminophénylacétique ;
4-(2aminoéthyl)-1-carboxyméthyl-pipérazine ;
acide trans-4-aminocyclohexane carboxylique ;
acide cis-4-aminocyclohexane carboxylique ;
acide cis-4-aminocyclohexane acétique ;
acide trans-4-aminocyclohexane acétique ;
4-amino-1-carboxyméthyl pipéridine ;
acide 4-aminobenzoïque ;
acide 4(2-aminoéthoxy)benzoïque.

Dans la formule (II) susmentionnée, A est un groupe de formule (1), (2), (3), (4), (5), (6), (7), (8), (9), (10), (11) ou (12) ; B¹ est un groupe de formule (13), (14), (15), (16), (17), (18), (19) ou (20) et B² est un groupe de formule (13), (14), (15), (16), (17), (18), (19), (20), (21), (22), (23) ou (24).

X_{g} répond aux mêmes définitions que Xₑ et X_{f}.

Le terme "Pys" désigne le groupe pyridine sulfényle et le terme "Npys" désigne le groupe nitropyridine sulfényle.

La biotine répond à la formule

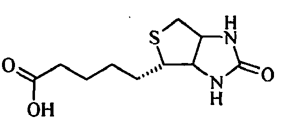

Un groupe biotinyle répond donc à la formule suivante

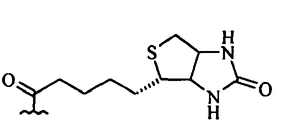

L'expression "chaîne d'un acide aminé protéinogénique" désigne une chaîne latérale d'un acide aminé présent dans les protéines, correspondant ou non à un des 20 acides aminés du code génétique.

Les composés de formule (II) correspondent à l'une des formules générales suivantes :

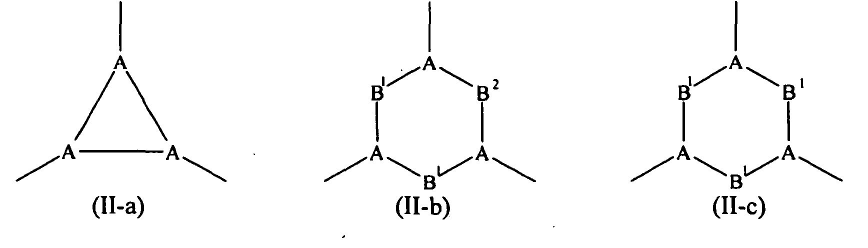

La présente invention concerne également les composés tels que définis ci-dessus, répondant à l'une des formules suivantes :

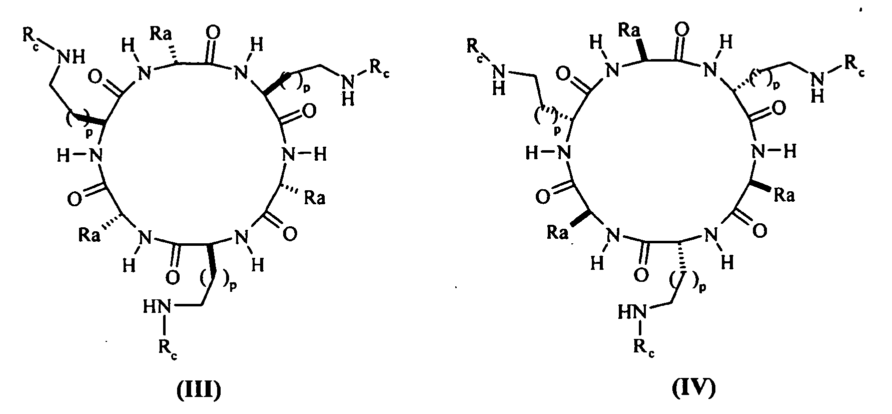

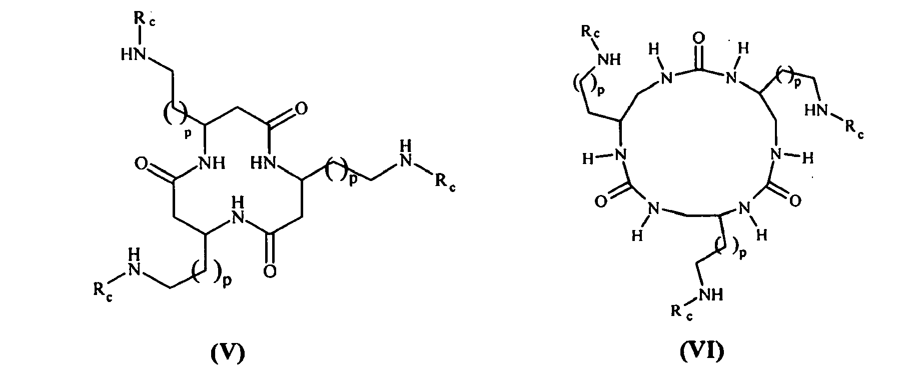

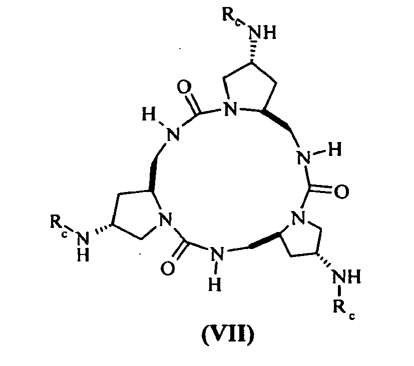

Ra, R_{c} et p étant tels que définis ci-dessus.

Le composé de formule (III) correspond à un composé de formule (I) avec Y répondant à la formule (II-c) telle que définie ci-dessus, dans laquelle A est un groupe de formule (5) et B¹ est un groupe de formule (16) où n = 0.

Le composé de formule (IV) correspond à un composé de formule (I) avec Y répondant à la formule (II-c) telle que définie ci-dessus, dans laquelle A est un groupe de formule (6) et B¹ est un groupe de formule (15) où n = 0.

Le composé de formule (V) correspond à un composé de formule (I) avec Y répondant à la formule (II-a) telle que définie ci-dessus, dans laquelle A est un groupe de formule (5) où n = 1.

Le composé de formule (VI) correspond à un composé de formule (I) avec Y répondant à la formule (II-a) telle que définie ci-dessus, dans laquelle A est un groupe de formule (11) où E = NH.

Le composé de formule (VII) correspond à un composé de formule (I) avec Y répondant à la formule (II-a) telle que définie ci-dessus, dans laquelle A est un groupe de formule (10) où E = NH.

Ces différents composés sont particulièrement intéressants dans la mesure où leurs structures sont des structures cycliques rigides et planes de conformations bien définies permettant d'orienter et de positionner les résidus en contact avec le récepteur (Xₐ, X_{b}, X_{c} et X_{d}), avec une géométrie et une distance préférentielles pour l'interaction.

Selon un mode de réalisation avantageux, les composés de la présente invention répondent à la formule suivante (III-a) :

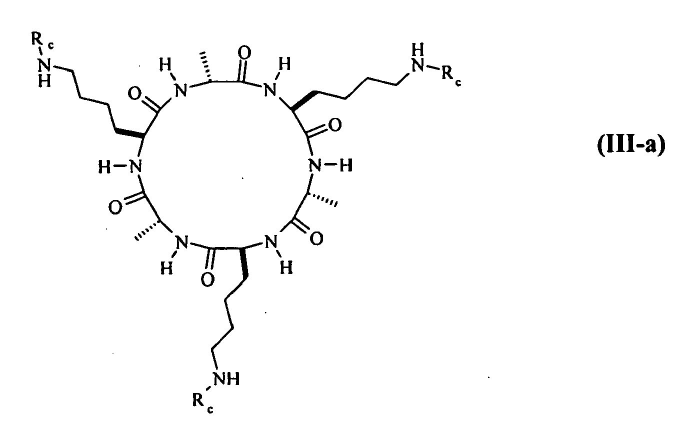

Le composé de formule (III-a) correspond à un composé de formule (III), dans laquelle Rₐ représente un groupe méthyle et p = 3.

Selon un mode de réalisation avantageux, les composés de la présente invention répondent à la formule suivante (V-a) :

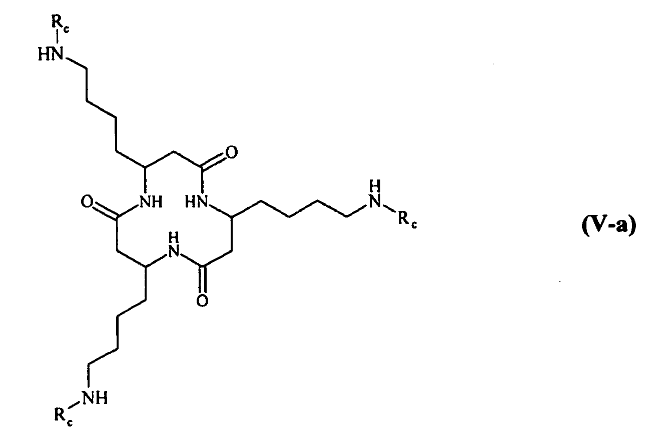

Le composé de formule (V-a) correspond à un composé de formule (V), dans laquelle p = 3.

Selon un mode de réalisation avantageux, les composés de la présente invention répondent à l'une des formules suivantes :

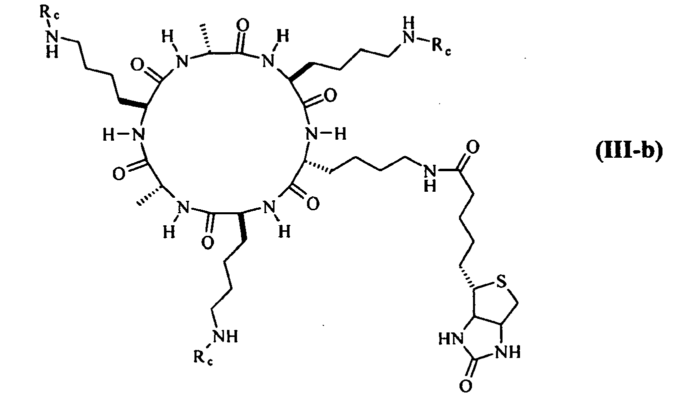

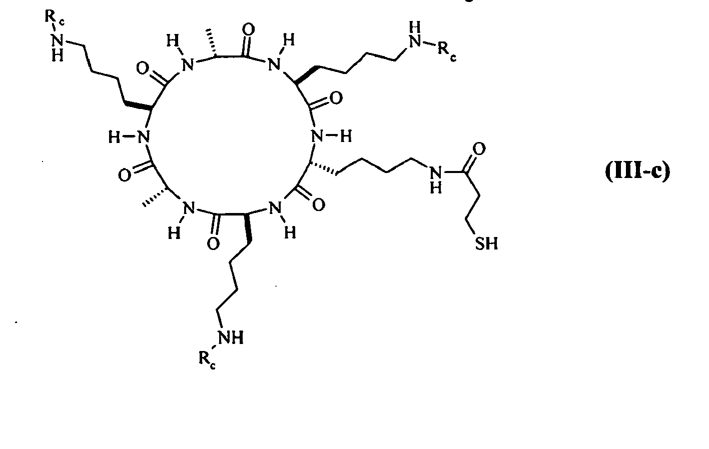

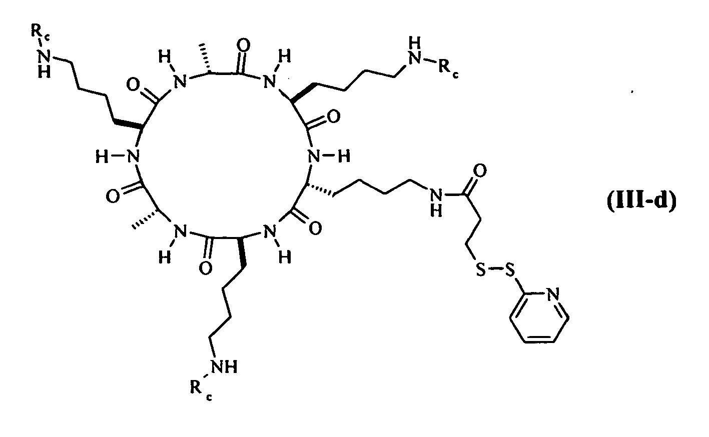

R_{c} étant tel que défini ci-dessus.

Les composés susmentionnés de formules (III-b), (III-c) et (III-d) correspondent à des composés de formule (III-a) marqués respectivement par de la biotine, par un groupe -CO-(CH₂)₂-SH et 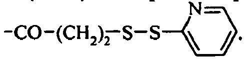 Plus exactement, ces composés sont des composés de formule (I) avec Y répondant à la formule (II-b) telle que définie ci-dessus, dans laquelle A est un groupe de formule (5), B¹ est un groupe de formule (16) où n = 0 et B² est un groupe de formule (22) où p = 3.

Dans le composé de formule (III-b), D est un groupe biotinyle.

Dans le composé de formule (III-c), D est un groupe -CO-(CH₂)_{q}-SH, où q = 2. Dans le composé de formule (III-d), D est un groupe -CO-(CH₂)_{q}-S-pys, où q = 2.

Les composés préférés de l'invention sont des composés de formule (I) telle que définie ci-dessus, dans laquelle R_{c} est :
- H-Lys-(D)Pro-Tyr-Tyr-NH-(CH₂)₅-CO- ou
- Ahx-(D)Pro-Tyr-Tyr-NH-(CH₂)₅-CO- ou
- H-Lys-ψ(CH₂N)-(D)Pro-Tyr-Tyr-NH-(CH₂)₅-CO-, -ψ(CH₂N)- correspondant à une liaison pseudopeptidique de type méthylène-amino.

Le groupe H-Lys-(D)Pro-Tyr-Tyr-NH-(CH₂)₅-CO- est un groupe R_{c} dans lequel i = 0, Xₐ est un résidu de lysine, X_{b} est un résidu de D-proline, X_{c} est un résidu de tyrosine, X_{d} est un résidu de tyrosine et Xₑ est un résidu de l'acide aminé de formule NH₂-(CH₂)ₙ-COOH où n = 5.

Le groupe H-Lys-ψ(CH₂N)-(D)Pro-Tyr-Tyr-NH-(CH₂)₅-CO- est un groupe R_{c} dans lequel i = 0, X'ₐ est un résidu de lysine, X'_{b} est un résidu de D-proline, -ψ(CH₂N)-est une liaison pseudopeptidique de type méthylène-amino (le groupe CONH de la liaison peptidique est remplacé par un groupe CH₂-N), X_{c} est un résidu de tyrosine, X_{d} est un résidu de tyrosine et Xₑ est un résidu de l'acide aminé de formule NH₂-(CH₂)ₙ-COOH où n = 5.

De tels composés répondent à l'une des formules suivantes :

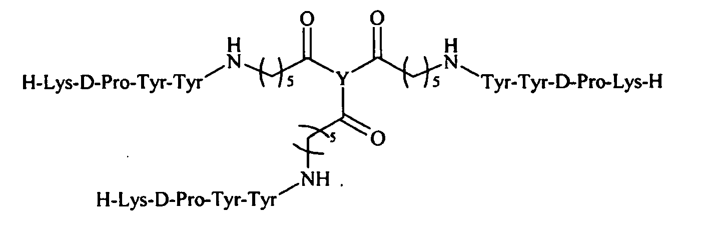

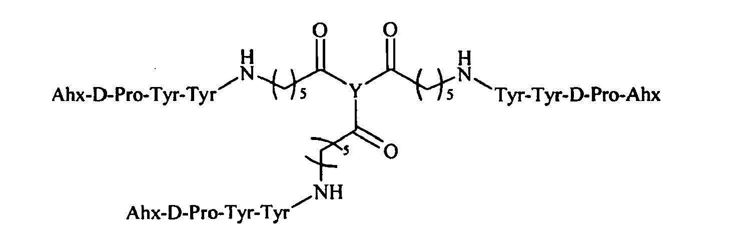

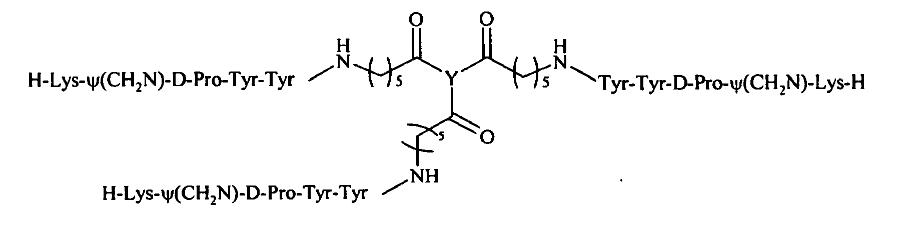

Y étant tel que défini ci-dessus.

Ces composés peuvent également être représentés par l'une des formules suivantes :

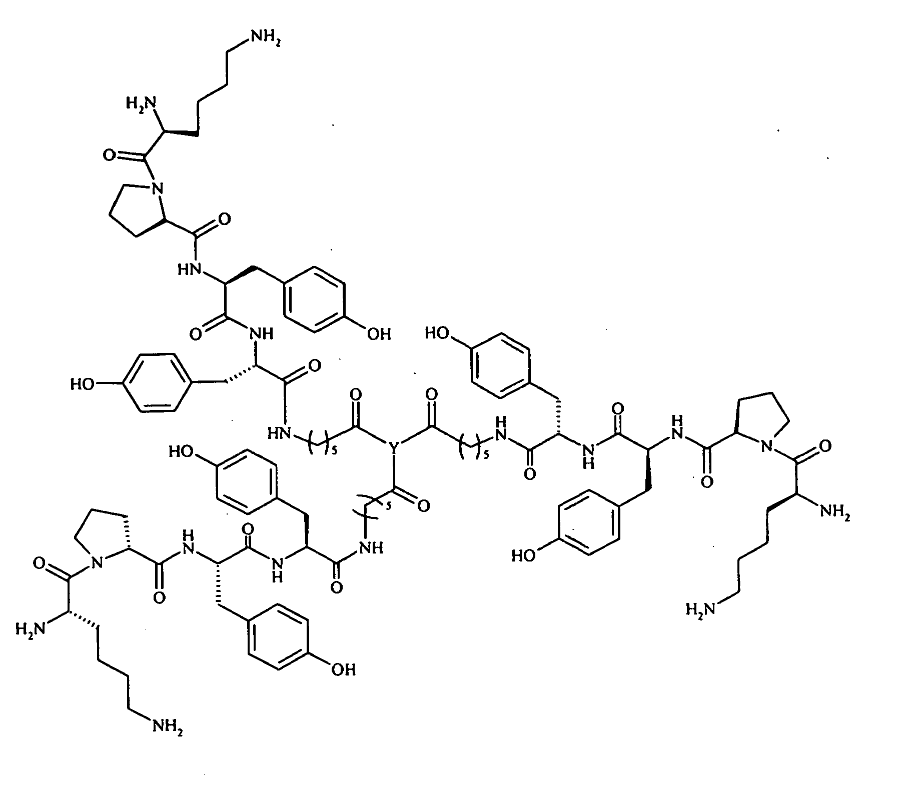

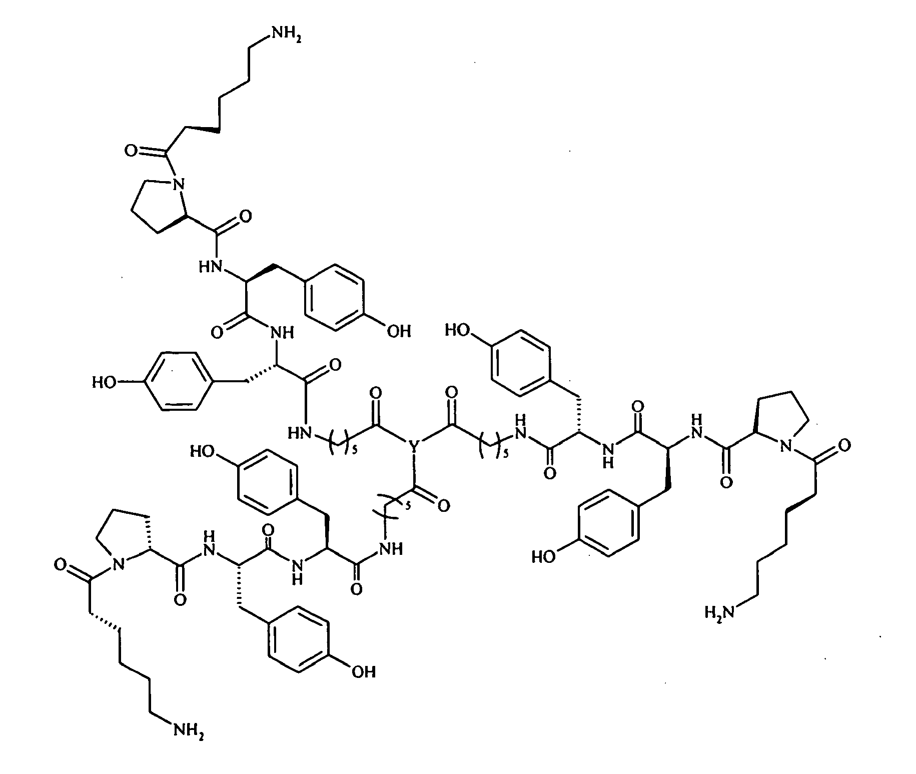

Des composés préférés de l'invention répondent à l'une des formules suivantes : 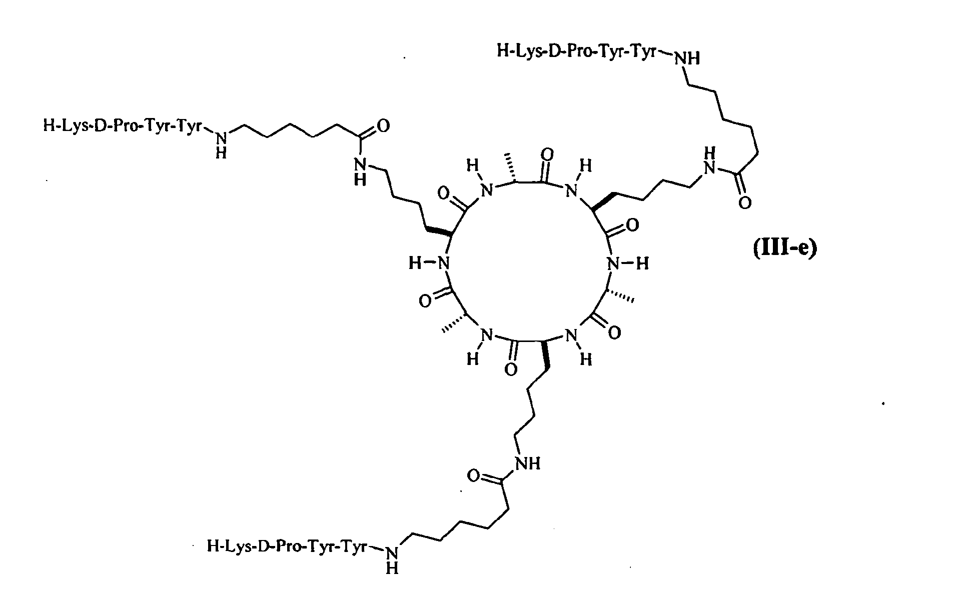 ou

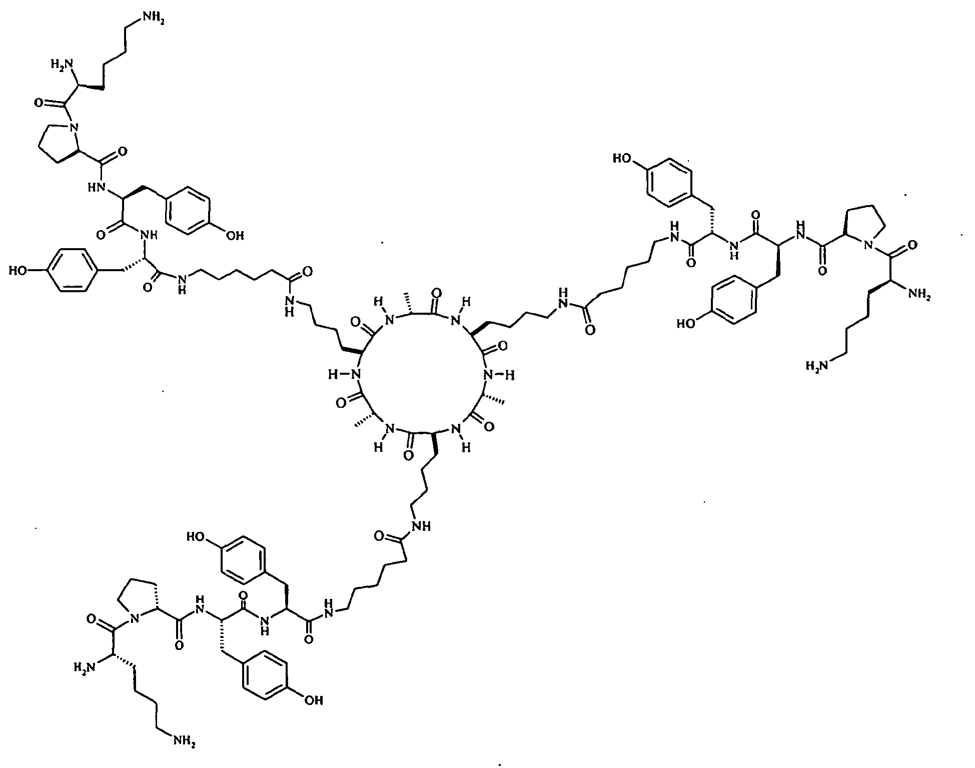

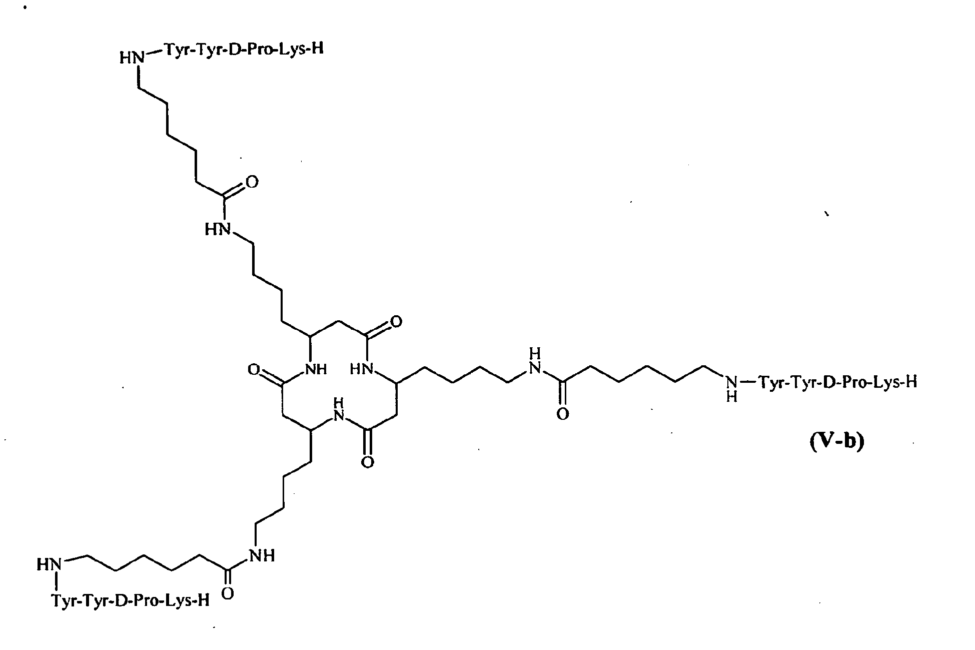

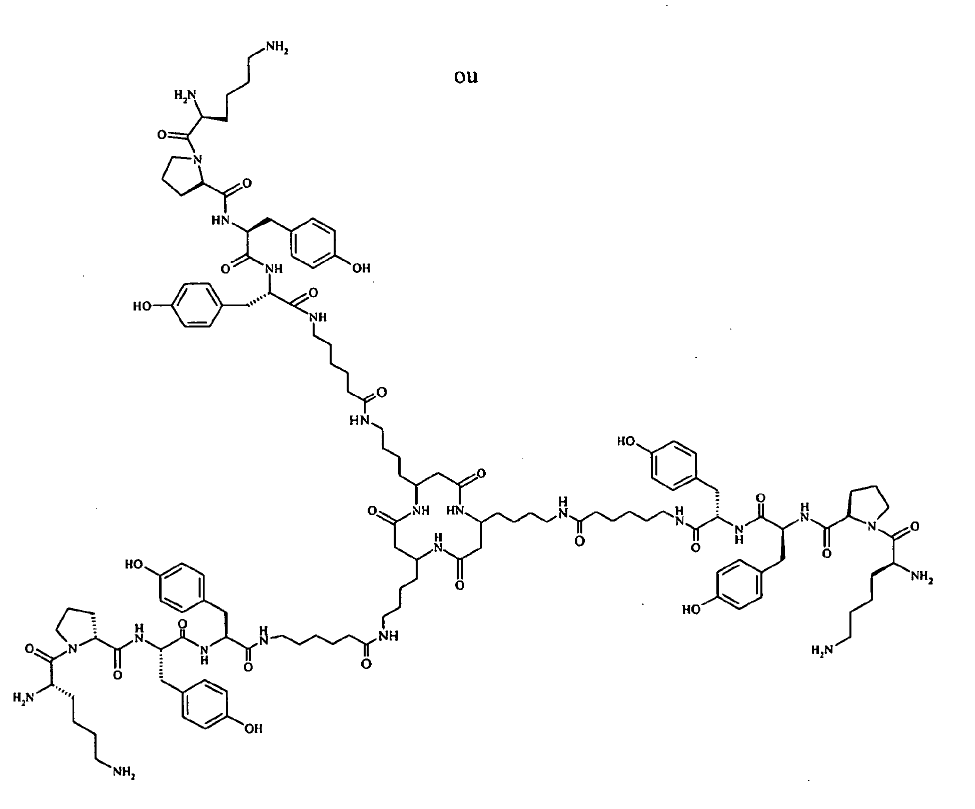

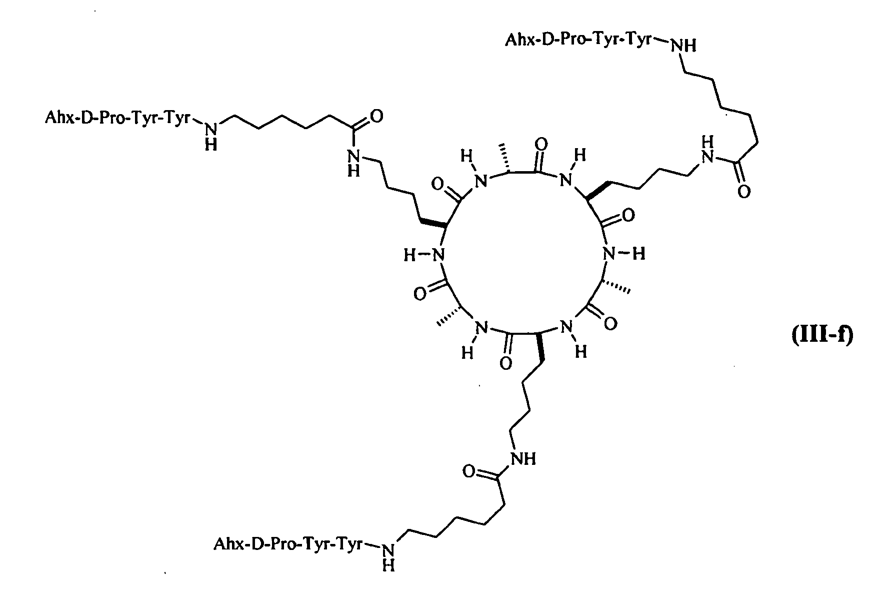

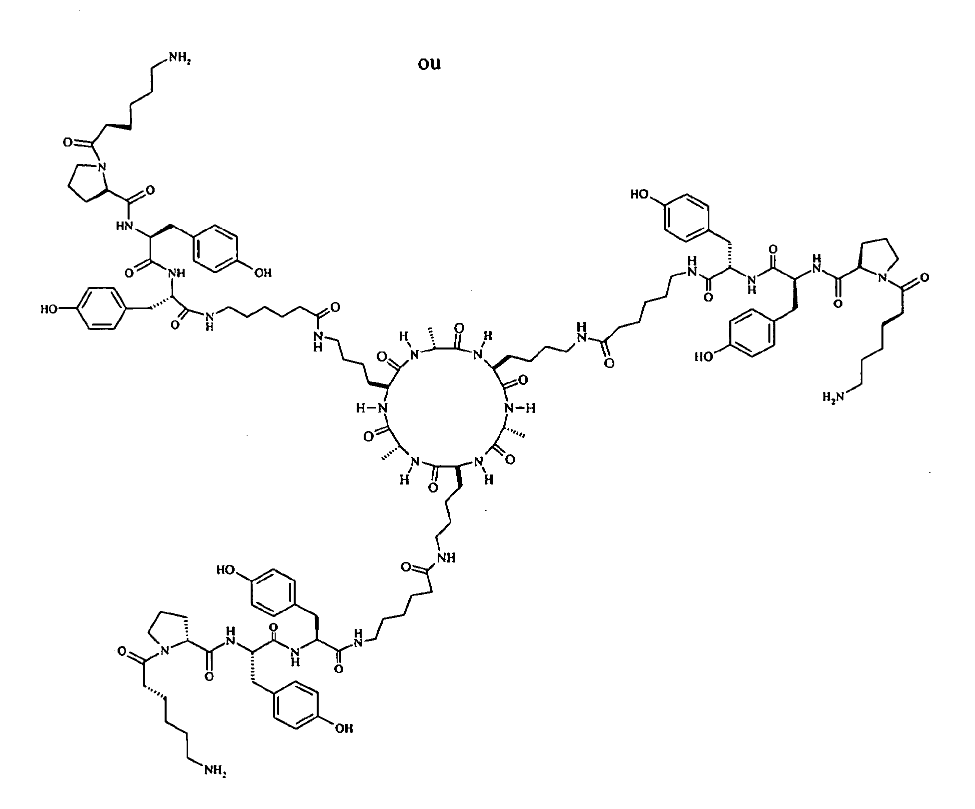

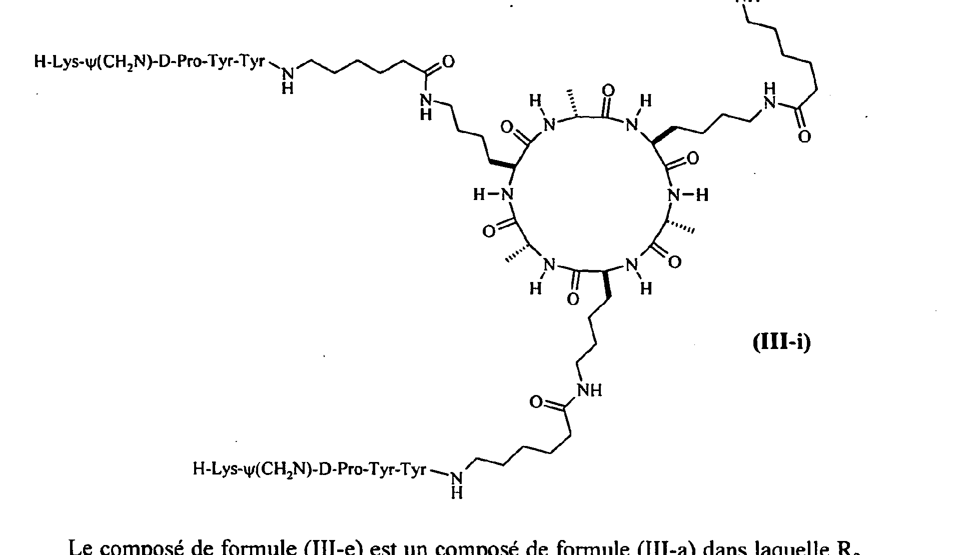

Le composé de formule (III-e) est un composé de formule (III-a) dans laquelle R_{c} est H-Lys-(D)Pro-Tyr-Tyr-NH-(CH₂)₅-CO-.

Le composé de formule (V-b) est un composé de formule (V-a) dans laquelle R_{c} est H-Lys-(D)Pro-Tyr-Tyr-NH-(CH₂)₅-CO-.

Le composé de formule (III-f) est un composé de formule (III-a) dans laquelle R_{c} est Ahx-(D)Pro-Tyr-Tyr-NH-(CH₂)₅-CO-.

Le composé de formule (III-i) est un composé de formule (III-a) dans laquelle Ruz est H-Lys-ψ(CH₂N)-(D)Pro-Tyr-Tyr-NH-(CH₂)₅-CO-.

La présente invention concerne également les composés répondant à l'une des

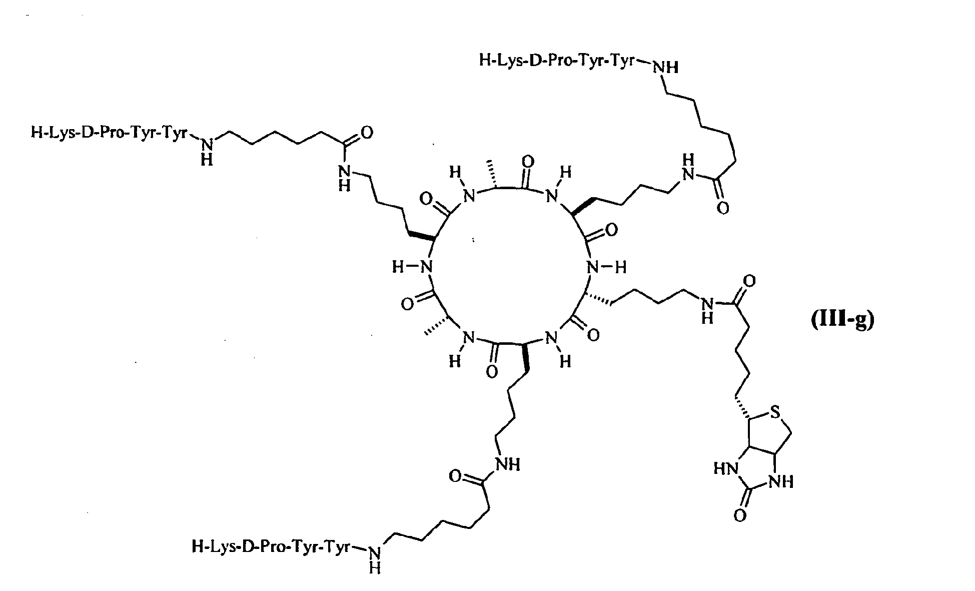

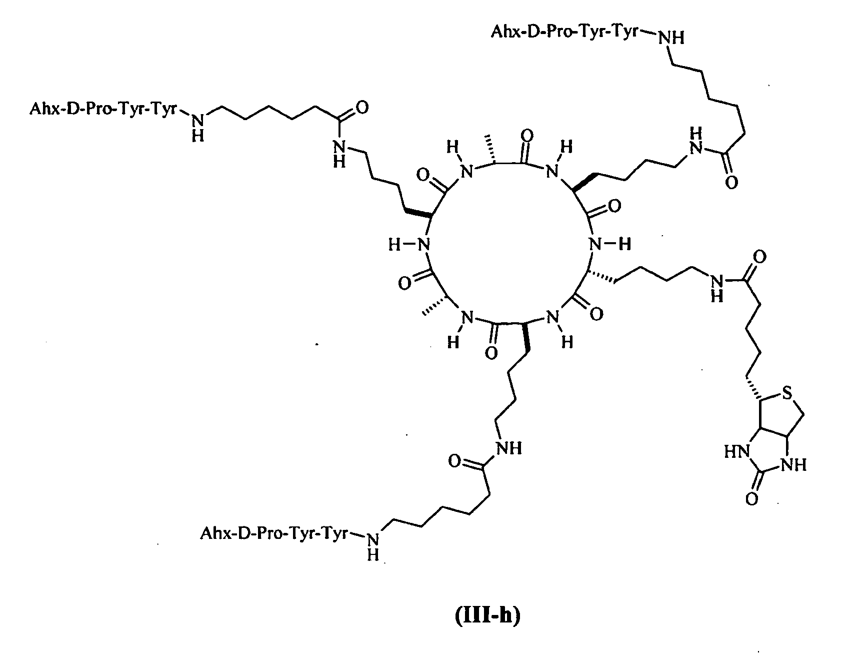

Le composé (III-g) est un composé de formule (III-e) marqué par de la biotine. Ce composé correspond également à un composé de formule (III-b) dans lequel R_{c} est un groupe H-Lys-(D)Pro-Tyr-Tyr-.

Le composé (III-h) est un composé de formule (III-f) marqué par de la biotine. Ce composé correspond également à un composé de formule (III-b) dans lequel R_{c} est un groupe Ahx-(D)Pro-Tyr-Tyr-.

La présente invention concerne également une composition pharmaceutique caractérisée en ce qu'elle comprend, à titre de substance active, un composé tel que défini ci-dessus, répondant à la formule (I), en association avec un vecteur pharmaceutiquement acceptable.

La présente invention concerne également une composition vaccinale, caractérisée en ce qu'elle comprend, à titre de substance active, un composé tel que défini ci-dessus, répondant à la formule (I), en association avec un adjuvant pharmaceutiquement acceptable.

La présente invention concerne également l'utilisation de composés tels que définis ci-dessus, répondant à la formule (I), pour la préparation d'un médicament destiné au traitement de pathologies impliquant l'inhibition ou l'activation de la réponse immunitaire.

La réponse immunitaire doit être inhibée au cours des maladies inflammatoires (rhumatismes inflammatoires), des maladies auto-immunes, des réactions d'hypersensibilités en général et des allergies en particulier, des rejets de greffes, des réactions du greffon contre l'hôte.

La réponse immunitaire doit être activée dans les vaccinations en général, dans l'immunothérapie des cancers, dans les maladies bactériennes ou virales induisant une immunosuppression (rougeole, SIDA, virus de l'herpes, cytomégalovirus...), dans le traitement des maladies bactériennes virales ou impliquant des agents infectieux non conventionnels (prions) chez les personnes présentant un déficit immunitaire primaire ou secondaire.

La présente invention concerne également l'utilisation telle que mentionnée ci-dessus, pour la préparation d'un médicament destiné au traitement de pathologies impliquant l'inhibition de la réponse immunitaire, telles que les rejets de greffes, les allergies ou les maladies auto-immunes.

Les maladies impliquant l'inhibition de la réponse immunitaire comprennent les maladies auto-immunes telles que les diabètes, la sclérose en plaques, le lupus érythémateux disséminé ou l'arthrite rhumatoïde, les rejets de greffes, notamment dans le cadre d'allogreffes, de xénogreffes ou les réactions du greffon contre l'hôte, ainsi que les réactions d'hypersensibilités telles que les allergies, notamment le rhume des foins et les dermatites atopiques, ou les granulomes.

Les composés selon la présente invention, utilisés dans le cadre de l'inhibition de la réponse immunitaire, peuvent être administrés par voie intraveineuse, par les voies muqueuses (orales, aériennes, nasales, vaginales), par voie sous-cutanée, intradermique ou épicutanée.

La présente invention concerne également une composition pharmaceutique, caractérisée en ce qu'elle comprend un composé selon la présente invention, pour le traitement de pathologies impliquant l'inhibition de la réponse immunitaire, lequel composé est présent dans la composition pharmaceutique en quantités telles qu'il peut être administré à raison d'environ 100 ng à environ 5 mg par jour et par individu.

La présente invention concerne également l'utilisation telle que mentionnée ci-dessus, pour la préparation d'un médicament destiné au traitement de pathologies impliquant l'augmentation de la réponse immunitaire, telles que les cancers ou les infections parasitaires, bactériennes, virales ou impliquant des agents infectieux non conventionnels tels que les prions.

Les cas impliquant l'activation de la réponse immunitaire comprennent les vaccinations en général, notamment les vaccins contre la grippe ou contre les maladies infantiles, l'immunothérapie des cancers, notamment dans le cadre de mélanomes ou de cancers à métastases, ou les maladies bactériennes ou virales induisant une immunosuppression, notamment dans le cadre de la rougeole, du SIDA, du virus de l'herpès ou du cytomégalovirus, ou les vaccins destinés aux personnes présentant un déficit immunitaire primaire ou secondaire.

Les composés selon la présente invention, utilisés dans le cadre de l'activation de la réponse immunitaire, peuvent être administrés par voie intraveineuse, par les voies muqueuses (orales, aériennes, nasales, vaginales), par voie sous-cutanée, intradermique ou épicutanée.

La présente invention concerne également une composition pharmaceutique, caractérisée en ce qu'elle comprend un composé selon la présente invention, pour le traitement de pathologies impliquant l'activation de la réponse immunitaire, lequel composé est présent dans la composition pharmaceutique en quantités telles qu'il peut être administré à raison d'environ 100 ng à environ 5 mg par jour et par individu.

La présente invention concerne également un procédé de préparation sur support solide d'un composé de formule (I) telle que définie ci-dessus, ledit procédé étant caractérisé en ce qu'il comprend les étapes suivantes:
- la formation d'un précurseur linéaire de Y, lequel précurseur est constitué d'un enchaînement d'acides aminés formant une chaîne peptidique en croissance, synthétisée par des cycles successifs de couplage entre des résidus d'acides aminés N-protégés, dont trois portent un groupe de type amine, et la fonction amine de la chaîne peptidique en croissance, et de déprotection, le premier résidu d'acide aminé étant accroché sur un support solide,
- la cyclisation du précurseur linéaire de Y protégé susmentionné,
- le clivage des susdits groupes protecteurs, pour libérer les susdites fonctions amines protégées,
- le couplage des fonctions amines libérées susmentionnées avec un peptide déjà formé ou formé in situ par l'assemblage séquentiel des résidus d'acides aminés correspondant au groupe R_{c}, et
- le clivage de la molécule du support solide, après la suppression de tous les groupements protecteurs présents sur les chaînes latérales fonctionnalisées du groupe R_{c}, afin d'obtenir le composé selon l'invention.

Les composés de l'invention sont obtenus par synthèse sur support solide selon le procédé décrit ci-après. Le groupe Y est d'abord construit sur support solide par synthèse de son précurseur linéaire et cyclisation. Ainsi, un premier résidu d'acide aminé, dont la fonction acide est convenablement protégée (ester d'allyle par exemple), est accroché sur le support par une réaction d'amination réductrice, en utilisant une résine fonctionnalisée par un aldéhyde (résines commerciales). Le précurseur linéaire de Y est ensuite assemblée par cycles successifs de couplage (de manière classique en synthèse de peptide) avec un acide aminé N-protégé (N-Fmoc-Xaa-OH par exemple, Xaa représentant un acide aminé ou un peptide en croissance quelconque) et de déprotection (pipéridine 20% dans du DMF pour le clivage d'un groupe Fmoc). Les techniques de lavage et de filtration de la résine ainsi que de déprotection du groupement Fmoc sont celles couramment utilisées en synthèse peptidique en phase solide. Les trois acides aminés portant une chaîne de type amine sont fonctionnalisés et la fonction amine est protégée par un groupement protecteur orthogonal aux autres (TEOC ou méthyltrityl par exemple). A la fin de l'assemblage, le dernier groupement N-protecteur est clivé (en présence de pipéridine 20% dans du DMF dans le cas d'un groupement Fmoc) et la protection de l'ester C-terminal est clivée. Le précurseur linéaire est alors cyclisé "tête à queue" ("head to tail") en présence d'un réactif de couplage (classique en synthèse peptidique) et d'une base tertiaire comme la DIEA ou la collidine par exemple. La réaction de cyclisation peut être suivie par un test colorimétrique tel que le test de Kaiser (Kaiser et al., 1970). A la fin de la cyclisation, les groupements protecteurs des acides aminés possédant une fonction amine protégée sont clivés et le bras espaceur, convenablement protégé (Fmoc-Ahx (acide 6-amino hexanoïque)-COOH par exemple), est couplé en présence d'un agent de couplage sur les trois fonctions amines libres. A l'issue de ce couplage, le groupement protecteur du bras espaceur est clivé et le groupe R_{c} est assemblé par des méthodes classiques de synthèse peptidique. A la fin de la synthèse et une fois le dernier groupe protecteur enlevé, la molécule est clivée de la résine, par exemple par traitement à l'acide trifluoroacétique, lyophilisée après précipitation à l'éther et purifiée par CLHP préparative en phase inverse sur une colonne C18 par exemple.

La présente invention concerne également des composés marqués par la biotine, répondant à la formule suivante 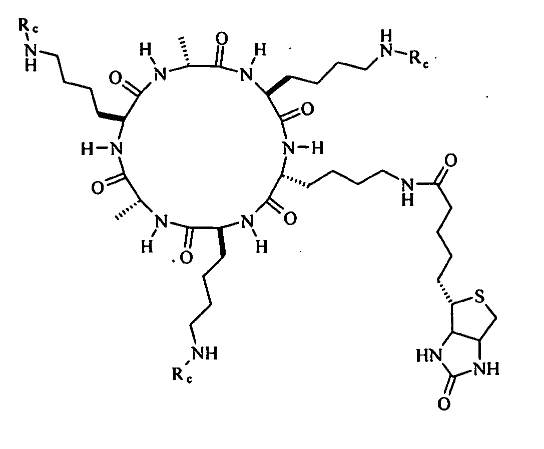 dans laquelle :
- R_{c} représente un groupe de formule H-Xₐ-X_{b}-X_{c}-X_{d}-Xₑ-(X_{f})ᵢ- ou H-X'ₐ-L-X'_{b}-X_{c}-X_{d}-Xₑ-(X_{f})ᵢ-, dans laquelle :
   - i représente 0 ou 1,
   - Xₐ est choisi parmi les résidus d'acides aminés suivants :
      - lysine ;
      - arginine ;
      - ornithine ;
      - les β-acides aminés correspondant à la lysine, l'arginine ou l'ornithine, portant la substitution en position α ou β ;
      - acide tranéxamique ;
      - acide N-méthyl-tranéxamique ;
      - acide 8-amino-3,6-dioxaoctanoïque ;
      - acide 4(pipéridin-4-yl)butanoïque ;
      - acide 3(pipéridin-4-yl)propionique ;
      - N-(4-aminobutyl)-glycine ;
      - NH₂-(CH₂)ₙ-COOH, n variant de 1 à 10 ;
      - NH₂-(CH₂-CH₂-O)ₘ-CH₂CH₂COOH, m variant de 3 à 6 ;
      - 4-carboxyméthyl-pipérazine ;
      - acide 4-(4-aminophényl)butanoïque ;
      - acide 3-(4-aminophényl)propanoïque ;
      - acide 4-aminophénylacétique ;
      - 4-(2aminoéthyl)-1-carboxyméthyl-pipérazine ;
      - acide trans-4-aminocyclohexanecarboxylique ;
      - acide cis-4-aminocyclohexanecarboxylique ;
      - acide cis-4-aminocyclohexane acétique ;
      - acide trans-4-aminocyclohexane acétique ;
      - 4-amino-1-carboxyméthyl pipéridine ;
      - acide 4-aminobenzoïque ;
      - acide 4(2-aminoéthoxy)benzoïque ;
   - X_{b} est choisi parmi les résidus d'acides aminés suivants :
      - D-alanine ;
      - D-valine ;
      - L-proline substituée en position β, γ ou δ ;
      - D-proline substituée ou non en position β, γ ou δ ;
      - acides aminés naturels N-alkylés, le groupe alkyle étant un groupe méthyle, éthyle ou benzyle ;
      - acides aminés dialkylés acycliques de formule suivante : 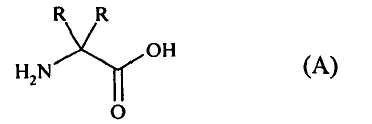 R représentant H, Me, Et, Pr ou Bu ;
      - acides aminés dialkylés cycliques de formule suivante : 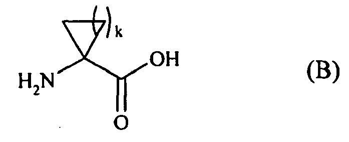 k représentant 1, 2, 3 ou 4 ;
   - X_{c} est choisi parmi les résidus d'acides aminés suivants :
      - tyrosine ;
      - phénylalanine ;
      - 3-nitro-tyrosine ;
      - 4-hydroxyméthyl-phénylalanine ;
      - 3,5-dihydroxy-phénylalanine ;
      - 2,6-diméthyl-tyrosine ;
      - 3,4-dihydroxy-phénylalanine (DOPA) ;
   - X_{d} est choisi parmi les résidus d'acides aminés suivants :
      - tyrosine ;
      - phénylalanine ;
      - 3-nitro-tyrosine ;
      - 4-hydroxyméthyl-phénytataninc ;
      - 3,5-dihydroxy-phénylalanine ;
      - 2,6-diméthyl-tyrosine ;
      - 3,4-dihydroxy-phénylalanine ;
   - Xₑ est choisi parmi les résidus d'acides aminés suivants :
      - NH₂-(CH₂)ₙ-COOH, n variant de 2 à 10 ;
      - NH₂-(CH₂-CH₂-O)ₘ-CH₂CH₂COOH, m variant de 3 à 6 ;
      - acide 8-amino-3,6-dioxaoctanoïque ;
      - acide tranéxamique ;
      - acide N-méthyl-tranéxamique ;
      - acide 4(pipéridin-4-yl)butanoïque ;
      - acide 3(pipéridin-4-yl)propionique ;
      - N-(4-aminobutyl)-glycine ;
      - 4-carboxyméthyl-pipérazine ;
      - acide 4-(4-aminophényl)butanoïque ;
      - acide 3-(4-aminophényl)propanoïque ;
      - acide 4-aminophénylacétique ;
      - 4-(2aminoéthyl)-1-carboxyméthyl-pipérazine ;
      - acide trans-4-aminocyclohexanecarboxylique ;
      - acide cis-4-aminocyclohexanecarboxylique ;
      - acide cis-4-aminocyclohexane acétique ;
      - acide trans-4-aminocyclohexane acétique ;
      - 4-amino-1-carboxyméthyl pipéridine ;
      - acide 4-aminobenzoïque ;
      - acide 4(2-aminoéthoxy)benzoïque ;
   - X_{f} est choisi parmi les résidus d'acides aminés suivants :
      - NH₂-(CH₂)ₙ-COOH, n variant de 2 à 10 ;
      - NH₂-(CH₂-CH₂-O)ₘ-CH₂CH₂COOH, m variant de 3 à 6 ;
      - acide 8-amino-3,6-dioxaoctanoïque ;
      - acide tranéxamique ;
      - acide N-méthyl-tranéxamique ;
      - acide 4(pipéridin-4-yl)butanoïque ;
      - acide 3(pipéridin-4-yl)propionique ;
      - N-(4-aminobutyl)-glycine ;
      - 4-carboxyméthyl-pipérazine ;
      - acide 4-(4-aminophényl)butanoïque ;
      - acide 3-(4-aminophényl)propanoïque ;
      - acide 4-aminophénylacétique ;
      - 4-(2aminoéthyl)-1-carboxyméthyl-pipérazine ;
      - acide trans-4-aminocyclohexanecarboxylique ;
      - acide cis-4-aminocyclohexanecarboxylique ;
      - acide cis-4-aminocyclohexane acétique ;
      - acide trans-4-aminocyclohexane acétique ;
      - 4-amino-1-carboxyméthyl pipéridine ;
      - acide 4-aminobenzoïque ;
      - acide 4(2-aminoéthoxy)benzoïque ;
   - L- représente une liaison de nature pseudopeptidique entre les résidus X'ₐ et X'_{b}, choisie notamment dans la liste ci-dessous :
- L- = -ψ(CH₂CH₂)- ; -ψ(CH(Fₖ)=CH(Fₖ'))-; -ψ(CH₂NH)-; -ψ(NHCO)-; -ψ(NHCONH)- ; -ψ(CO-O)- ; -ψ(CS-NH)- ; -ψ(CH(OH)-CH(OH) )-; -ψ(S-CH₂)- ; -ψ(CH₂-S)- ; -ψ(CH(CN)-CH₂)- ; -ψ(CH(OH))- ; -ψ(COCH₂)-; -ψ(CH(OH)CH₂)- ; -ψ(CH(OH)CH₂NH)-; -ψ(CH₂)-; -ψ(CH(Fₖ))-; -ψ(CH₂O)- ; -ψ(CH₂-NHCONH)- ; -ψ(CH(Fₖ)NHCONFₖ')- ; -ψ(CH₂-CONH)- ; -ψ(CH(Fₖ)CONH)- ; -ψ(CH(Fₖ)CH(Fₖ')CONH)- ;
   ou -ψ(CH₂N)- ; -ψ(NHCON)- ; -ψ(CS-N)- ; -ψ(CH(OH)CH₂N)- ; -ψ(CH₂-NHCON)- ; -ψ(CH₂-CON)-; -ψ(CH(Fₖ)CON)- ; -ψ(CH(Fₖ)CH(Fₖ')CON)-lorsque X'_{b} représente la chaîne latérale d'une proline,
   Fₖ et Fₖ' représentant, indépendamment l'un de l'autre, un hydrogène, un halogène, un groupe alkyle de 1 à 20 atomes de carbone, ou un groupe aryle dont la structure du cycle contient de 5 à 20 atomes de carbone,
   - X'ₐ représente la chaîne latérale de la lysine, de l'arginine ou de l'ornithine ; et
   - X'_{b} représente la chaîne latérale de l'un des résidus d'acides aminés suivants :
      - glycine ;
      - asparagine ;
      - D-alanine ;
      - D-valine ;
      - L-proline substituée ou non en position β, γ ou δ ;
      - D-proline substituée ou non en position β, γ ou δ ;
      - acides aminés naturels N-alkylés, le groupe alkyle étant un groupe méthyle, éthyle ou benzyle ;
      - acides aminés dialkylés acycliques de formule suivante : 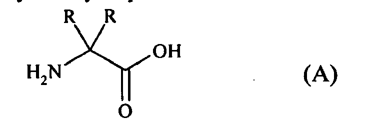 R représentant H, Me, Et, Pr ou Bu ;
      - acides aminés dialkylés cycliques de formule suivante : 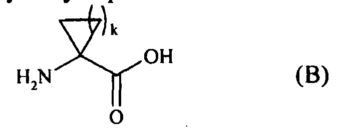 k représentant 1, 2, 3 ou 4.

### DESCRIPTION DES FIGURES

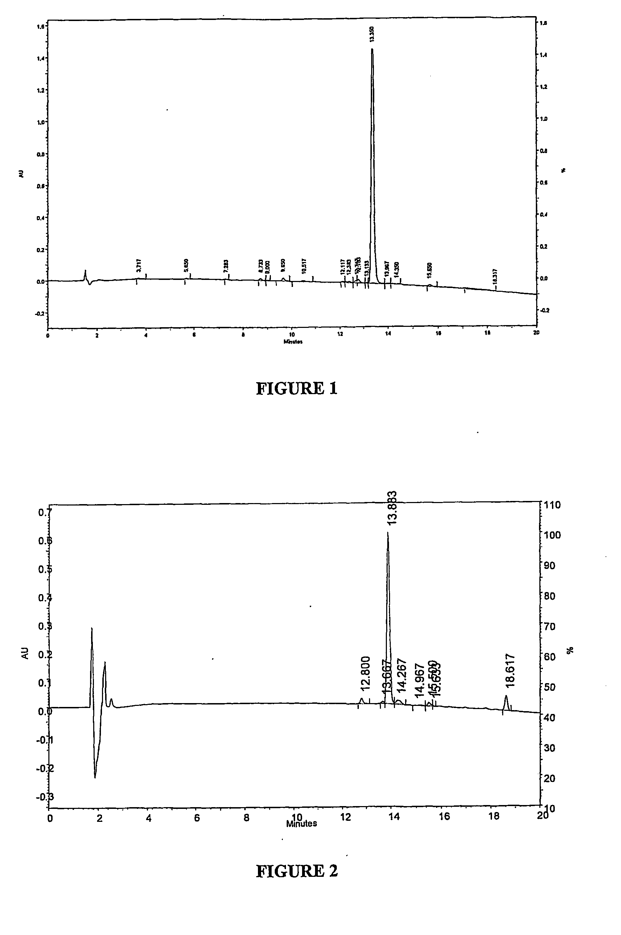

La Figure 1 représente le chromatogramme CLHP du pentapeptide protégé (1) de l'exemple 5.
La Figure 2 représente le chromatogramme CLHP de l'hexapeptide non cyclisé (2) de l'exemple 5.

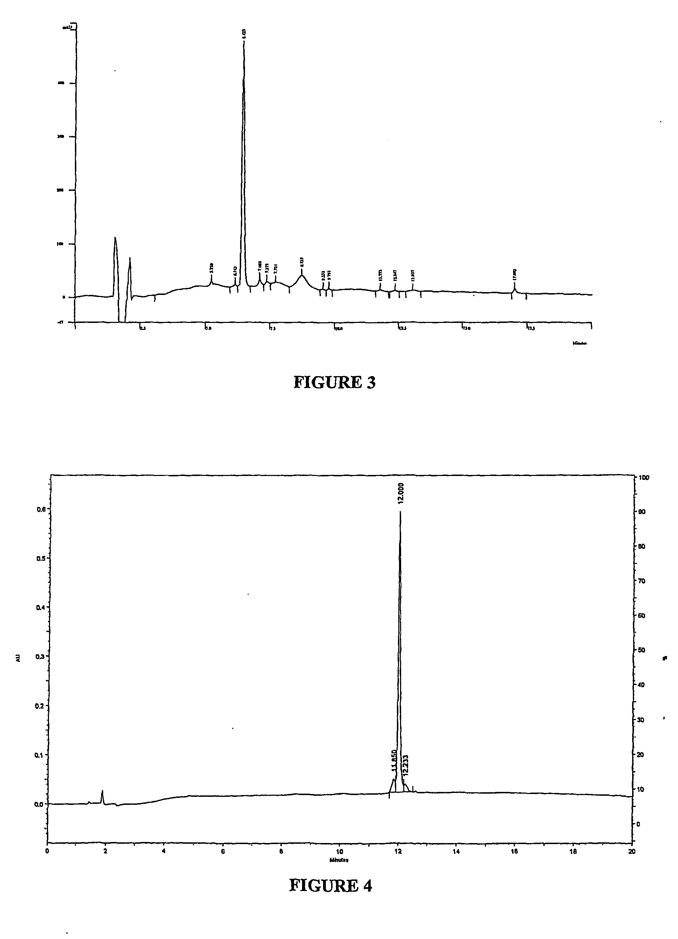

La Figure 3 représente le chromatogramme CLHP de l'hexapeptide cyclisé et déprotégé (3) de l'exemple 5.
La Figure 4 représente le chromatogramme CLHP du composé A de l'exemple 5 (L41).

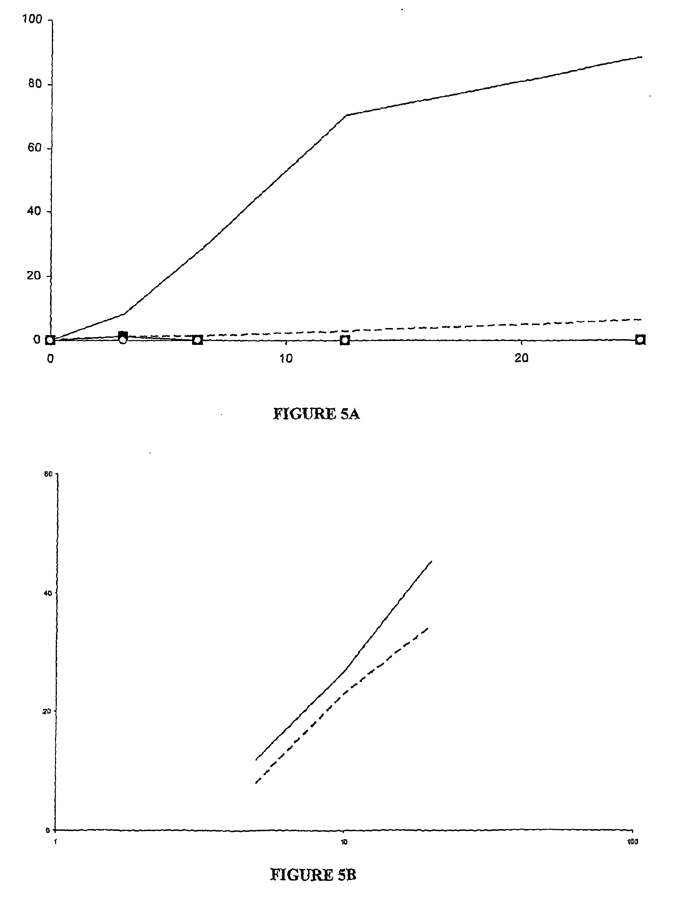

La Figure 5A représente le pourcentage d'apoptose des cellules BL41, induite par le ligand L36 incubé pendant 16h, en cytométrie de flux après un marquage au DiOC₆. La courbe en trait plein correspond à la dose-réponse de l'apoptose induite par le ligand L36. La courbe en traits pointillés correspond à la dose-réponse de l'apoptose induite par le ligand L36 sur la lignée cellulaire Jurkat qui n'exprime par CD40. La courbe en trait plein avec les carrés noirs correspond à la dose-réponse de l'apoptose induite par le pentapeptide contrôle (L37)(H-Lys-(D)Pro-Tyr-Tyr-Ahx-OH) sur BL41 et la courbe en trait plein avec les ronds blancs correspond à la dose-réponse de l'apoptose induite par le pentapeptide contrôle (L37) sur Jurkat.
La Figure 5B représente le pourcentage d'apoptose des cellules BL41, induite par les ligands L4 et L41 incubés pendant 16h, en cytométrie de flux après un marquage au DiOC₆. La courbe en trait plein correspond à la dose-réponse de l'apoptose induite par le ligand L4. La courbe en trait pointillé correspond à la dose-réponse de l'apoptose induite par le ligand L41.

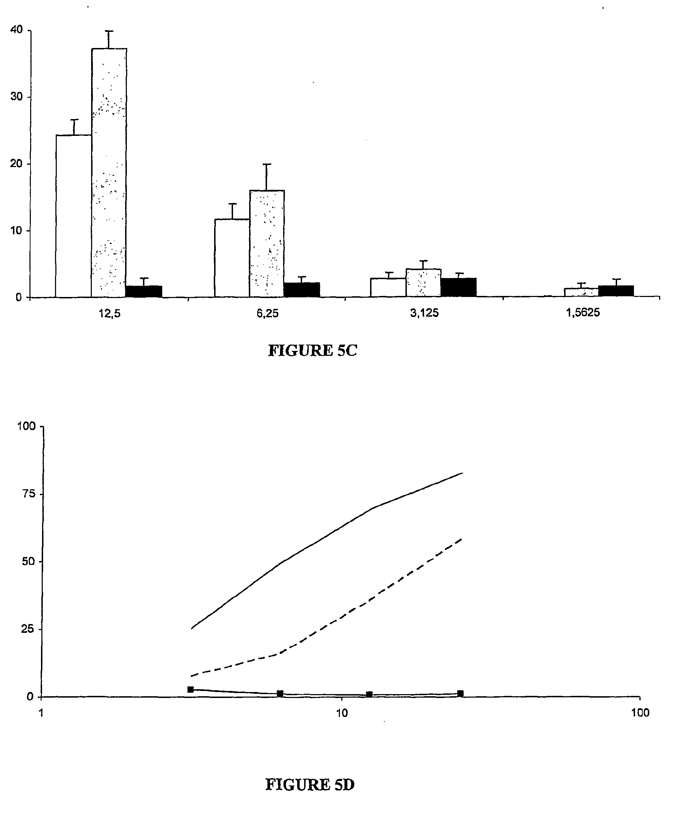

La Figure 5C représente le pourcentage d'apoptose des cellules BL41, induite par les ligands L4 et L40 incubés pendant 16h, en cytométrie de flux après un marquage au DiOC₆. Les valeurs correspondent aux moyennes plus ou moins les écart-moyens de 3 expériences indépendantes sur les cellules BL41. Les histogrammes blancs correspondent à la dose-réponse de l'apoptose induite par L4, les histogrammes noirs correspondent à la dose-réponse de l'apoptose induite par L45 et les histogrammes gris correspondent à la dose-réponse de l'apoptose induite par L40. Le peptide contrôle (L45)(H-Lys-Pro-Tyr-Tyr-Ahx-OH) dans lequel la D-Proline est remplacé par la L-Proline n'a pas d'activité.
La Figure 5D représente le pourcentage d'apoptose des cellules Raji, induite par les ligands L4, L43 et L44, incubés pendant 16h, en cytométrie de flux après un marquage au DiOC₆. La courbe en trait plein correspond à la dose-réponse de l'apoptose induite par L4, la courbe en trait pointillé correspond à la dose-réponse de l'apoptose induite par L43 et la courbe avec les carrés noirs correspond à la dose-réponse de l'apoptose induite par L44.

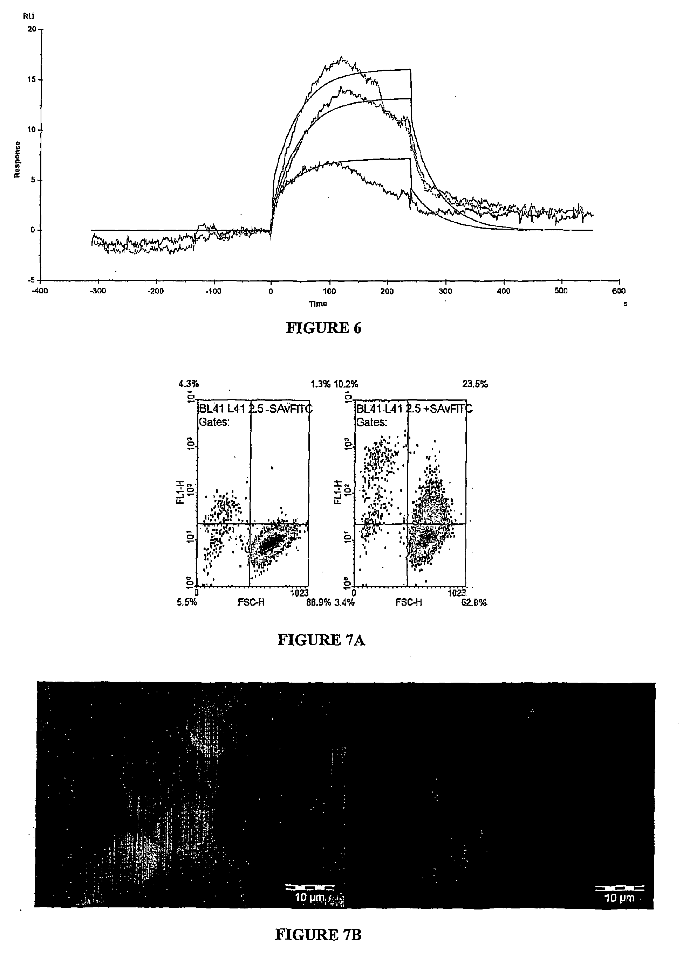

La Figure 6 représente des sensorgrammes mesurés pour la cinétique d'interaction directe du ligand L40 avec CD40 (réponse en fonction du temps). Ici, le ligand L40 est injecté aux concentrations 25, 100 et 200 nM à un flux de 30 µl/min pendant 4 minutes.
La Figure 7A représente le marquage en cytométrie de flux des cellules BL41 à l'aide du ligand biotinylé L41 révélé par une streptavidine-FITC. L'axe des ordonnées correspond à l'intensité de fluorescence (FL1-H) des cellules marquées avec le ligand L41 puis la streptavidine, l'axe des abscisses correspond à la taille des cellules (FSC-H). La courbe de densité de gauche correspond aux cellules marquées avec le ligand biotinylé mais sans la streptavidine, celle de droite correspond aux cellules marquées avec le ligand biotinylé puis la streptavidine couplée au fluorochrome FITC. La coloration du gris au noir indique une densité en cellules à un point d'abscisse FSC et d'ordonnée FL1 décroissante.
La Figure 7B représente le marquage en microscopie à fluorescence des cellules BL41 à l'aide du ligand biotinylé L41 et un anticorps dirigé contre le CD40, révélés respectivement par une streptavidine Alexa⁵⁴⁶ et un anticorps de chèvre dirigé contre la partie Fc des anticorps de souris couplé au fluorochrome Alexa⁴⁸⁸. La coloration grise de la partie gauche de la Figure correspond au marquage de l'anticorps dirigé contre CD40 et la coloration grise de la partie droite de la Figure au marquage du ligand biotinylé L41.

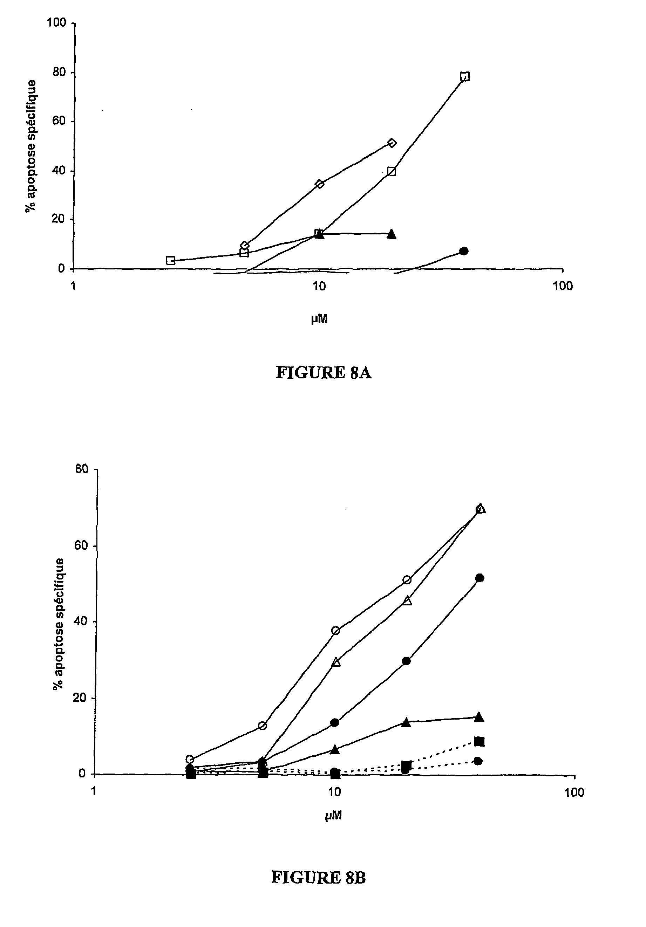

La figure 8A représente le pourcentage d'apoptose spécifique des cellules du lymphome de Burkitt Raji (CD40+) et de la leucémie T Jurkat (CD40-) à 5 × 10⁵ cellules/ml après 16 heures de culture en présence de différentes concentrations des ligands L4 et L62. La courbe avec les losanges blancs correspond à la dose-réponse de l'apoptose induite par L4 sur les cellules Raji ; la courbe avec les carrés blancs correspond à la dose-réponse de l'apoptose induite par L62 sur les cellules Raji ; la courbe avec les triangles noirs correspond à la dose-réponse de l'apoptose induite par L4 sur les cellules Jurkat et la courbe avec les ronds noirs correspond à la dose-réponse de l'apoptose induite par L62 sur les cellules Jurkat.
La figure 8B représente le pourcentage d'apoptose spécifique des cellules Raji, Jurkat et du lymphome de Burkitt BL41 (CD40+) traitées avec les différents ligands L62 et L40 dans les mêmes conditions que dans la figure 8A lors d'une expérience indépendante. La courbe en trait plein avec les ronds blancs correspond à la dose-réponse de l'apoptose induite par L40 sur les cellules Raji ; la courbe en trait plein avec les triangles blancs correspond à la dose-réponse de l'apoptose induite par L62 sur les cellules Raji ; la courbe en trait plein avec les ronds noirs correspond à la dose-réponse de l'apoptose induite par L40 sur les cellules BL41 ; la courbe en trait plein avec les triangles noirs correspond à la dose-réponse de l'apoptose induite par L62 sur les cellules BL41 ; la courbe en trait pointillé avec les carrés noirs correspond à la dose-réponse de l'apoptose induite par L40 sur les cellules Jurkat et la courbe en trait pointillé avec les ronds noirs correspond à la dose-réponse de l'apoptose induite par L62 sur les cellules Jurkat.

### Liste des abréviations :

- ACN: acétonitrile
- AcOH: acide acétique
- BOP: benzotriazol-1-yloxy-tris(diméthylamino)phosphonium hexafluorophosphate
- CCM: chromatographie sur couche mince
- CMA: mélange de chloroforme, de méthanol et d'acide acétique
- CLHP: chromatographie liquide haute performance
- DBU: 1,8-diazabicyclo[5,4,0]undécen-7-ène
- DCM: dichlorométhane
- DEA: diéthylamine
- DIC: diisopropylcarbodiimide
- DIEA: diisopropyléthylamine
- DiOC₆: iodure de 3,3'-dihexyloxacarbocyanine
- DMAP: 4-diméthyl aminopyridine
- DMF: diméthylformamide
- EDC: méthiodure de 1-éthyl-3-(3-diméthylaminopropyl)carbodiimide
- HATU: O-(7-azabenzotriazol-1-yl)-1,1,3,3-tétraméthyluranium hexafluorophosphate
- HOAt: 7-azabenzotriazole
- HoBt: 1-hydroxybenzotriazole
- IpOH: isopropanol
- NMM: N-méthyle morpholine
- OAll: -O-allyle
- OMtt: -O-méthyltrityle
- TFA: acide trifluoroacétique

### PARTIE EXPÉRIMENTALE

### EXEMPLE 1- SYNTHÈSE DES PENTAPEPTIDES PROTÉGÉS ET DÉPROTÉGÉS

### I - Pentapeptide protégé L44' et pentapeptide déprotégé L44

Le pentapeptide déprotégé L44 répond à la formule suivante :

H-Ahx-(D)Pro-Tyr-Tyr-Ahx-OH

### 1) Préparation du pentapeptide protégé Boc-Ahx-(D)Pro-Tyr(tBu)-Tyr(tBu)-Ahx-OH (L44')

1,4 g (1 éq.) de résine 2-chlorotritylchloride **(R1-A)** (1,3 mmol/g) est placé dans une seringue et lavé deux fois sous agitation au DCM distillé. Une solution de 1,3 g (2 éq.) d'acide Fmoc-6-aminocaproïque et de 1,9 ml (6 éq.) de DIEA dans le DCM est alors ajouté à la résine. Après trois heures d'agitation, le mélange est filtré et lavé au DCM. La résine est ensuite mise à regonfler dans du méthanol pendant une heure sous agitation. Après quoi elle est filtrée et lavée au DMF, IpOH, DCM, éther. **R-2A** est obtenue après séchage.

**R-2A** obtenue précédemment est mis sous agitation dans une solution 25% pipéridine/DMF pendant 20 minutes. La résine est ensuite filtrée pour donner **R3-A** et la solution est récoltée. Un test UV de la solution récoltée permet de déterminer la substitution de la résine **R-2A** (0,6 mmol/g).

### Procédure générale du couplage d'un acide aminé :

Une solution de 5 éq. de Fmoc-Xaa-OH, 5 éq. de BOP, et 5 éq. de HoBt dans du DMF est ajouté à 1 éq. de résine **R2-A.** 15 éq. de DIEA sont ensuite ajoutés au système, puis l'ensemble est agité pendant 30 minutes. Cette opération est répétée une seconde fois. Le couplage est vérifié par un test à la ninhydrine. La déprotection du groupement Fmoc est réalisée dans une solution 25% pipéridine/DMF sous agitation pendant 30 minutes. Un deuxième couplage peut à nouveau être réalisé.

La résine **R-4B** est obtenue après l'incorporation des différents acides aminés désirés pour synthétiser le peptide.

Un mélange de 1,1,1,3,3,3-hexafluoro-2-propanol et de DCM (60/40) est ajouté à la résine **R4-B.** La seringue est agitée pendant 2 heures puis est filtrée et rincée plusieurs fois au DCM. Cette opération est répétée une seconde fois. Les solutions sont assemblées et évaporées pour donner le peptide protégé **L44'.**

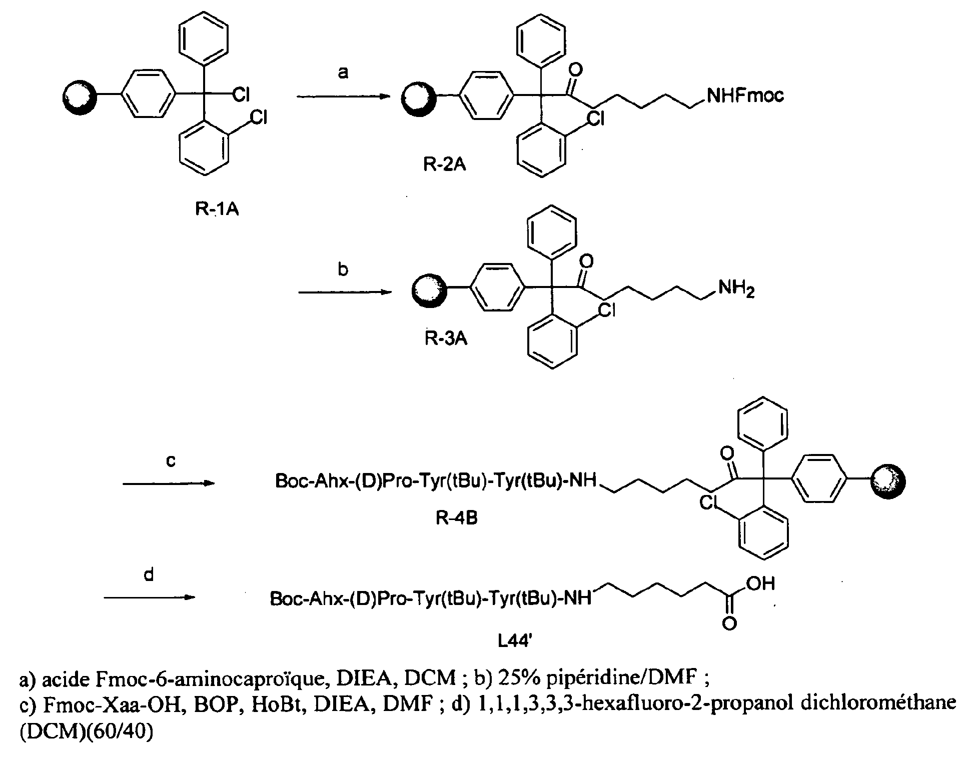

Le peptide **L44'** est caractérisé par :
**CLHP :** *t*_{R} 14,909 min (gradient linéaire, 30-100% B, 20 min)
**MALDI-TOF :** calculé pour C₄₈H₇₃N₅O₁₀Na [M+Na⁺] : 903.12; observé: 902,57.

### 2) Préparation du pentapeptide déprotégé L44 : NHZ-Ahx (D)Pro-Tyr-Tyr Ahx OH

Le peptide **L44'** est traité au TFA en présence d'eau. Après 30 minutes d'agitation, la solution est précipitée à l'éther, filtré et lyophilisée pour donner **L44.**

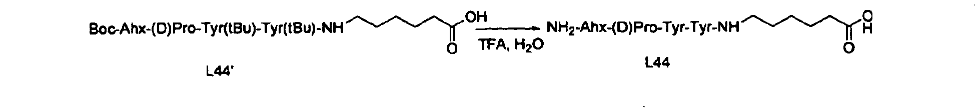

Ce peptide L 44 est caractérisé par :
**CLHP :** t_{R} 10,272 min (gradient linéaire, 5-65% B, 20 min)
**MALDI-TOF :** calculé pour C₃₅H₅₀N₅O₈ [M+H]⁺ : 667,8 ; observé : 668,2 ; calculé pour C₃₅H₄₉N₅O₈Na [M+Na⁺] : 690,8 ; observé : 690,23.

### II - Pentapeptide protégé L37' et pentapeptide déprotégé L37

Le pentapeptide déprotégé L37 répond à la formule suivante :
H-Lys-(D)Pro-Tyr-Tyr-Ahx-OH

### 1) Préparation du pentapeptide protégé, L37' : Boc-Lys(Boc)-(D)Pro-Tyr(tBu)-Tyr(tBu)-Ahx-OH

1,4 g (1 éq.) de résine 2-chlorotritylchloride **(R1-A)**(1,3mmol/g) est placé dans une seringue et lavé deux fois sous agitation au DCM distillé. Une solution de 1,3 g (2 éq.) d'acide Fmoc-6-aminocaproïque et de 1,9 ml (6 éq.) de DIEA dans le DCM est alors ajouté à la résine. Après trois heures d'agitation, le mélange est filtré et lavé au DCM. La résine est ensuite mise à regonfler dans méthanol pendant une heure sous agitation. Après quoi elle est filtrée et lavée au DMF, IpOH, DCM, éther. **R-2A** est obtenu après séchage.

**R-2A** obtenu précédemment est mis sous agitation dans une solution 25% pipéridine/DMF pendant 20 minutes. La résine est ensuite filtrée pour donner **R3-A** et la solution est récoltée. Un test UV de la solution récoltée permet de déterminer la substitution de la résine **R-2A** (0,6 mmol/g).

### Procédure générale du couplage d'un acide aminé:

Une solution de 5eq. de FmocXaaOH, 5eq. de BOP, et 5eq. de HoBt dans du DMF est ajouté à 1eq. de résine **R2-A.**15eq. de DIEA est ensuite ajouté au système, puis l'ensemble est agité pendant 30 minutes. Cette opération est répétée une seconde fois. Le couplage est vérifié par un test à la ninhydrine. La déprotection du groupement fmoc est réalisé dans une solution 25% pipéridine/DMF sous agitation pendant 30minutes. Un deuxième couplage peut à nouveau être réalisé.

La résine **R-4A** est obtenue après l'incorporation des différents acides aminés désirés pour synthétiser le peptide.

Un mélange de 1,1,1,3,3,3-hexafluoro-2-propanol et de DCM (60/40) est ajouté à la résine **R4-A.** La seringue est agitée pendant 2 heures puis est filtrée et rincée plusieurs fois au DCM. Cette opération est répétée une seconde fois. Les solutions sont assemblées et évaporées pour donner le peptide **L37'.**

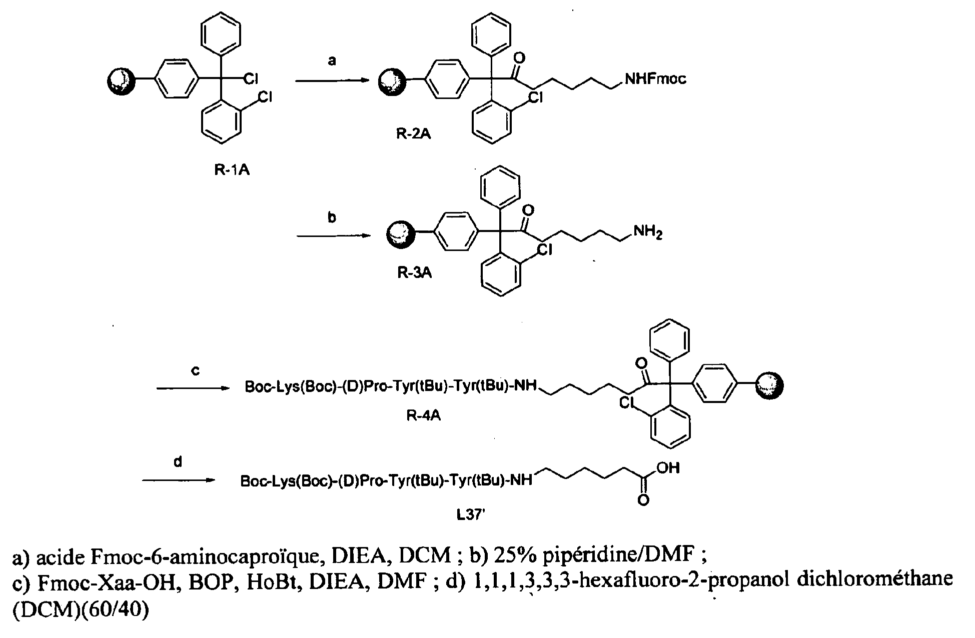

Le peptide **L37'** est caractérisé par :
**CLHP :** *t*_{R} 15,605 min (gradient linéaire, 30-100% B, 20 min)
**MALDI-TOF :** calculé pour C₅₃H₈₂N₆O₁₂Na⁺ [M+Na⁺] : 1018,25 ; observé : 1018,40 ; calculé pour C₅₃H₈₂N₆O₁₂K⁺ [M+K+] : 1034,25 ; observé : 1034,64.

### 2) Préparation du pentapeptide déprotégé L37 : NH₂-Lys-(D)Pro-Tyr-Tyr-Ahx-OH

Le peptide **L37'** est traité au TFA en présence d'eau. Après 30 minutes d'agitation, la solution est précipitée à l'éther, filtré et lyophilisée pour donner **L37.**

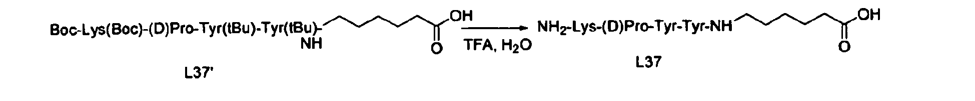

Ce peptide **L37** est caractérisé par :
**CLHP :** *t*_{R} 9,065 min (gradient linéaire, 5-65% B, 20 min)
**MALDI-TOF** : calculé pour C₃₅H₅₁N₆O₈ [M+H⁺] : 683,81 ; observé : 682,91 ; calculé pour C₃₅H₅₀N₆O₈Na [M+Na⁺] : 705,81 ; observé : 705,22 ; calculé pour C₃₅H₅₀N₆O₈K⁺ [M+K⁺] : 721,81 ; observé : 721,67.

Le pentapeptide déprotégé L45 est synthétisé selon le même protocole que celui utilisé pour la synthèse de L37 et L44.

### III - Pentapeptide protégé L63' et pentapeptide déprotégé L63

Le pentapeptide déprotégé L63 répond à la formule suivante :
H-Lys-ψ(CH₂N)_{D}Pro-Tyr-Tyr-Ahx-OH

### 1) Préparation du pentapeptide protégé Boc-Lys(Boc) ψ(CH₂N)_{D}Pro-Tyr(tBu)-Tyr(tBu)-Ahx-OH (L63')

0,3g (1 éq.) de résine 2-chlorotritylchloride **(R1-A)**(1,6 mmol/g) est placé dans une seringue et lavé deux fois sous agitation au DCM distillé. Une solution de 340 mg (2 éq.) d'acide Fmoc-6-aminocaproïque et de 478 µl (6 éq.) de DIEA dans le DCM est alors ajouté à la résine. Après trois heures d'agitation, le mélange est filtré et lavé au DCM. La résine est ensuite mise à regonfler dans du méthanol pendant une heure sous agitation. Après quoi elle est filtrée et lavée au DMF, IpOH, DCM, éther. **R-2A** est obtenu après séchage.

**R-2A** obtenu précédemment est mis sous agitation dans une solution 25% pipéridine/DMF pendant 20 minutes. La résine est ensuite filtrée pour donner **R3-A** et la solution est récoltée. Un test UV de la solution récoltée permet de déterminer la substitution de la résine **R-2A** (0,44 mmol/g).

### Procédure générale du couplage d'un acide aminé :

Une solution de 5 éq. de Fmoc-Xaa-OH, 5 éq. de BOP, et de 5 éq. de HoBt dans du DMF est ajouté à 1 éq. de résine **R2-A.** 15 éq. de DIEA sont ensuite ajoutés au système, puis l'ensemble est agité pendant 30 minutes. Cette opération est répétée une seconde fois. Le couplage est vérifié par un test à la ninhydrine. La déprotection du groupement Fmoc est réalisée dans une solution 25% pipéridine /DMF sous agitation pendant 30 minutes. Un deuxième couplage peut à nouveau être réalisé.

La résine **R-4C** est obtenue après l'incorporation du résidu D-Proline.

Une solution de Boc-Lys(Boc)-H (3 éq) dans du DMF est ajoutée à 1 éq de résine **R-4C.** NaBH₃CN solubilisé dans DMF est ensuite ajouté au système, puis l'ensemble est agité pendant 1 heure. Cette opération est répétée une seconde fois. Le couplage est vérifié par un test à la ninhydrine. La résine **R-4D** est obtenue.

Un mélange de 1,1,1,3,3,3-hexafluoro-2-propanol et de DCM (60/40) est ajouté à la résine **R4-D.** La seringue est agitée pendant 2 heures puis est filtrée et rincée plusieurs fois au DCM. Cette opération est répétée une seconde fois au DCM. Les solutions sont assemblées et évaporées pour donner le peptide protégé **L63'.**

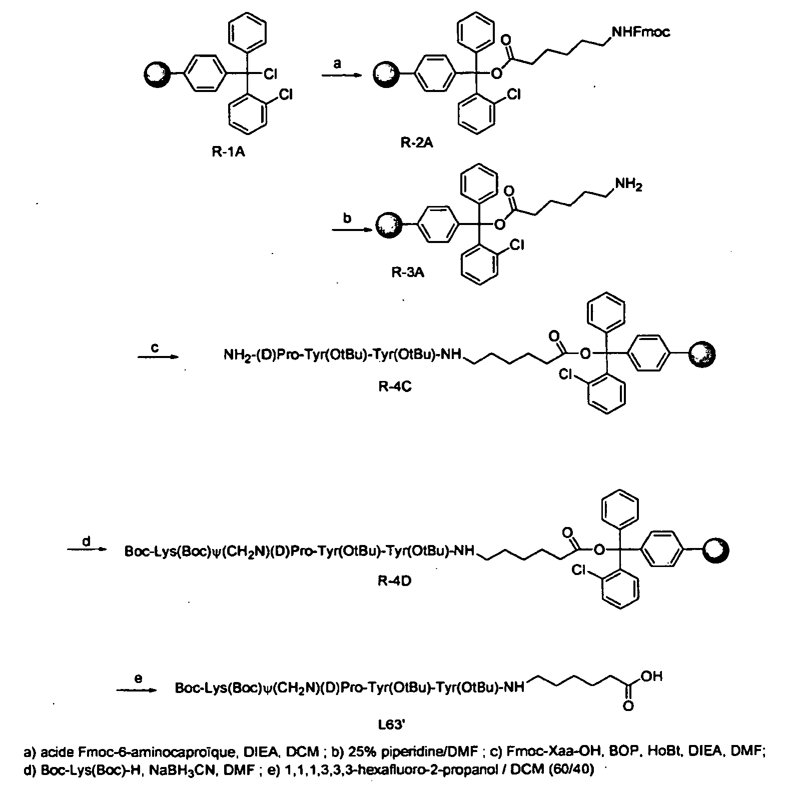

Le peptide **L63'** est caractérisé par :
**CLHP :** *t*_{R} 13,77. min (gradient linéaire, 30-100% B, 20 min)
**MALDI-TOF:** calculé pour C₅₃H₈₄N₆O₁₁H⁺ : 982,28 ; observé [M+H⁺] = 982,38 ; calculé pour C₅₃H₈₄N₆O₁₁Na⁺ : 1004,28 ; observé 1004,44 ; calculé pour C₅₃H₈₄N₆O₁₁K⁺ : 1020,28 ; observé 1020,45.

### 2) Préparation du pentapeptide protégé NH2-Lysψ(CH₂N)_{D}Pro-Tyr-Tyr-Alix-OH (L63)

Le peptide **L63** est traité au TFA en présence d'eau. Après 30 minutes d'agitation, la solution est précipitée à l'éther, filtrée et lyophilisée pour donner **L63**.

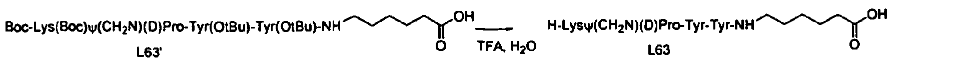

Ce peptide L63 est caractérisé par :
**CLHP** : *t*_{R} 9,77 min (gradient linéaire, 5-65% B, 20 min)
**MALDI-TOF** : calculé pour C₃₅H₅₂N₆O₇Na⁺ : 691,82; observé 689,68 ; calculé pour C₃₅H₅₂N₆O₇K⁺ : 707,82 ; observé 707,15.

### IV - Pentapeptide protégé L69' et pentapeptide déprotégé L69

Le pentapeptide déprotégé **L69** répond à la formule suivante :
H-Lys-Gly-Dopa-Tyr-Ahx-OH

### 1) Préparation du pentapeptide protégé Boc-Lys(Boc)-Gly-Dopa(Acetonid)-Tyr(tBu)-Ahx-OH (L69')

0,3 mg (1 éq.) de résine 2-chlorotritylchloride **(R1-A)**(1,6 mmol/g) est placé dans une seringue et lavé deux fois sous agitation au DCM distillé. Une solution de 340 mg (2 éq.) d'acide Fmoc-6-aminocaproïque et de 478 µl (6 éq.) de DIEA dans le DCM est alors ajouté à la résine. Après trois heures d'agitation, le mélange est filtré et lavé au DCM. La résine est ensuite mise à regonfler dans du méthanol pendant une heure sous agitation. Après quoi elle est filtrée et lavée au DMF, IpOH, DCM, éther. **R-2A** est obtenu après séchage.

**R-2A** obtenu précédemment est mis sous agitation dans une solution 25% pipéridine/DMF pendant 20 minutes. La résine est ensuite filtrée pour donner R3-A et la solution est récoltée. Un test UV de la solution récoltée permet de déterminer la substitution de la résine **R-2A** (0,44 mmol/g).

### Procédure générale du couplage d'un acide aminé :

Une solution de 5 éq. de Fmoc-Xaa-OH, 5 éq. de BOP, et de 5 éq. de HoBt dans du DMF est ajouté à 1 éq. de résine **R2-A.** 15 éq. de DIEA sont ensuite ajoutés au système, puis l'ensemble est agité pendant 30 minutes. Cette opération est répétée une seconde fois. Le couplage est vérifié par un test à la ninhydrine. La déprotection du groupement Fmoc est réalisée dans une solution 25% pipéridine /DMF sous agitation pendant 30 minutes. Un deuxième couplage peut à nouveau être réalisé.

La résine **R-4E** est obtenue près l'incorporation des différents acides aminés désirés pour synthétiser le peptide.

Un mélange de 1,1,1,3,3,3-hexafluoro-2-propanol et de DCM (60/40) est ajouté à la résine **R4-E.** La seringue est agitée pendant 2 heures puis est filtrée et rincée plusieurs fois au DCM. Cette opération est répétée une seconde fois au DCM. Les solutions sont assemblées et évaporées pour donner le peptide protégé **L69'.**

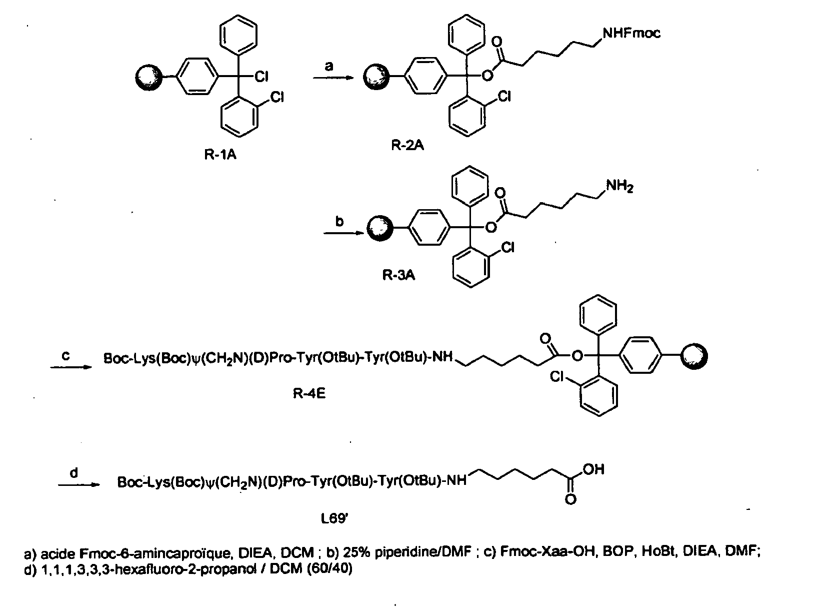

Le peptide **L69'** est caractérisé par :
**CLHP** : *t*_{R} 12,83 min (gradient linéaire, 30-100% B, 20 min)
**MALDI-TOF :** calculé pour C₄₉H₇₄N₆O₁₃Na⁺ : 978,15; observé 979,55 ; calculé pour C₄₉H₇₄N₆O₁₃K⁺ : 994,15 ; observé 995,89.

### 2) Préparation du pentapeptide protégé H-Lys-Gly-Dopa-Tyr-Ahx-OH (L69)

Le peptide **L69** est traité au TFA en présence d'eau. Après 30 minutes d'agitation, la solution est précipitée à l'éther, filtrée et lyophilisée pour donner **L69**.

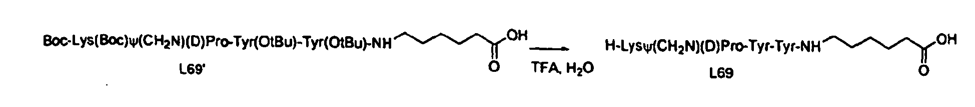

Ce peptide L69 est caractérisé par :
**CLHP :** *t*_{R} 9,18 min (gradient linéaire, 5-65% B, 20 min)
**MALDI-TOF :** calculé pour C₃₂H₄₆N₆O₉K⁺: 697,74; observé 697,40.

### EXEMPLE 2 - PRÉPARATION DU COMPOSÉ DE FORMULE (V-b) (L36)

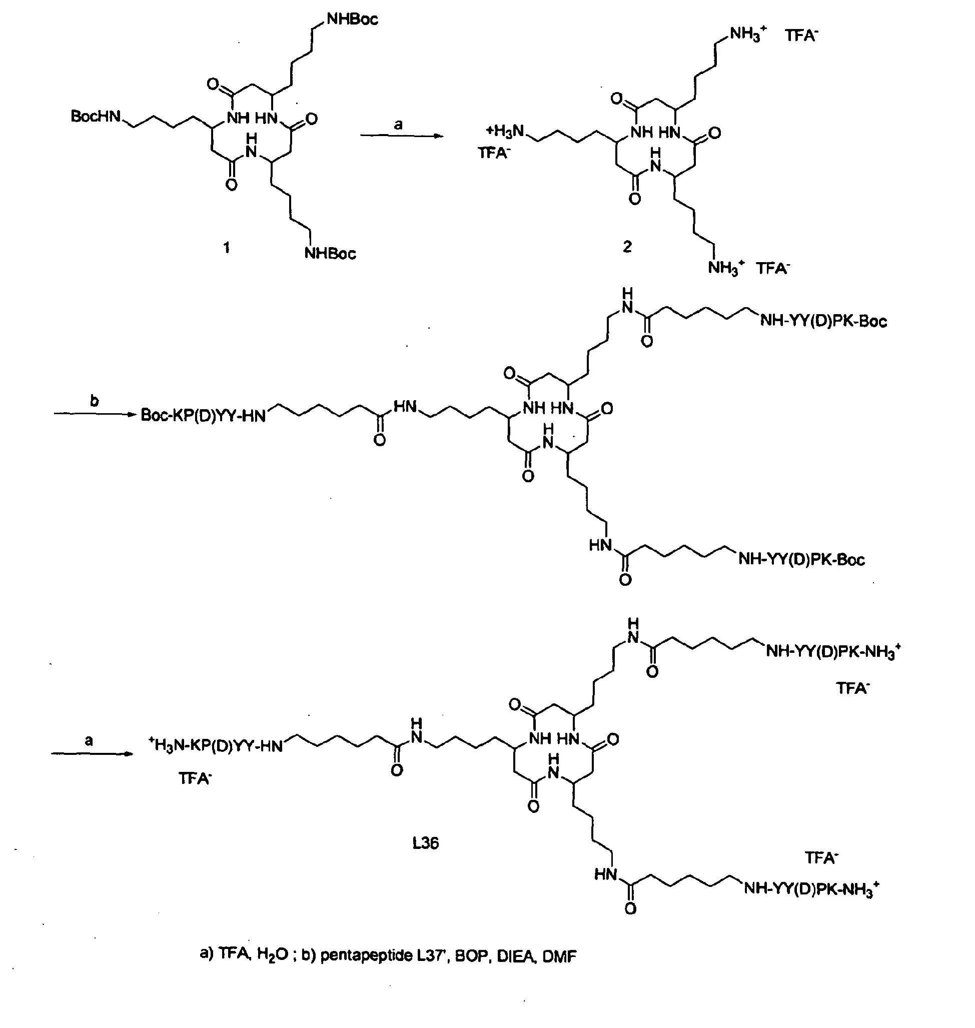

13 mg (1 éq.) de cycle protégé 1 est traité au TFA. Après 30 minutes d'agitation, la solution est évaporée avec du cyclohexane pour donner le composé **2**.

Le composé **2** est repris dans le DMF. 56 mg (3,3 éq.) de pentapeptide **L37'** Boc-Lys(Boc)-(D)Pro-Tyr(tBu)-Tyr(tBu)-Ahx-OH (voir ci-dessus), 25 mg (3,3 éq.) de BOP et 30 µL (10 éq.) de DIEA est ajouté. La réaction est agitée pendant 25 heures à température ambiante, puis est précipitée au NaHCO₃ (aq.). Après filtration, le précipité est lavé successivement à H₂O, KHSO₄ 1N, H₂O et AcOEt.

Le solide formé est alors traité au TFA en présence d'eau. Après 30 minutes d'agitation, la solution est précipitée à l'éther et filtrée pour donner 35 mg de ligand **L36** (rendement brut de réaction 86%). 11 mg de **L36** pur sont obtenus après purification par RP-CLHP (rendement après purification 27%).

Le ligand **L36** est caractérisé par :
**HLPC :** *t*_{R} 10,115 min (gradient linéaire, 5-65% B, 20 min)
**MALDI-TOF :** calculé pour C₁₂₆H₁₈₇N₂₄O₂₄ [M+H⁺] : 2422 ; observé : 2420,68.

### EXEMPLE 3 - PRÉPARATION DU MACROCYCLE POUR L40 ET L43

### Schéma de synthèse

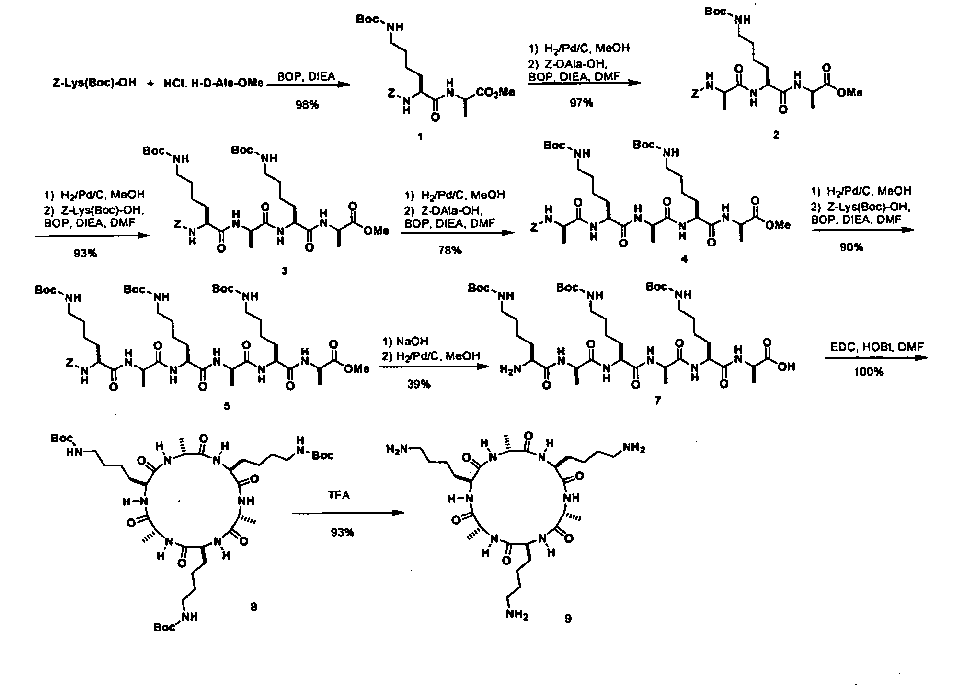

### Composé 1

On a dissous Z-Lys(Boc)-OH (5,99 g; 15,75 mmol) dans du DMF (50 ml) contenant HCl, H₂N-(D)Ala-OMe (2,09 g ; 15 mmol), BOP (6,96 g ; 15,75 mmol) et on a ajouté de la DIEA (7,6 ml ; 45 mmol) à cette solution. Le mélange réactionnel est agité pendant 2 heures à température ambiante. Ensuite on ajoute une solution de bicarbonate de sodium saturée (500 ml) sous agitation, puis on ajoute de l'acétate d'éthyle (200 ml). La phase organique est lavée avec une solution de bicarbonate de sodium saturée (2 × 100 ml), de l'eau (2 × 100 ml), une solution aqueuse de sulfate d'hydrogène de potassium 1 M (2 × 100 ml), de l'eau, de la saumure (1 × 100 ml), on sèche l'ensemble sur du sulfate de sodium et on le concentre sous vide pour obtenir un solide blanc. Rendement : 98% (6,85 g) ; CLHP : t_{R} = 9,3 min, pureté >97%, 30-100 en 20 min.

### Composé 2

Le composé 1 (4,66g ; 10 mmol) est dissous dans du MeOH (100 ml) avec HCl (10 mmol) à température ambiante et hydrogéné en présence d'un catalyseur 10% Pd/C. Après 3 heures, on enlève le catalyseur par filtration et le filtrat est concentré sous vide pour obtenir un solide blanc. Rendement : 100% (3,71 g), pureté CLHP >97%, CCM Rf 0,3 CMA 60/10/5.

Le sel HCl (3,68 g ; 10 mmol) est dissous dans du DMF (20 ml) contenant du Z-(D)Ala-OH (2,34 g ; 10,5 mmol) et du BOP (4,64g ; 10,5 mmol). On ajoute à cette solution de la DIEA (3,4 ml ; 20 mmol) et le mélange réactionnel est agité pendant 4 heures à température ambiante. Ensuite, on ajoute une solution de bicarbonate de sodium saturée (500 ml) sous agitation, puis on ajoute de l'acétate d'éthyle (200 ml). La phase organique est lavée avec une solution de bicarbonate de sodium saturée (2 × 100 ml), de l'eau (2 × 100 ml), une solution aqueuse de sulfate d'hydrogène de potassium 1 M (2 × 100 ml), de l'eau, de la saumure (1 × 100 ml), on sèche l'ensemble sur du sulfate de sodium et on le concentre sous vide pour obtenir un solide blanc. Rendement : 97% (5,2 g) ; CLHP : t_{R} = 8,9 min, pureté >88%, 30-100 en 20 min.

### Composé 3

Le composé 2 (5,1 g ; 9,5 mmol) est dissous dans du MeOH (100 ml) avec HCl (9,5 mmol) à température ambiante et hydrogéné en présence d'un catalyseur 10% Pd/C. Après 3 heures, on enlève le catalyseur par filtration et le filtrat est concentré sous vide. Le sel attendu HCl est cristallisé dans de l'éther et séché sous vide sur KOH. Rendement : 100% (4,2 g).

Le sel HCl (4,2 g ; 9,5 mmol) est dissous dans du DMF (20 ml) contenant du Z-Lys(Boc)-OH (3,8 g ; 10,5 mmol) et du BOP (4,42 g ; 10 mmol). On ajoute à cette solution de la DIEA (4,2 ml ; 25 mmol) et le mélange réactionnel est agité pendant la nuit à température ambiante. Ensuite, on ajoute une solution de bicarbonate de sodium saturée (500 ml) sous agitation, puis on ajoute de l'acétate d'éthyle (200 ml). La phase organique est lavée avec une solution de bicarbonate de sodium saturée (2 × 100 ml), de l'eau (2 × 100 ml), une solution aqueuse de sulfate d'hydrogène de potassium 1 M (2 × 100 ml), de l'eau, de la saumure (1 × 100 ml), on sèche l'ensemble sur du sulfate de sodium et on le concentre sous vide pour laisser un résidu qui se solidifie lors de la trituration dans de l'hexane. Il est récolté, lavé avec de l'hexane, du diisopropyléther et séché sous vide sur KOH. Rendement : 93% (6,8 g) ; CLHP : t_{R} = 10,9 min, pureté 90%, 30-100 en 20 min.

### Composé 4

Le composé 3 (6,8 g ; 8,9 mmol) est dissous dans du MeOH (100 ml) avec HCl (8,9 mmol) à température ambiante et hydrogéné en présence d'un catalyseur 10% Pd/C. Après 3 heures, on enlève le catalyseur par filtration et le filtrat est concentré sous vide pour donner un solide blanc. Rendement : 91% (5,4 g) ; CCM Rf 0,3 CMA 60/10/5.

Le sel HCl (5,4 g ; 8,1 mmol) est dissous dans du DMF (25 ml) contenant du Z-(D)Ala-OH (1,9 g; 8,5 mmol) et du BOP (3,8 g; 8,5 mmol). On ajoute à cette solution de la DIEA (4,2 ml ; 25 mmol) et le mélange réactionnel est agité pendant 6 heures à température ambiante. Ensuite, on ajoute une solution de bicarbonate de sodium saturée (500 ml) sous agitation, puis on ajoute de l'acétate d'éthyle (200 ml). La phase organique est lavée avec une solution de bicarbonate de sodium saturée (2 × 100 ml), de l'eau (2 × 100 ml), une solution aqueuse de sulfate d'hydrogène de potassium 1 M (2 ×100 ml), de l'eau, de la saumure (1 × 100 ml), on sèche l'ensemble sur du sulfate de sodium et on le concentre sous vide pour laisser un résidu qui se solidifie lors de la trituration dans de l'hexane. Il est récolté, lavé avec de l'hexane, du diisopropyléther et séché sous vide sur KOH. Rendement : 78% (5,3 g) ; CLHP : t_{R} = 10,67 min, pureté 77%, 30-100 en 20 min.

### Composé 5

Le composé 4 (5,3 g ; 6,3 mmol) est dissous dans du MeOH (100 ml) avec HCl (6,3 mmol) à température ambiante et hydrogéné en présence d'un catalyseur 10% Pd/C. Après 5 heures, on enlève le catalyseur par filtration et le filtrat est concentré sous vide. Le sel attendu HCl est cristallisé dans de l'éther et séché sous vide sur KOH. Rendement: 91% (4,7 g).

Le sel HCl (4,7 g ; 5,5 mmol) est dissous dans du DMF (20 ml) contenant du Z-Lys(Boc)-OH (2,2 g ; 5,8 mmol) et du BOP (2,56 g ; 5,8 mmol). On ajoute à cette solution de la DIEA (1,9 ml ; 11 mmol) et le mélange réactionnel est agité pendant la nuit à température ambiante. Ensuite, on ajoute une solution de bicarbonate de sodium saturée (500 ml) sous agitation, puis on ajoute de l'acétate d'éthyle (200 ml). La phase organique est lavée avec une solution de bicarbonate de sodium saturée (2 × 100 ml), de l'eau (2 × 100 ml), une solution aqueuse de sulfate d'hydrogène de potassium 1 M (2 × 100 ml), de l'eau, de la saumure (1 × 100 ml), on sèche l'ensemble sur du sulfate de sodium et on le concentre sous vide pour laisser un résidu qui se solidifie lors de la trituration dans de l'hexane. Il est récolté, lavé avec de l'hexane, du diisopropyléther et séché sous vide sur KOH. Rendement : 90% (5,3 g) ; CLHP : t_{R} = 12,26 min, pureté 60%, 30-100 en 20 min.

### Composé 6

Le composé 5 (5,3 g ; 4,9 mmol) est dissous dans de l'acétone (20 ml) et de la soude NaOH 1N (5,9 mmol) est ajoutée à 0°C. Le mélange réactionnel est maintenu à 0°C pendant 1 heure avant de le laisser se réchauffer à température ambiante. Après 6 heures, le mélange réactionnel est concentré sous vide. Ensuite, on ajoute de l'acétate d'éthyle (100 ml) sous agitation, puis une solution aqueuse de sulfate d'hydrogène de potassium 1 N (2 × 100 ml), de l'eau, de la saumure (1 × 100 ml), on sèche l'ensemble sur du sulfate de sodium et on le concentre sous vide pour laisser un résidu qui se solidifie lors de la trituration dans du diisopropyléther. Il est récolté, lavé avec du diisopropyléther et séché sous vide sur KOH. Le peptide brut est purifié par chromatographie sur une colonne de gel de silice en utilisant CMA 120/10/5. Rendement : 44% (2,3 g) ; CLHP : t_{R} = 11,28 min, pureté 78%, 30-100 en 20 min.

### Composé 7

Le composé 6 (1,5 g; 1,46 mmol) est dissous dans du MeOH (10 ml) à température ambiante et hydrogéné en présence d'un catalyseur 10% Pd/C. Après 4 heures, on enlève le catalyseur par filtration et le filtrat est concentré sous vide. Le sel attendu HCl est cristallisé dans du diisopropyléther et séché sous vide sur KOH. Rendement : 88% (1,17 g) ; CLHP : t_{R} = 16,9 min, pureté 70%, 5-65 en 20 minutes.

### Composé 8

Le composé 7 (800 mg ; 0,87 mmol) est dissous dans du DMF (80 ml) à température ambiante contenant de l'EDC, du HCl (201 mg ; 1,05 mmol), de l'HOBt (142 mg ; 1,05 mmol) et on ajoute de la DIEA (373 ml ; 2,19 mmol) à cette solution. Le mélange réactionnel est agité pendant 2 jours à température ambiante et le mélange réactionnel est concentré sous vide.

On ajoute sous agitation une solution saturée de bicarbonate de sodium, puis de l'acétate d'éthyle (100 ml). La phase organique est lavée avec une solution saturée de bicarbonate de sodium (2 × 100 ml), puis une solution aqueuse de sulfate d'hydrogène de potassium 1N (2 × 100 ml), de l'eau, de la saumure (1 × 100 ml), on sèche l'ensemble sur du sulfate de sodium et on le concentre sous vide pour donner un solide blanc. Rendement : 100% (0,78 g) ; M+H⁺ = 896.

### Composé 9

Le composé 8 (650 mg ; 0,76 mmol) est dissous dans du TFA (6 ml) pendant 1 heure à température ambiante. L'amine du mélange est concentrée sous vide. L'amine attendue est précipitée dans de l'éther (50 ml). Elle est récoltée, lavée avec de l'éther et séchée sous vide. Rendement : 98% (0,70 g) ; M+H⁺ = 598,5 (calc.= 597,6).

### EXEMPLE 4 - PRÉPARATION DU COMPOSÉ DE FORMULE (III-e) (L40)

Le composé L40 répond à la formule suivante :

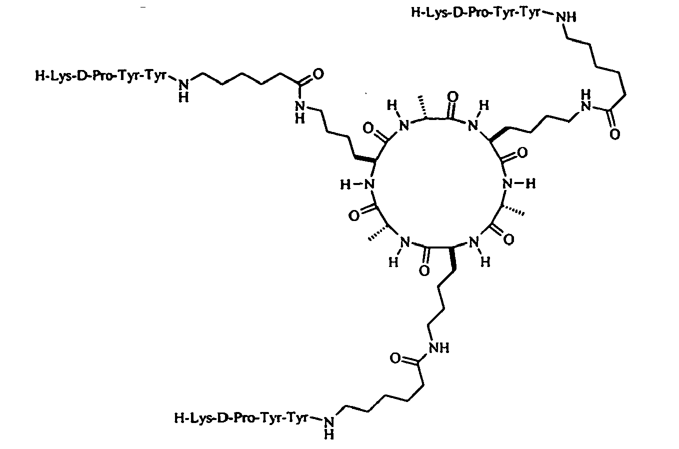

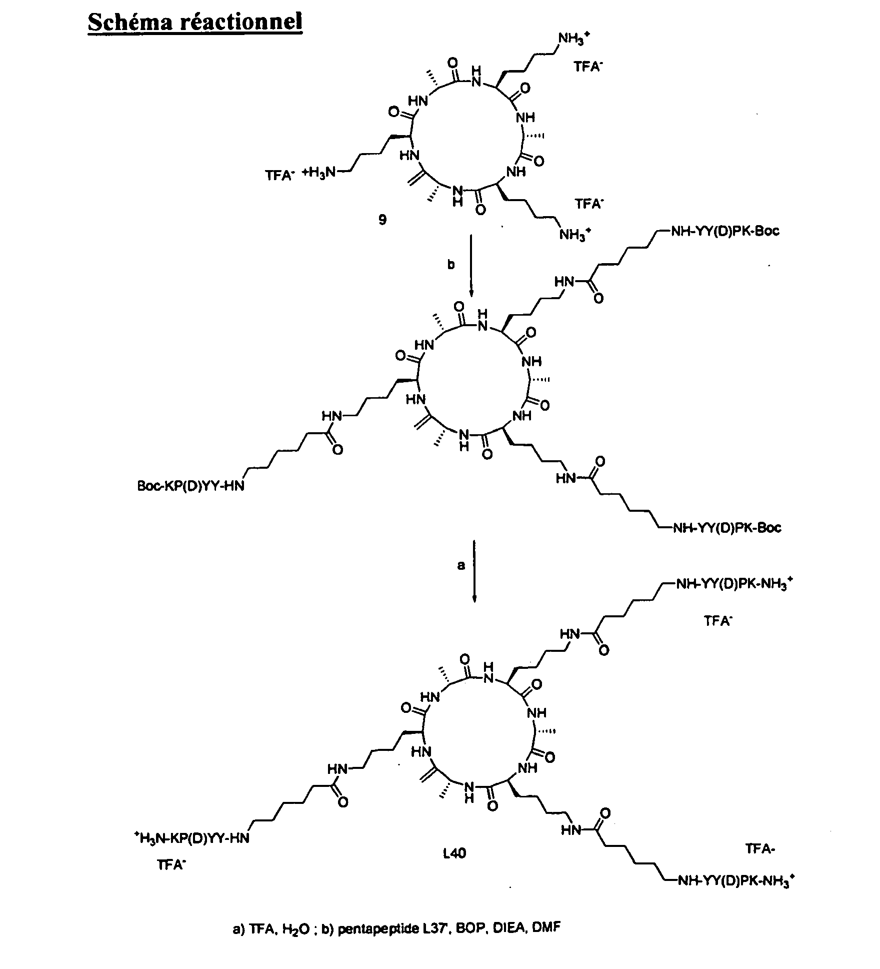

A 10 mg (1 éq.) de cycle **9** dans le DMF (voir exemple 3) sont ajoutés 35mg (3,3 éq.) de pentapeptide Boc-Lys(Boc)-(D)Pro-Tyr(tBu)-Tyr(tBu)-Ahx-OH (L37'), 16 mg (3,3 éq.) de BOP et on ajoute 20 µL (10 éq.) de DIEA. Le mélange réactionnel est agité pendant 25 heures à température ambiante, puis est précipité au NaHCO₃ (aq.). Après filtration, le précipité est lavé successivement à H₂O, KHSO₄ 1N, H₂O et AcOEt.

Le solide formé est alors traité au TFA en présence d'eau. Après 30 minutes d'agitation, la solution est précipitée à l'éther et filtrée pour donner 40 mg de ligand **L40.**

6mg de **L40** pur sont obtenus après purification par RP-CLHP (rendement après purification 22%).

Le ligand **L40** est caractérisé par :
**CLHP** : *t*_{R} 10,203 min (gradient linéaire, 5-65% B, 20 min)
**MALDI-TOF :** calculé pour C₁₃₂H₁₉₆N₂₇O₂₇ [M+H⁺] : 2593,16; observé: 2593,24 ; calculé pour C₁₃₂H₁₉₅N₂₇O₂₇Na [M+Na⁺] : 2616,16 ; observé 2616,78.

### EXEMPLE 5 - PRÉPARATION DU COMPOSÉ DE FORMULE (III-f) (L43)

Le composé L43 répond à la formule suivante :

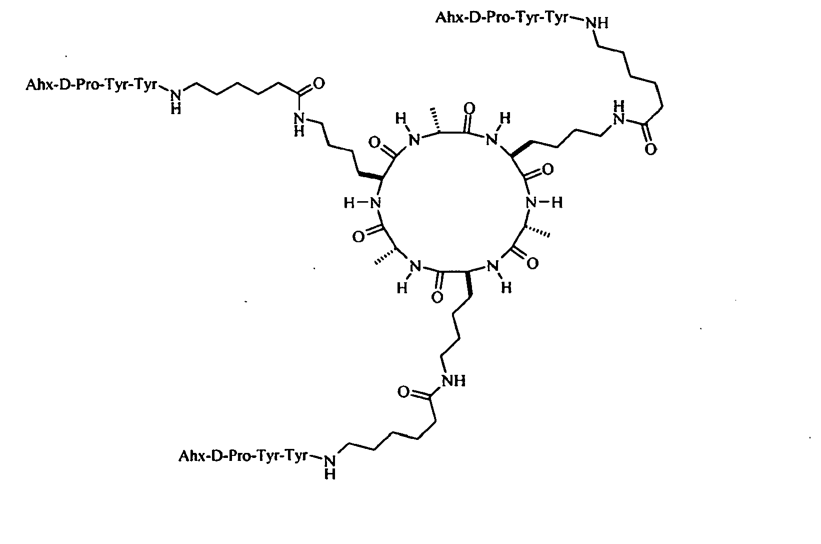

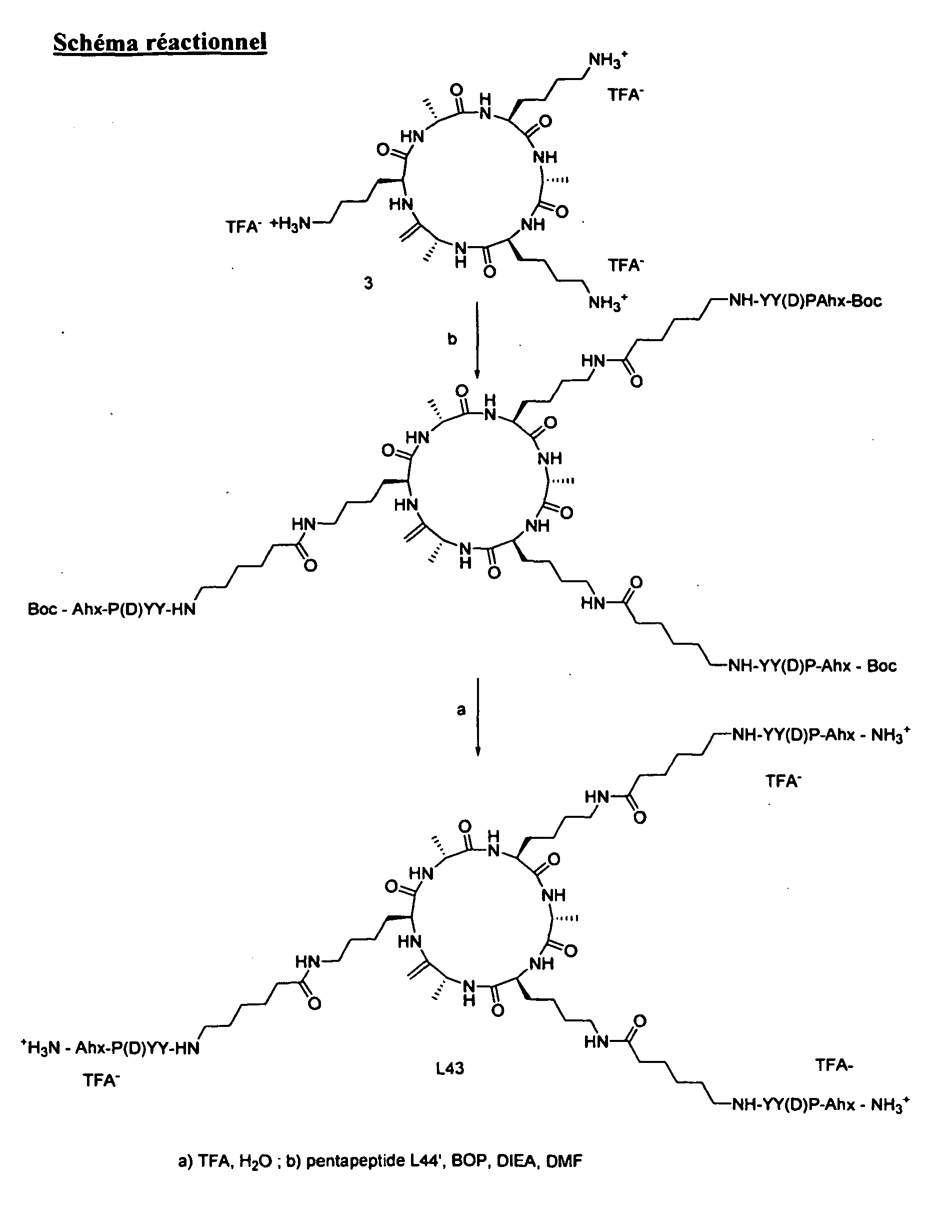

A 10mg (1eq.) de cycle **9** dans le DMF (voir exemple 3) sont ajoutés 35 mg (3,3 éq.) de pentapeptide Boc-Ahx-(D)Pro-Tyr(tBu)-Tyr(tBu)-Ahx-OH (L44'), 16 mg (3,3 éq.) de BOP et on ajoute 20 µL (10 éq.) de DIEA. Le mélange réactionnel est agité pendant 25 heures à température ambiante, puis est précipité au NaHCO₃ (aq.). Après filtration, le précipité est lavé successivement à H₂O, KHSO₄ 1N, H₂O et AcOEt.

Le solide formé est alors traité au TFA en présence d'eau. Après 30 minutes d'agitation, la solution est précipitée à l'éther et filtrée pour donner 20 mg de ligand **L43.**

8 mg de **L43** pur sont obtenus après purification par RP-CLHP (rendement après purification 30%).

Le ligand **L43** est caractérisé par :
**HLPC :** *t*_{R} 13,935 min (gradient linéaire, 5-65% B, 20 min)
**MALDI-TOF :** calculé pour C₁₃₂H₁₉₃N₂₄O₂₇ [M+H⁺] : 2548,11 ; observé: 2547,88 ; calculé pour C₁₃₂H₁₉₂N₂₄O₂₇Na⁺ [M+Na⁺] : 2571,11 ; observé: 2570,62; calculé pour C₁₃₂H₁₉₃₂N₂₄O₂₇K⁺ [M+K⁺] : 2587,11 ; observé : 2586,13.

### EXEMPLE 6 - PRÉPARATION DU COMPOSÉ L41

Le composé L41 répond à la formule suivante :

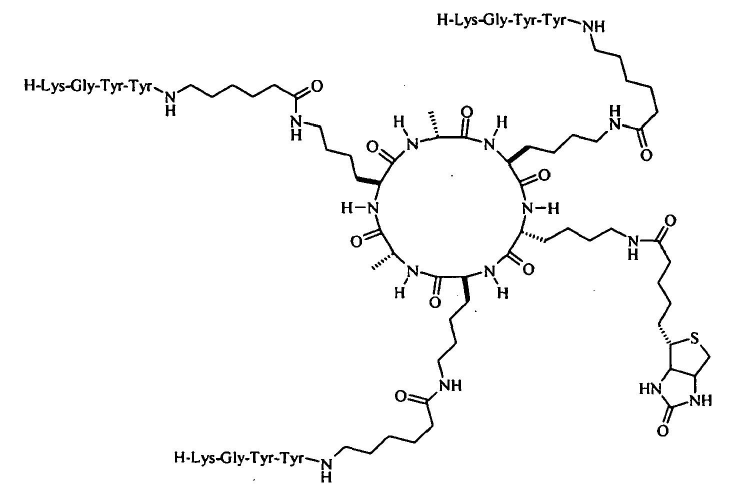

Il peut également être représenté par la formule suivante :

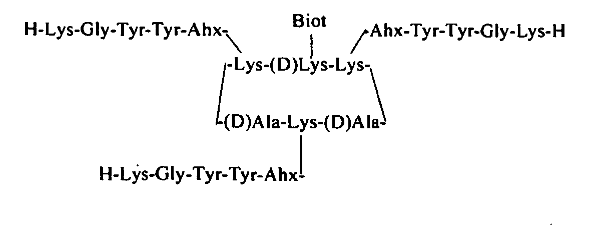

La stratégie de synthèse du composé A (L41) est décrite ci-dessous. La synthèse des intermédiaires 1 et 2 a été réalisée en phase solide et les étapes suivantes ont été effectuées en solution.

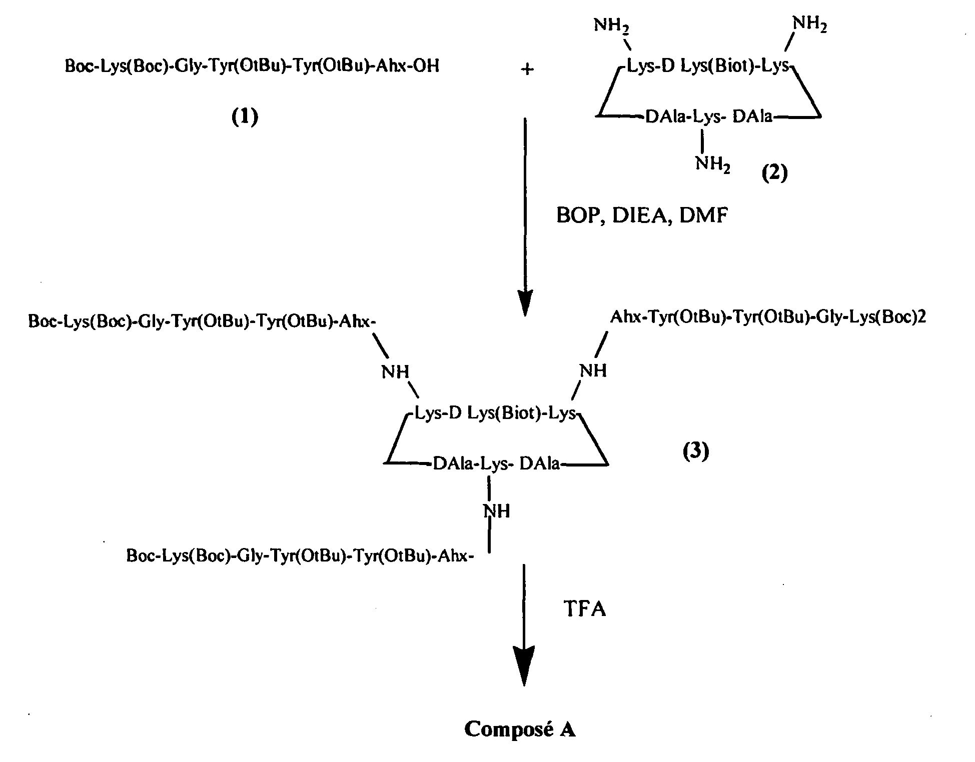

### 1) Synthèse du pentapeptide Boc-Lys(Boc)-Gly-Tyr(OtBu)-Tyr(OtBu)-Ahx-OH (1):

La synthèse de ce peptide a été effectuée suivant une stratégie Fmoc en phase solide pas à pas. Le peptide a été assemblé sur une résine 2-chlorotrityl (Senn chemicals, 1.3 mmoles/g) et l'échelle de synthèse était de 1,3 mmoles.

Le couplage du premier aminoacide est effectué dans le dichlorométhane anhydre (4 ml) avec 2 équivalents de Fmoc-Ahx-OH et 6 équivalents de DIEA pendant 3 heures. Le test de substitution réalisé, après lavages et séchage de la résine, montre un taux de substitution de 0,6 mmoles/g.

Le couplage des aminoacides suivants, Fmoc-Tyr(OtBu)-OH, Fmoc-Gly-OH et Boc-Lys(Boc)-OH est effectué dans le DMF avec 5 équivalents d'aminoacides en présence de BOP/HOBt et de DIEA. Un double couplage est réalisé de manière systématique.

La déprotection des groupements Fmoc est effectuée en utilisant une solution de 25% de pipéridine dans de la DMF pendant 20 minutes.

Le clivage du peptide de la résine est effectué à l'aide d'une solution de 1,1,1,3,3,3-hexafluoro-2-propanol/dichlorométhane (60/40) pendant 2 heures à température ambiante.

La résine est filtrée et le filtrat est concentré et séché sous vide. Le peptide protégé est directement utilisé sans purification. Le rendement de la synthèse est quantitatif.

Le pentapeptide protégé est analysé par CLHP (colonne C18, Macherey Nagel, SP 250/10 nucléosil 300-7) en utilisant un mélange eau/acétonitrile/TFA, un gradient de 30 à 100% B en 20 minutes (A : eau, 0,1%TFA ; B : acétonitrile, 0,08%TFA), un débit de 1,2 ml/minute et une absorption à 210 nm)(Figure 1). Le taux de pureté est de 92%.

### 2) Synthèse de l'hexapeptide cyclisé (2) :

La synthèse de l'hexapeptide H-(D)Ala-Lys(Boc)-(D)Lys(Biot)-Lys(Boc)-(D)Ala-Lys(Boc)-OH a été effectuée suivant une stratégie Fmoc en phase solide pas à pas et selon le même protocole que celui décrit pour la synthèse du pentapeptide en utilisant les aminoacides suivants : Fmoc-Lys(Boc)-OH, Fmoc-(D)Ala-OH et Fmoc-(D)Lys(Biot)-OH (marquage avec la biotine). Le rendement de synthèse est également quantitatif et le peptide est directement utilisé sans aucune purification.

L'hexapeptide non cyclisé est analysé par CLHP (colonne C 18, Macherey Nagel, SP 250/10 nucléosil 300-7) en utilisant un mélange eau/acétonitrile/TFA, un gradient de 20 à 80% B en 20 minutes (A : eau, 0, 1 %TFA ; B : acétonitrile, 0,08%TFA), un débit de 1,2 ml/minute et une absorption à 210 nm)(Figure 2).

L'hexapeptide (660 mg ; 0,55 mmol) est dissous dans du DMF (70 ml) à température ambiante contenant EDC, HCl (127 mg ; 0.66 mmol), HOBt (89 mg ; 0.66 mmol) et de la DIEA (140 µl, 0.82 mmol). Après 48 heures, le milieu réactionnel est précipité dans une solution contenant du NaHCO₃ saturé (300 ml). L'hexapeptide cyclisé est ensuite lavé avec une solution de KHSO₄ 1N et une solution saturée de NaCl puis séché sous vide (540 mg ; Rdt : 83%).

La déprotection des groupements Boc est effectuée avec de l'acide trifluoroacétique (3 ml) pendant 1 heure. Le peptide est ensuite précipité par addition de diéthyléther (50 ml), filtré et séché sous vide (510 mg ; Rdt : 95%).

L'hexapeptide cyclisé et déprotégé est analysé par CLHP (colonne C18, Macherey Nagel, SP 250/10 nucléosil 300-7) en utilisant un mélange eau/acétonitrile/TFA, un gradient de 5 à 65% B en 20 minutes (A : eau ; 0,1%TFA ; B : acétonitrile ; 0,08%TFA), un débit de 1,2 ml/minute et une absorption à 210 nm) (Figure 3).

### 3) Couplage des intermédiaires (1) et (2) :

L'hexapeptide cyclisé et déprotégé **(2)** (61 mg ; 50 µmol) est dissous à température ambiante dans du DMF (2 ml) contenant le pentapeptide **(1)** (157 mg ; 165 µmol), BOP (73 mg; 165 µmol), et de la DIEA (85 µl). Après 48 heures, le milieu réactionnel est précipité dans une solution contenant du NaHCO₃ saturé (300 ml). Le précipité est ensuite lavé avec une solution de KHSO₄ 1N et une solution saturée de NaCl puis séché sous vide (180 mg ; Rdt : 100%).

La déprotection des groupements Boc et tBu du composé **(3)** est réalisée avec de l'acide trifluoroacétique (3 ml) pendant 1 heure. Le peptide est ensuite précipité par addition de diéthyléther (50 ml), filtré et séché sous vide (160 mg avant purification ; Rdt : 100%).

Le peptide est ensuite purifié par CLHP phase inverse sur une CLHP préparative Perkin-Elmer, sur une colonne C18 (Macherey Nagel, SP 250/10 nucléosil 300-7) en utilisant un mélange eau/acétonitrile/TFA, un gradient de 5 à 65% B en 30 minutes (A : eau, 0,1% TFA ; B : acétonitrile - 0,08% TFA) et un débit de 6 ml/minute. Les fractions présentant une pureté supérieure à 97% sont réunies et lyophilisées. (Figure 4 : profil CLHP du composé A après purification ; taux de pureté = 97,2%).

Le rendement de la purification CLHP est de 17%.

### EXEMPLE 7 - PREPARATION DU COMPOSE DE FORMULE (III-i) (L62)

Le composé **L62** répond à la formule suivante :

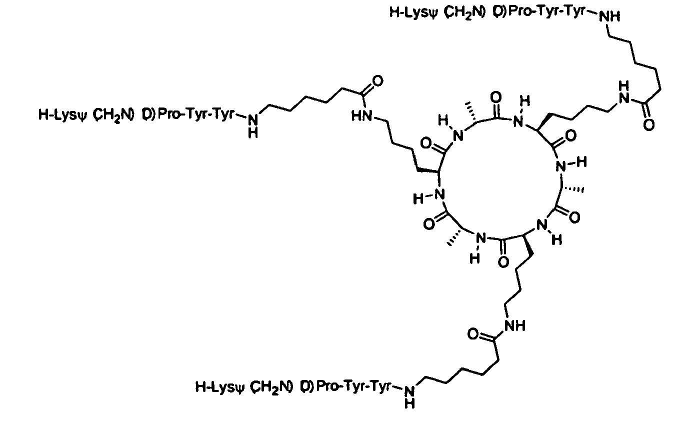

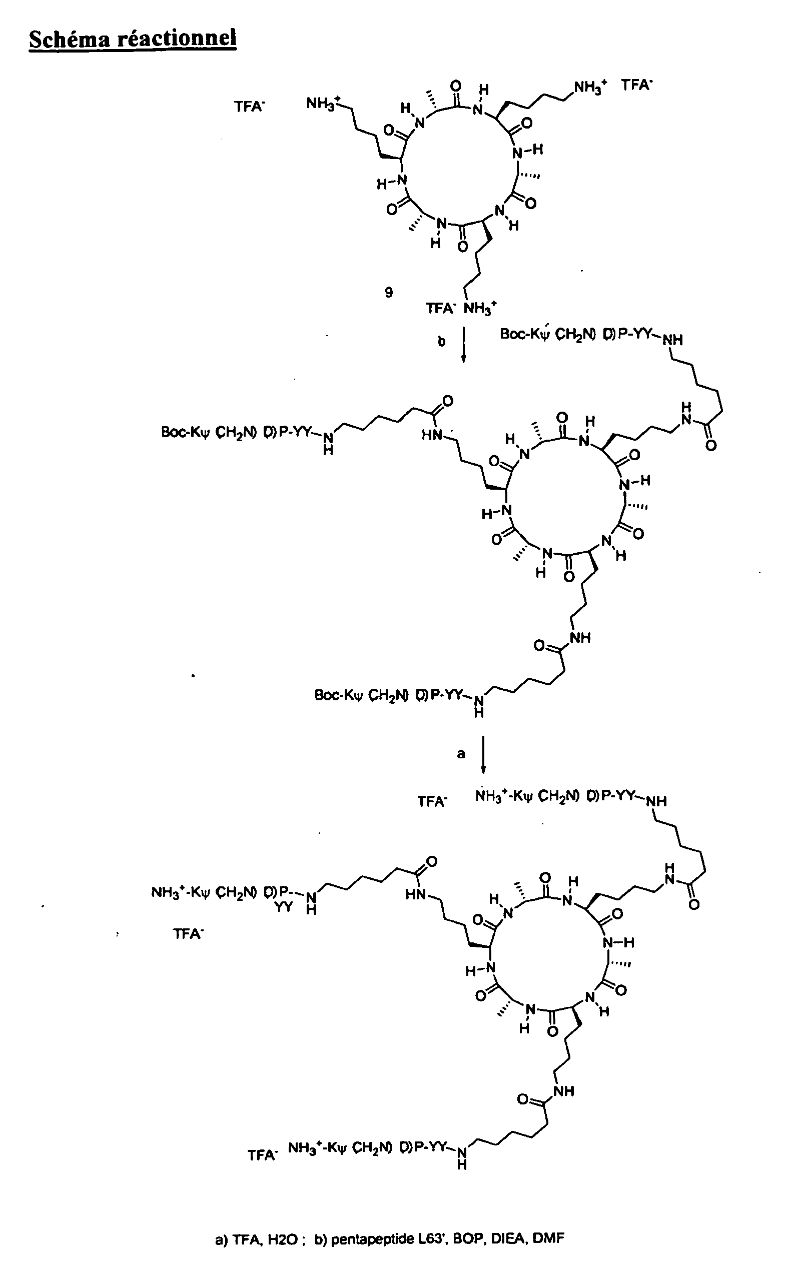

A 10 mg (1 éq.) de cycle **9** dans le DMF (voir exemple 3) sont ajoutés 35 mg (3,3 éq.) de pentapeptide Boc-Lys(Boc)ψ(CH₂N)_{D}Pro-Tyr(tBu)-Tyr(tBu)-Ahx-OH (**L63'**), 16 mg (3,3 éq.) de BOP et on ajoute 20 µl (10 éq.) de DIEA. Le mélange réactionnel est agité pendant 25 heures à température ambiante, puis est précipité au NaHCO₃ (aq). Après filtration, le précipité est lavé successivement à H₂O, KHSO₄ 1N, H₂O et AcOEt.

Le solide formé est alors traité au TFA en présence d'eau. Après 30 minutes d'agitation, la solution est précipité à l'éther et filtrée pour donner 20 mg de ligand **L62.**

2 mg de **L62** pur sont obtenus après purification par RP-CLHP (rendement après purification 7%).

Le ligand **L62** est caractérisé par :
**CLHP** : *t*_{R} 11,6 min (gradient linéaire, 5-65% B, 20 min)
**MALDI-TOF :** calculé pour C₁₃₂H₂₀₁N₂₇O₂₄H⁺ : 2550,21 ; observé 2551 ; calculé pour C₁₃₂H₂₀₁N₂₇O₂₄K⁺ : 2590,21 ; observé 2590.

### EXEMPLE 8 - PREPARATION DU COMPOSE DE FORMULE (III-j) (L68)

Le composé **L68** répond à la formule suivante :

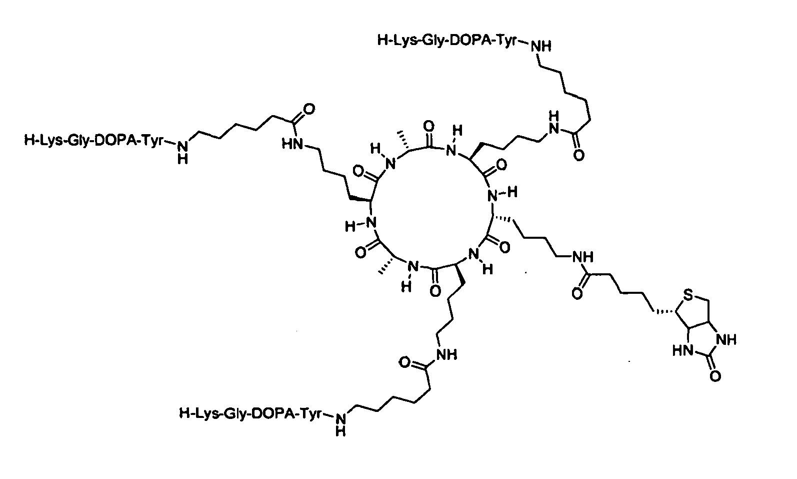

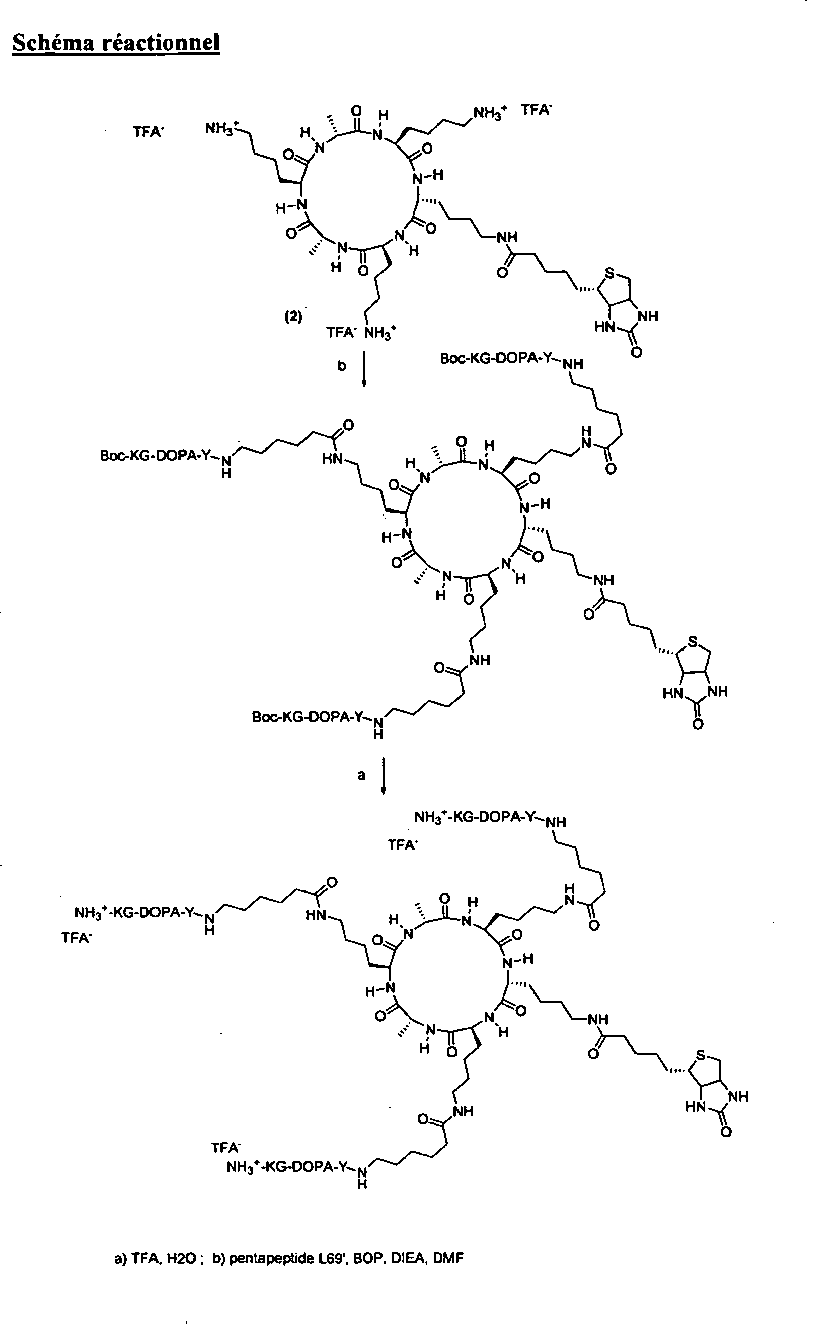

A 15mg (1 éq.) de cycle biotine (2) dans le DMF (voir exemple 3) sont ajoutés 39 mg (3,3 éq.) de pentapeptide Boc-Lys(Boc)-Gly-Dopa(Acetonid)-Tyr(tBu)-Ahx-OH (**L69'**), 18 mg (3,3 éq.) de BOP et on ajoute 30 µl (10 éq.) de DIEA. Le mélange réactionnel est agité pendant 25 heures à température ambiante, puis est précipité au NaHCO₃ (aq). Après filtration, le précipité est lavé successivement à H₂O, KHSO₄ 1N, H₂O et AcOEt.

Le solide formé est alors traité au TFA en présence d'eau. Après 30 minutes d'agitation, la solution est précipité à l'éther et filtrée pour donner 50 mg de ligand **L68.**

9 mg de **L68** pur sont obtenus après purification par RP-CLHP (rendement après purification 21 %).

Le ligand **L68** est caractérisé par :
**CLHP** : *t*_{R} 11,1 min (gradient linéaire, 5-65% B, 20 min)
**MALDI-TOF** : calculé pour C₁₃₆H₂₀₄N₃₀O₃₂SH⁺ : 2804,35 ; observé 2802,96 ; calculé pour C₁₃₆H₂₀₄N₃₀O₃₂SNa⁺: 2827,35 ; observé 2824,91 ; calculé pour C₁₃₆H₂₀₄N₃₀O₃₂SK⁺ : 2841,35 ; observé 2841,98.

### TESTS BIOLOGIQUES

Ces tests s'effectuent en 3 phases. On choisit d'abord les ligands les plus intéressants grâce à des tests simples d'activation de l'apoptose de cellules de lymphomes, puis leur interaction avec CD40 est finement analysée avec la technologie BIAcore, et enfin l'interaction des ligands biotinylés avec le CD40 membranaire est étudiée en microscopie en fluorescence et cytométrie de flux.

L'effet agoniste ou antagoniste des ligands a été testé grâce à deux tests biologiques classiques. Le premier test est basé sur la propriété de certains lymphomes B de cesser de proliférer et d'entrer en apoptose lors du pontage de leur molécule CD40 membranaire (Tong et al., 1999). En effet, certains lymphomes B entrent en apoptose lors du pontage de leur molécule CD40 par un anticorps anti-CD40 ou par du CD40L soluble de manière indépendante de la molécule CD95. Cette induction d'apoptose se traduit par une diminution de prolifération. Les ligands agonistes de CD40 vont induire une augmentation de l'apoptose et par conséquent une diminution de prolifération. Dans ce test, on incube les cellules BL41 avec des ligands de CD40 et on mesure après 24h soit l'inhibition de prolifération de ces cellules, en étudiant l'incorporation de thymidine tritiée, soit le pourcentage de cellules apoptotiques par une étude en cytométrie de flux. Dans le second test, on étudie l'expression de la molécule membranaire CD95 par des cellules B transformées (lymphomes de Burkitt) après activation par l'interaction CD40-CD40L (Schattner et al., 1996). Dans ce test, le partenaire CD40L est exprimé sur des fibroblastes transfectés (3T6-CD40L)(Buelens et al., 1997). Les cellules du lymphome de Burkitt BL41 sont incubées avec des fibroblastes transfectés ou non avec CD40L. Après 48h, l'expression de CD95 est évaluée par cytométrie de flux. L'expression de CD95 est induite en présence de CD40L et un anticorps anti-CD40L commercial inhibe les conséquences de l'interaction CD40-CD40L.

L'effet agoniste des ligands est évalué par la mesure après incubation des cellules B avec les ligands soit de l'expression de CD95 en cytométrie de flux soit de l'inhibition de la prolifération et/ou de l'augmentation de la mort par apoptose. L'effet antagoniste est évalué par la mesure de la diminution de l'expression de CD95 induite par le CD40L en présence des différents ligands.

### A - Etude de l'effet biologique agoniste des ligands

### Principe

L'effet agoniste des ligands a été testé grâce à un test biologique simple qui est basé sur la propriété de certains lymphomes B d'entrer en apoptose lors du pontage de leur molécule CD40 membranaire (Tong et al., 1999). En effet, ces lymphomes B entrent en apoptose lors du pontage de leur molécule CD40 par un anticorps anti-CD40 ou par du CD40L soluble. Les ligands agonistes de CD40 vont induire de la même manière une augmentation de l'apoptose de cellules de lymphome de Burkitt qui est mesurée par une analyse précise en cytométrie de flux.

### Mode opératoire

Les cellules (5.10⁵/mL) des lymphomes de Burkitt BL41 ou Raji (ATCC CCL-86), deux lignées cellulaires semblables, sont cultivées en présence des différents ligands aux concentrations désirées. Après 16h, les cellules sont marquées avec de l'iodure de 3,3'-dihexyloxacarbocyanine (DiOC₆) à 40nM (Petit et al., 1995) pour évaluer le pourcentage de cellules apoptotiques en cytométrie de flux. Comme contrôle, l'effet des ligands a été testé sur les cellules de lymphomes T Jurkat qui n'expriment pas CD40 et ne réagissent pas en présence d'un CD40 ligand soluble ou membranaire.

### Résultats

Le ligand L36 (V-b) induit fortement l'apoptose des cellules BL41 puisqu'à 25 µM, on détecte plus de 80% d'apoptose spécifique (Figure 5A). Le ligand biotinylé L41 a une activité biologique comparable à son homologue non-biotinylé L4 (L41 non biotinylé)(Figure 5B). Le ligand L40 est plus efficace que L4 puisqu'à 12,5µM, L40 induit 37,5% d'apoptose spécifique et L4 24% (Figure 5C). Enfin le ligand L43 est moins efficace que son homologue L4 (Figure 5D). On peut noter qu'aucun de ces ligands n'induit l'apoptose des cellules de la lignée Jurkat.

Par ailleurs, d'après les figures 8A et 8B, on constate que l'introduction d'une liaison réduite entre les deux premiers résidus (L62) permet d'obtenir un ligand efficace et sélectif.

### B - Etude de l'interaction entre les ligands et le CD40

### Principe

Le BIAcore 3000 est un appareil qui permet l'étude des interactions entre deux molécules. Basé sur la résonance plasmonique de surface, il permet de réaliser des mesures en temps réel et ainsi de suivre les cinétiques d'interaction (association et dissociation) d'un "analyte" (qui se trouve dans une solution injectée) avec un "ligand" (immobilisé sur une micropuce supportant la mesure). Les mesures cinétiques à différentes concentrations d'analyte permettent de calculer les constantes d'affinité de l'interaction entre le ligand et l'analyte. La micropuce contient quatre cellules de mesure différentes, ce qui permet de comparer directement une cellule de référence, sur laquelle une protéine non pertinente (protéine qui n'a aucune affinité pour l'analyte étudié mais proche du ligand) est immobilisée, avec la cellule sur laquelle le ligand est immobilisé.

Le test s'effectue en deux étapes: mesure de l'interaction directe des ligands avec le CD40 immobilisé sur la micropuce et mesure de l'inhibition de l'interaction CD40-CD40L par les ligands.

### 1- Interaction des ligands sur le CD40

### Mode opératoire

Sur une micropuce ont été immobilisés des anticorps de lapin dirigés contre la partie constante d'immunoglobulines de souris. On immobilise dans la cellule de référence un anticorps monoclonal de souris d'isotype IgG2a (LG11.2) à 100 nM avec un flux de 5 µL/min durant une minute. Dans la cellule du ligand, on immobilise dans les mêmes conditions le CD40 recombinant associé à la partie constante de la chaîne lourde d'IgG2a de souris (*human CD40 muIg fusion protein,* ANCELL Corporation, Bayport, MN).

Sur les deux cellules (référence et ligand) sont ensuite injectés, comme analytes, les ligands de CD40 à différentes concentrations. Le flux est de 30 µL/min pendant 4 minutes pour étudier l'association puis, en absence d'analyte, les conditions sont identiques pour étudier la dissociation.

Afin de régénérer les cellules (c'est-à-dire retirer toutes les protéines adsorbées de manière non covalente), une solution de 50 mM de HCl est injectée à 5 µL/min durant 1 minute. Les cellules sont ensuite prêtes pour une nouvelle analyse.

### Résultats

Les cinétiques d'association directe à CD40 des ligands L40 et L41 ont été mesurées (Figure 6) et ont permis de calculer des constantes de dissociation de 3,1 × 10⁻⁷M pour L40 et de 2,8 × 10⁻⁸M pour L41. Pour information, la constante de dissociation calculée pour L4 dans les mêmes conditions est de 9,4 × 10⁻⁸M.

### 2- Inhibition de l'interaction CD40-CD40L

### Mode opératoire

Sur une micropuce ont été immobilisés des anticorps de lapin dirigés contre la partie constante d'immunoglobulines de souris. On immobilise dans la cellule de référence un anticorps monoclonal de souris d'isotype IgG2a (LG11.2) à 40 nM avec un flux de 5 µL/min durant deux minutes. Dans la cellule du ligand, on immobilise dans les mêmes conditions le CD40 recombinant associé à la partie constante de la chaîne lourde d'IgG2a de souris (*human CD40 muIg fusion protein,* ANCELL Corporation, Bayport, MN).

Sur les deux cellules (référence et ligand) est ensuite injecté, comme analyte, le CD40L (*human CD154 muCD8 fusion protein,* ANCELL Corporation) à 35 nM en présence des ligands à différentes concentrations. Le flux en présence des analytes est de 10 µL/min pendant 5 minutes pour étudier l'association puis, en absence d'analyte, les conditions sont identiques pour étudier la dissociation.

Afin de régénérer les cellules (c'est-à-dire retirer toutes les protéines adsorbées de manière non covalente), une solution de 10 mM de HCl est injectée à 5 µL/min durant 1 minute. Les cellules sont ensuite prêtes pour une nouvelle analyse.

### Résultats

L'association de CD40L à CD40 a été étudiée en présence de différentes concentrations de ligand L40 ou L43. A 1 µM, le ligand L40 inhibe la liaison de CD40L à CD40 à 100%, alors que dans les mêmes conditions le ligand L43 l'inhibe à 30%.

### C - Etude de l'interaction entré les ligands biotinylés et le CD40 membranaire

### Principe

L'analyse directe de l'interaction d'un ligand avec le CD40 membranaire est rendue possible avec la biotinylation des ligands. La liaison de ces ligands au CD40 est révélée à l'aide d'une streptavidine, ligand spécifique et très affin de la biotine, marquée avec un fluorochrome. L'objectif est la détection du CD40 à l'aide de ligands spécifiques, de la même manière qu'avec des anticorps anti-CD40 monoclonaux. Ces ligands spécifiques ne reconnaissant pas le même épitope que les anticorps anti-CD40, ils ont pu être utilisés dans des expériences de double marquage en microscopie à fluorescence pour confirmer leur colocalisation avec les anticorps anti-CD40, donc leur spécificité pour CD40.

### Mode opératoire

Pour une analyse en cytométrie en flux, on incube 10⁶ cellules avec 2,5 µM de ligand biotinylé dans 100 µl de tampon phosphate à 4°C pendant 15 minutes. Les cellules sont ensuite mises en présence de streptavidine marquée du fluorochrome fluorescéine isothiocyanate, diluée 500 fois dans 100 µl de tampon phosphate et ce à 4°C pendant 10 minutes. Les cellules ainsi marquées sont analysées en cytométrie en flux.

Pour une analyse en microscopie à fluorescence ou confocale, on fixe 10⁶ cellules avec 1% de paraformaldéhyde dans le tampon phosphate une nuit à 4°C. Le lendemain, les cellules sont mises en présence de 2,5 µM de ligand biotinylé et d'un anticorps monoclonal de souris dirigé contre CD40 dilué 100 fois, le tout dans 100 µl de tampon phosphate contenant 1% d'albumine sérique de boeuf à 4°C pendant 30 minutes. Les cellules sont ensuite incubées avec une streptavidine marquée du fluorochrome Alexa⁵⁴⁶ diluée 500 fois et d'un anticorps de chèvre dirigé contre la partie Fc des anticorps de souris couplé au fluorochrome Alexa⁴⁸⁸ dilué 500 fois, le tout dans 100 µl de tampon phosphate/albumine sérique de boeuf et ce à 4°C pendant 15 minutes. Les cellules ainsi marquées sont alors remises en suspension dans un milieu de montage avant de les disposer entre lame et lamelles et de les observer au microscope.

### Résultats

Le ligand biotinylé L41 a été utilisé en marquage en cytométrie de flux. Son interaction avec le CD40 membranaire des cellules BL41 est révélée à l'aide d'une streptavidine marquée du fluorochrome fluorescéine isothiocyanate (FITC) détecté dans le canal FL1 du cytomètre (Figure 7A). Le résultat du double marquage en microscopie indique que le ligand L41 est spécifique de CD40 puisque le marquage correspond à celui de l'anticorps dirigé contre CD40 (Figure 7B).

### RÉFÉRENCES

- Buelens, C., Verhasselt, V., De Groote, D., Goldman, M., Willems, F. (1997) Human dendritic cell responses to lipopolysaccharide and CD40 ligation are differentially regulated by interleukin-10, Eur J Immunol., 27: 1848-52,
- Chaussabel, D., Jacobs, F., de Jonge, J., de Veerman, M., Carlier, Y., Thielemans, K., Goldman, M., Vray, B. (1999) CD40 ligation prevents Trypanosoma cruzi infection through interleukin-12 upregulation, Infect Immun., 67: 1929-34,
- Diehl, L., den Boer, A.T., Schoenberger, S.P., van der Voort, E.I., Schumacher, T.N., Melief, C.J., Offringa, R., Toes, R.E. (1999) CD40 activation in vivo overcomes peptide-induced peripheral cytotoxic T-lymphocyte tolerance and augments- anti-tumor vaccine efficacy, Nat Med., 5: 774-9,
- Howard, L.M., Miga, A.J., Vanderlugt, C.L., Dal Canto, M.C., Laman, J.D., Noelle, R.J., Miller, S.D. (1999) Mechanisms of immunotherapeutic intervention by anti-CD40L (CD154) antibody in an animal model of multiple sclerosis, J Clin Invest., 103: 281-90,
- Kaiser, E. et coll. (1970) Anal. Biochem., 34, 595-598,
- Kikuchi, T., Moore, M.A., Crystal, R.G. (2000) Dendritic cells modified to express CD40 ligand elicit therapeutic immunity against preexisting murine tumors, Blood. 96: 91-9,
- Kirk, A.D., Burkly, L.C., Batty, D.S., Baumgartner, R.E., Berning, J.D., Buchanan, K., Fechner, J.H., Jr., Germond, R.L., Kampen, R.L., Patterson, N.B., Swanson, S.J., Tadaki, D.K., TenHoor, C.N., White, L., Knechtle, S.J., Harlan, D.M. (1999) Treatment with humanized monoclonal antibody against CD154 prevents acute renal allograft rejection in non-human primates, Nat Med., 5: 686-93,
- Lode, H.N., Xiang, R., Pertl, U., Forster, E., Schoenberger, S.P., Gillies, S.D., Reisfeld, R.A. (2000) Melanoma immunotherapy by targeted IL-2 depends on CD4(+) T-cell help mediated by CD40/CD40L interaction, J Clin Invest., 105: 1623-30,
- Petit, P.X., Lecoeur, H., Zorn, E., Dauguet, C., Mignotte, B., Gougeon, M.L. (1995) Alterations in mitochondrial structure and function are early events of dexamethasone-induced thymocyte apoptosis, J Cell Biol, 130: 157-167,
- Schattner, E.J., Mascarenhas, J., Bishop, J., Yoo, D.H., Chadburn, A., Crow, M.K., Friedman, S.M. (1996) CD4+ T-cell induction of Fas-mediated apoptosis in Burkitt's lymphoma B cells, Blood, 88: 1375-82,
- Sotomayor, E.M., Borrello, I., Tubb, E., Rattis, F.M., Bien, H., Lu, Z., Fein, S., Schoenberger, S., Levitsky, H.I. (1999) Conversion of tumor-specific CD4+ T-cell tolerance to T-cell priming through in vivo ligation of CD40. Nat Med., 5: 780-7,
- Spatola, A.F., "Peptide Backbone Modifications" in Chemistry and Biochemistry of Amino Acids, Peptides and Proteins, vol. III, B. Weinstein, ed. Marcel Dekker, New York, 1983, pages 267-357,
- Tong, A. W., B. Seamour, J. Chen, D. Su, G. Ordonez, L. Frase, G. Netto, and M. J. Stone. 2000. CD40 ligand-induced apoptosis is Fas-independent in human multiple myeloma cells. Leuk Lymphoma 36:543-558.

## Revendications

1. Composé répondant à la formule suivante (I) : 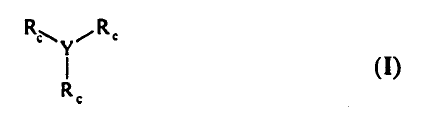 dans laquelle :
- Y représente un macrocycle dont le cycle comprend de 9 à 36 atomes, et est fonctionnalisé par trois fonctions amines ou COOH,
- R_{c} représente un groupe de formule H-Xₐ-X_{b}-X_{c}-X_{d}-Xₑ-(X_{f})ᵢ- ou H-X'ₐ-L-X'_{b}-X_{c}-X_{d}-Xₑ-(X_{f})ᵢ-, dans laquelle :
• i représente 0 ou 1,
• Xₐ est choisi parmi les résidus d'acides aminés suivants :
- lysine ;
- arginine ;
- ornithine ;
- les β-acides aminés correspondant à la lysine, l'arginine ou l'ornithine, portant la substitution en position α ou β ;
- acide tranéxamique ;
- acide N-méthyl-tranéxamique ;
- acide 8-amino-3,6-dioxaoctanoïque ;
- acide 4(pipéridin-4-yl)butanoïque ;
- acide 3(pipéridin-4-yl)propionique ;
- N-(4-aminobutyl)-glycine ;
- NH₂-(CH₂)ₙ-COOH, n variant de 1 à 10;
- NH₂-(CH₂-CH₂-O)ₘ-CH₂CH₂COOH, m variant de 3 à 6 ;
- 4-carboxyméthyl-pipérazine ;
- acide 4-(4-aminophényl)butanoïque ;
- acide 3-(4-aminophényl)propanoïque ;
- acide 4-aminophénylacétique ;
- 4-(2aminoéthyl)-1-carboxyméthyl-pipérazine ;
- acide trans-4-aminocyclohexanecarboxylique ;
- acide cis-4-aminocyclohexanecarboxylique ;
- acide cis-4-aminocyclohexane acétique ;
- acide trans-4-aminocyclohexane acétique ;
- 4-amino-1-carboxyméthyl pipéridine ;
- acide 4-aminobenzoïque ;
- acide 4(2-aminoéthoxy)benzoïque ;
• X_{b} est choisi parmi les résidus d'acides aminés suivants :
- D-alanine ;
- D-valine ;
- L-proline substituée en position β, γ ou δ ;
- D-proline substituée ou non en position β, γ ou δ ;
- acides aminés naturels N-alkylés, le groupe alkyle étant un groupe méthyle, éthyle ou benzyle ;
- acides aminés dialkylés acycliques de formule suivante : 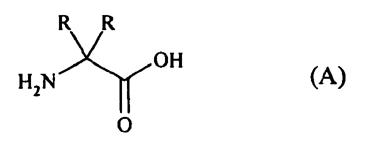 R représentant H, Me, Et, Pr ou Bu ;
- acides aminés dialkylés cycliques de formule suivante : 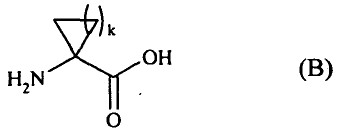 k représentant 1, 2, 3 ou 4 ;
• X_{c} est choisi parmi les résidus d'acides aminés suivants :
- tyrosine ;
- phénylalanine ;
- 3-nitro-tyrosine ;
- 4-hydroxyméthyl-phénylalanine ;
- 3,5-dihydroxy-phénylalanine ;
- 2,6-diméthyl-tyrosine ;
- 3,4-dihydroxy-phénylalanine (DOPA) ;
• X_{d} est choisi parmi les résidus d'acides aminés suivants :
- tyrosine ;
- phénylalanine ;
- 3-nitro-tyrosine ;
- 4-hydroxyméthyl-phénylalanine ;
- 3,5-dihydroxy-phénylalanine ;
- 2,6-diméthyl-tyrosine ;
- 3,4-dihydroxy-phénylalanine ;
• Xₑ est choisi parmi les résidus d'acides aminés suivants :
- NH₂-(CH₂)ₙ-COOH, n variant de 2 à 10 ;
- NH₂-(CH₂-CH₂-O)ₘ-CH₂CH₂COOH, m variant de 3 à 6 ;
- acide 8-amino-3,6-dioxaoctanoïque ;
- acide tranéxamique ;
- acide N-méthyl-tranéxamique ;
- acide 4(pipéridin-4-yl)butanoïque ;
- acide 3(pipéridin-4-yl)propionique ;
- N-(4-aminobutyl)-glycine ;
- 4-carboxyméthyl-pipérazine ;
- acide 4-(4-aminophényl)butanoïque ;
- acide 3-(4-aminophényl)propanoïque ;
- acide 4-aminophénylacétique ;
- 4-(2aminoéthyl)-1-carboxyméthyl-pipérazine ;
- acide trans-4-aminocyclohexanecarboxylique ;
- acide cis-4-aminocyclohexanecarboxylique ;
- acide cis-4-aminocyclohexane acétique ;
- acide trans-4-aminocyclohexane acétique ;
- 4-amino-1-carboxyméthyl pipéridine ;
- acide 4-aminobenzoïque ;
- acide 4(2-aminoéthoxy)benzoïque ;
• X_{f} est choisi parmi les résidus d'acides aminés suivants :
- NH₂-(CH₂)ₙ-COOH, n variant de 2 à 10 ;
- NH₂-(CH₂-CH₂-O)ₘ-CH₂CH₂COOH, m variant de 3 à 6 ;
- acide 8-amino-3,6-dioxaoctanoïque ;
- acide tranéxamique ;
- acide N-méthyl-tranéxamique ;
- acide 4(pipéridin-4-yl)butanoïque ;
- acide 3(pipéridin-4-yl)propionique ;
- N-(4-aminobutyl)-glycine ;
- 4-carboxyméthyl-pipérazine ;
- acide 4-(4-aminophényl)butanoïque ;
- acide 3-(4-aminophényl)propanoïque ;
- acide 4-aminophénylacétique ;
- 4-(2aminoéthyl)-1-carboxyméthyl-pipérazine ;
- acide trans-4-aminocyclohexanecarboxylique ;
- acide cis-4-aminocyclohexanecarboxylique ;
- acide cis-4-aminocyclohexane acétique ;
- acide trans-4-aminocyclohexane acétique ;
- 4-amino-1-carboxyméthyl pipéridine ;
- acide 4-aminobenzoïque ;
- acide 4(2-aminoéthoxy)benzoïque ;
• - L- représente une liaison de nature pseudopeptidique entre les résidus X'ₐ et X'_{b}, choisie notamment dans la liste ci-dessous :
- L- = -ψ(CH₂CH₂)- ; -ψ(CH(Fₖ)=CH(Fₖ'))- ; -ψ(CH₂NH)-; -ψ(NHCO)- ; -ψ(NHCONH)- ; -ψ(CO-O)- ; -ψ(CS-NH)- ; -ψ(CH(OH)-CH(OH) )-; -ψ(S-CH₂)-; -ψ(CH₂-S)- ; -ψ(CH(CN)-CH₂)- ; -ψ(CH(OH))- ; -ψ(COCH₂)-; -ψ(CH(OH)CH₂)- ; -ψ(CH(OH)CH₂NH)-; -ψ(CH₂)- ; -ψ(CH(Fₖ))- ; -ψ(CH₂O)- ; -ψ(CH₂-NHCONH)- ; -ψ(CH(Fₖ)NHCONFₖ')- ; -ψ(CH₂-CONH)- ; -ψ(CH(Fₖ)CONH)- ; -ψ(CH(Fₖ)CH(Fₖ')CONH)- ;
ou -ψ(CH₂N)- ; -ψ(NHCON)- ; -ψ(CS-N)- ; -ψ(CH(OH)CH₂N)- ; -ψ(CH₂-NHCON)- ; -ψ(CH₂-CON)- ; -ψ(CH(Fₖ)CON)- ; -ψ(CH(Fₖ)CH(Fₖ')CON)-lorsque X'_{b} représente la chaîne latérale d'une proline,
Fₖ et Fₖ' représentant, indépendamment l'un de l'autre, un hydrogène, un halogène, un groupe alkyle de 1 à 20 atomes de carbone, ou un groupe aryle dont la structure du cycle contient de 5 à 20 atomes de carbone,
• X'ₐ représente la chaîne latérale de la lysine, de l'arginine ou de l'ornithine ; et
• X'_{b} représente la chaîne latérale de l'un des résidus d'acides aminés suivants :
- glycine ;
- asparagine ;
- D-alanine ;
- D-valine ;
- L-proline substituée ou non en position β, γ ou δ ;
- D-proline substituée ou non en position β, γ ou δ ;
- acides aminés naturels N-alkylés, le groupe alkyle étant un groupe méthyle, éthyle ou benzyle ;
- acides aminés dialkylés acycliques de formule suivante : 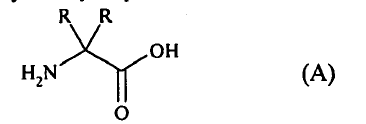 R représentant H, Me, Et, Pr ou Bu ;
- acides aminés dialkylés cycliques de formule suivante : 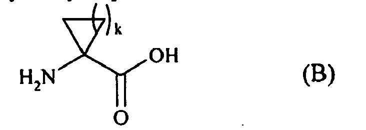 k représentant 1, 2, 3 ou 4.

2. Composé selon la revendication 1, **caractérisé en ce que** :
- Xₐ n'est pas un résidu de lysine.

3. Composé selon l'une quelconque des revendications 1 à 2, marqué par de la biotine.

4. Composé selon l'une quelconque des revendications 1 à 3, **caractérisé en ce que** Y répond à la formule suivante (II) : 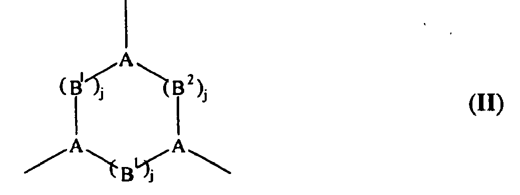 dans laquelle :
- j est 0 ou 1 ;
- A représente un résidu d'acide aminé ou un dérivé d'acide aminé choisi indifféremment parmi : 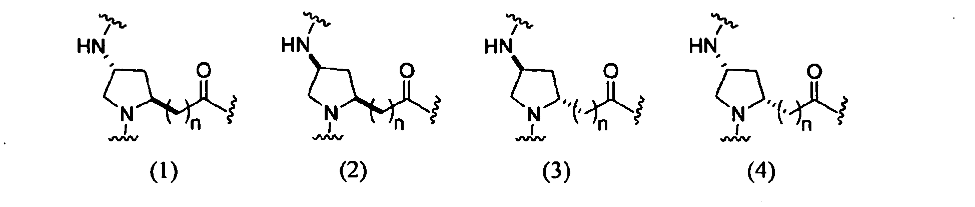 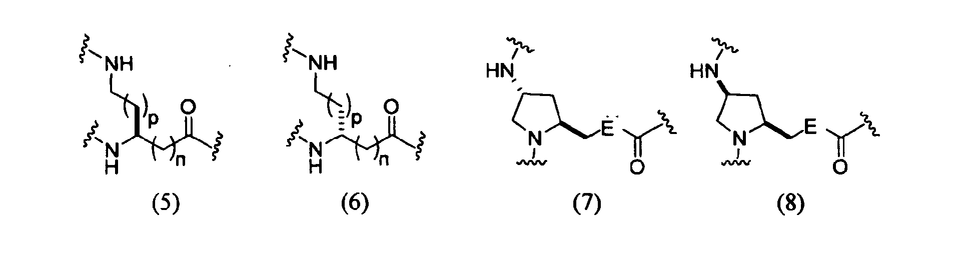 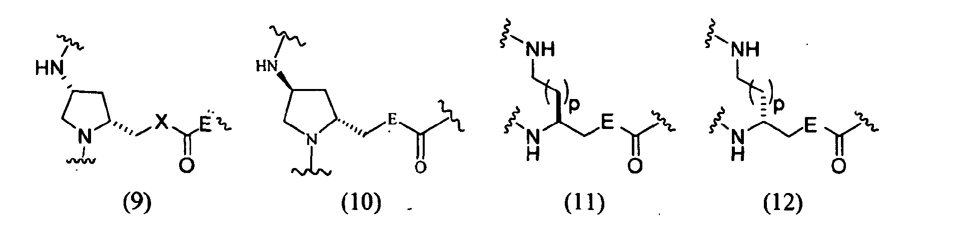
. n représentant 0, 1, 2 ou 3 ;
. p représentant 0, 1, 2 ou 3 ;
. E représentant NH ou O ;
- B¹ est un résidu d'acide aminé ou un dérivé d'acide aminé choisi de manière indépendante parmi : 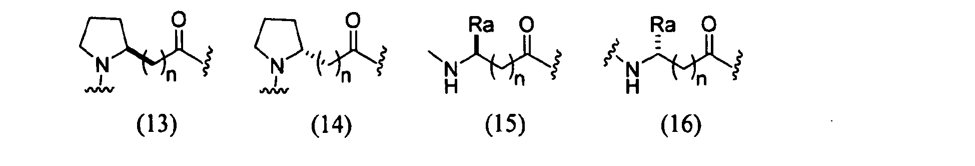 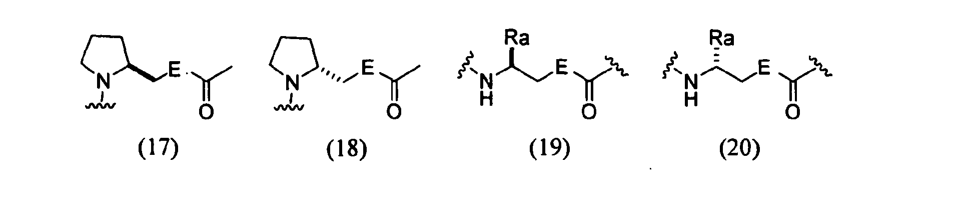
. n représentant 0, 1, 2 ou 3 ;
. p représentant 0,1, 2 ou 3 ;
. E représentant NH ou O ;
. Ra représentant la chaîne d'un acide aminé protéinogénique, C1-C8 alkylé ;
- B² peut être identique à B¹ ou choisi de manière indépendante parmi les groupes suivants : 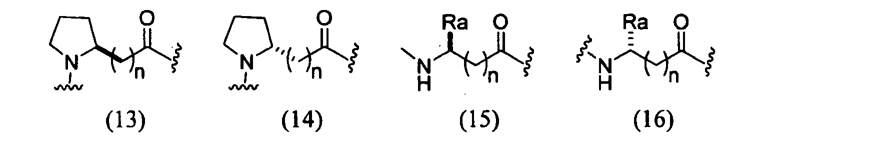 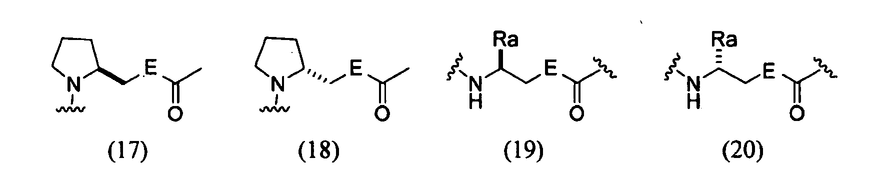 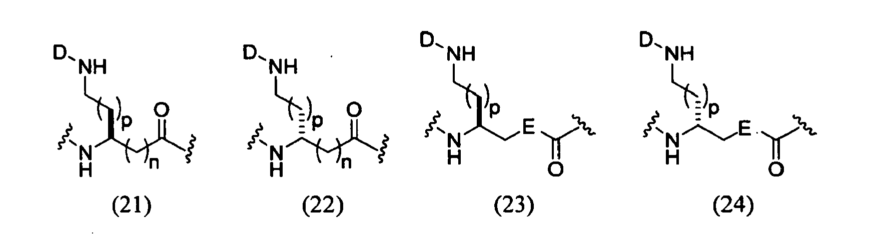
. n représentant 0, 1, 2 ou 3 ;
. p représentant 0, 1, 2 ou 3 ;
. E représentant NH ou O ;
. Ra représentant la chaîne d'un acide aminé protéinogénique, C1-C8 alkylé ;
. D représentant l'un des groupes suivants : (+)-Biotinyl-, (+)-Biotinyl-Xg-, HS-(CH₂)_{q}-CO-, Pys-S-(CH₂)_{q}-CO-, Npys-S-(CH₂)_{q}-CO-, HS-(CH₂)_{q}-CO-X_{g}-, Pys-S-(CH₂)_{q}-CO-X_{g}-, Npys-S-(CH₂)_{q}-CO-X_{g}-
q représentant un nombre entier variant de 2 à 6 ;
X_{g} correspondant au résidu de l'un des groupes suivants :
-NH₂-(CH₂)ₙ-COOH, n variant de 1 à 10 ;
-NH₂-(CH₂-CH₂-O)ₘ-CH₂CH₂COOH, m variant de 3 à 6 ;
acide 8-amino-3,6-dioxaoctanoïque ;
acide tranéxamique ;
acide N-méthyl-tranéxamique ;
acide 4(pipéridin-4-yl)butanoïque ;
acide 3(pipéridin-4-yl)-propionique ;
N-(4-aminobutyl)-glycine ;
4-carboxyméthyl-pipérazine ;
acide 4-(4-aminophenyl)butanoïque ;
acide 3-(4-aminophenyl)propanoïque ;
acide 4-aminophénylacétique ;
4-(2-aminoéthyl)-1-carboxyméthyl-pipérazine ;
acide trans-4-aminocyclohexane carboxylique ;
acide cis-4-aminocyclohexane carboxylique ;
acide cis-4-aminocyclohexane acétique ;
acide trans-4-aminocyclohexane acétique ;
4-amino-1-carboxyméthyl pipéridine ;
acide 4-aminobenzoïque ;
acide 4(2-aminoéthoxy)benzoïque.

5. Composé selon l'une quelconque des revendications 1 à 4, répondant à l'une des formules suivantes : 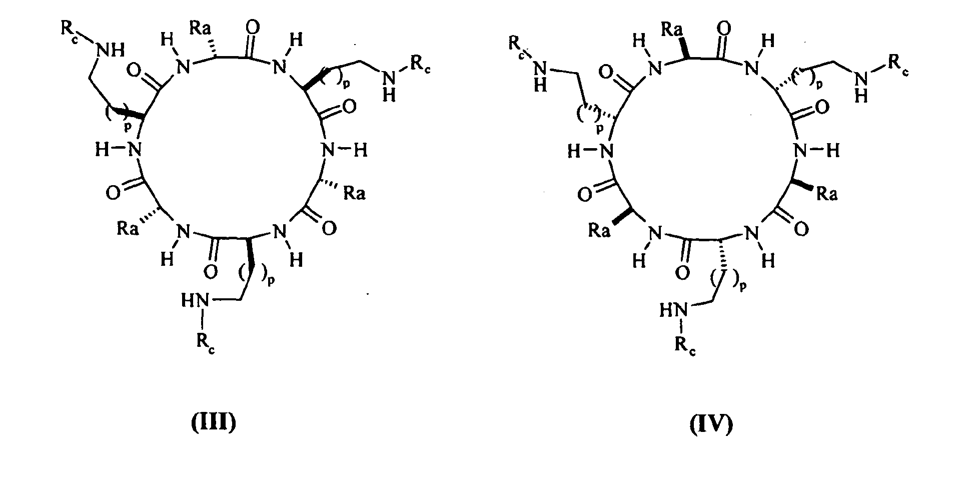 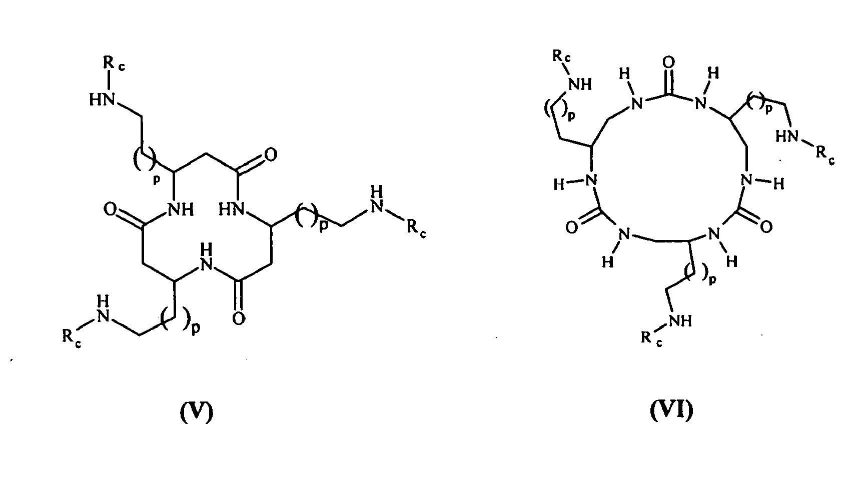 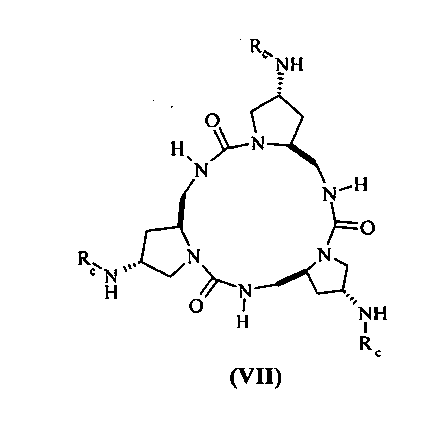 Ra, R_{c} et p étant tels que définis dans l'une quelconque des revendications 1 à 4.

6. Composé selon l'une quelconque des revendications 1 à 5, répondant à la formule suivante (III-a) :

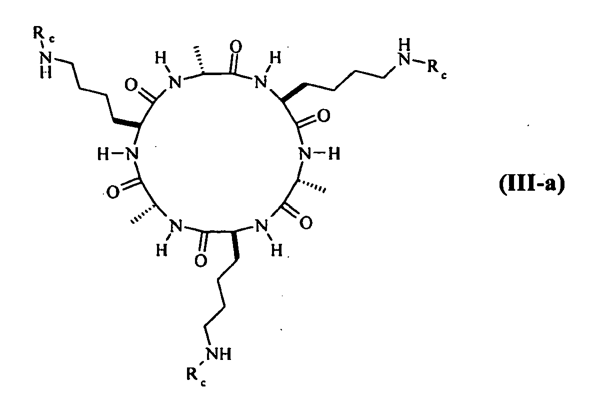

7. Composé selon l'une quelconque des revendications 1 à 5, répondant à la formule suivante (V-a) :

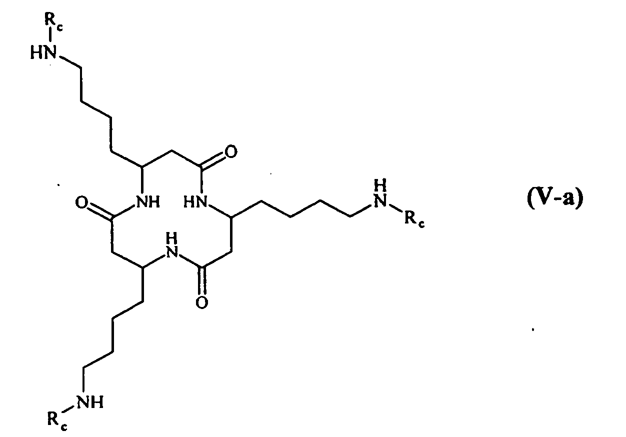

8. Composé selon l'une quelconque des revendications 1 à 5, répondant à l'une des formules suivantes : 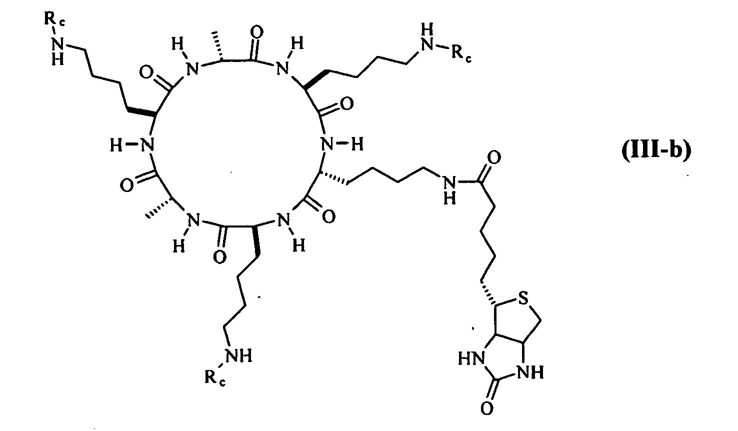 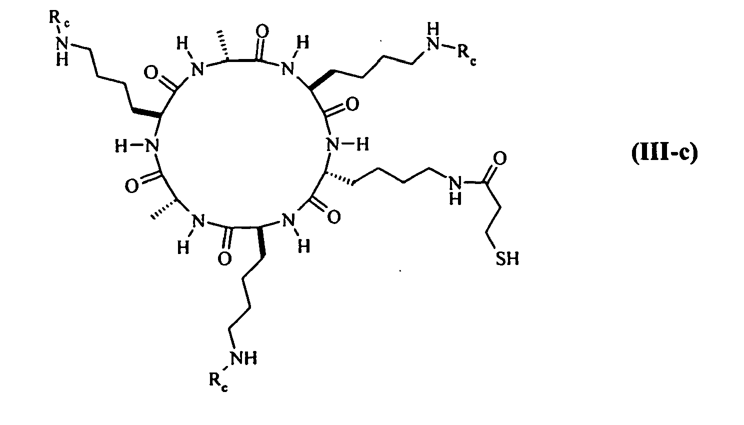 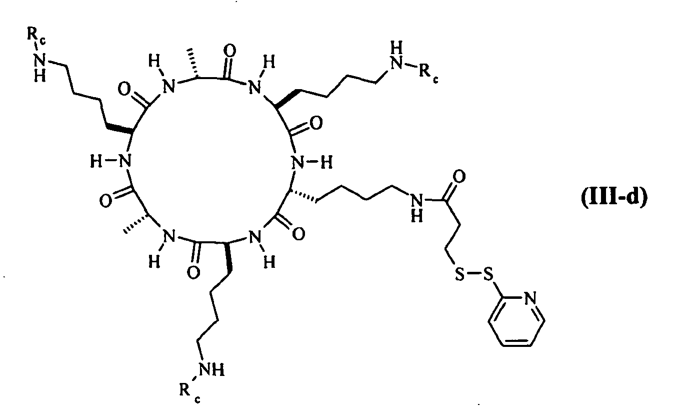 R_{c} étant tel que défini dans la revendication 1.

9. Composé selon l'une quelconque des revendications 1 à 8, dans lequel R_{c} est :
- H-Lys-(D)Pro-Tyr-Tyr-NH-(CH₂)₅-CO- ou
- Ahx-(D)Pro-Tyr-Tyr-NH-(CH₂)₅-CO- ou
- H-LYs-ψ(CH₂N)-(D)Pro-Tyr-Tyr-NH-(CH₂)₅-CO-, -ψ(CH₂N)- correspondant à une liaison pseudopeptidique de type méthylène-amino.

10. Composé selon l'une quelconque des revendications 1 à 7 et 9, répondant à l'une des formules suivantes :

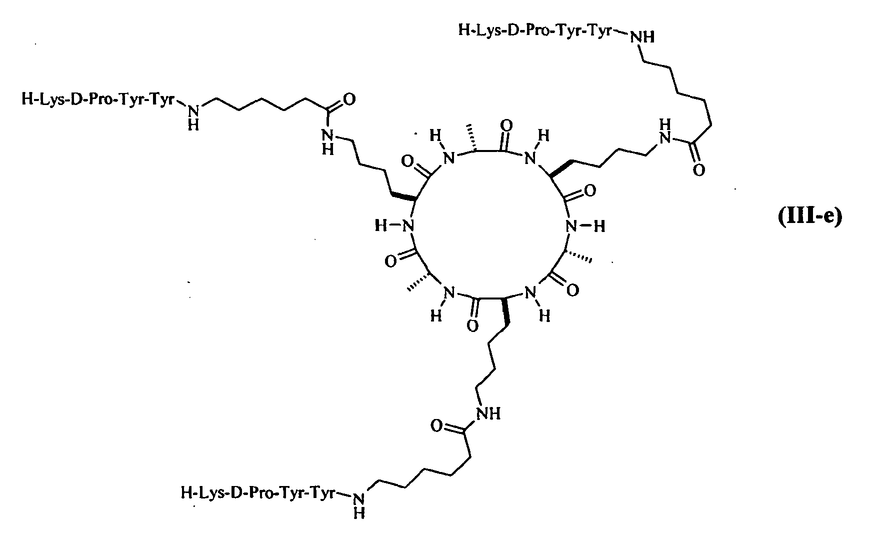

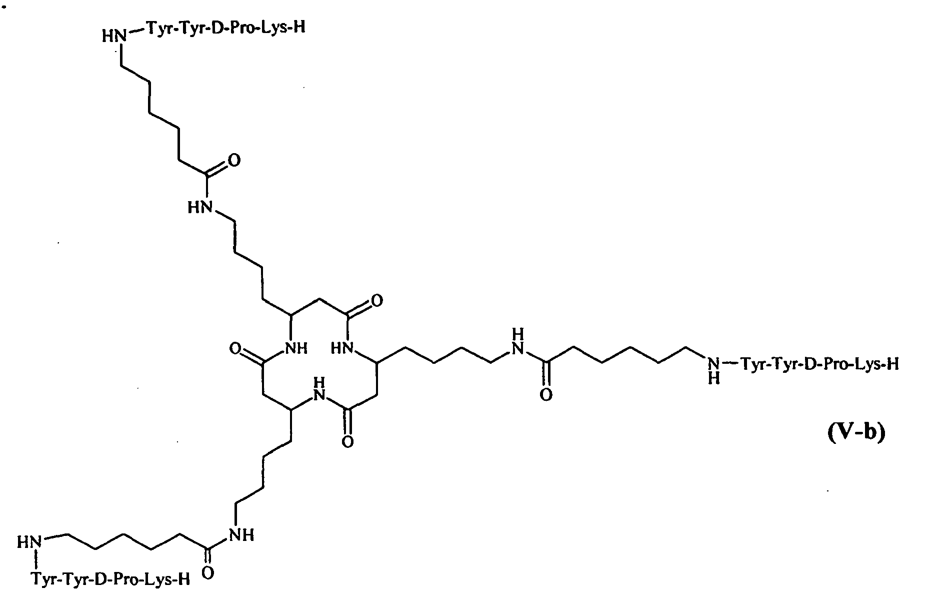

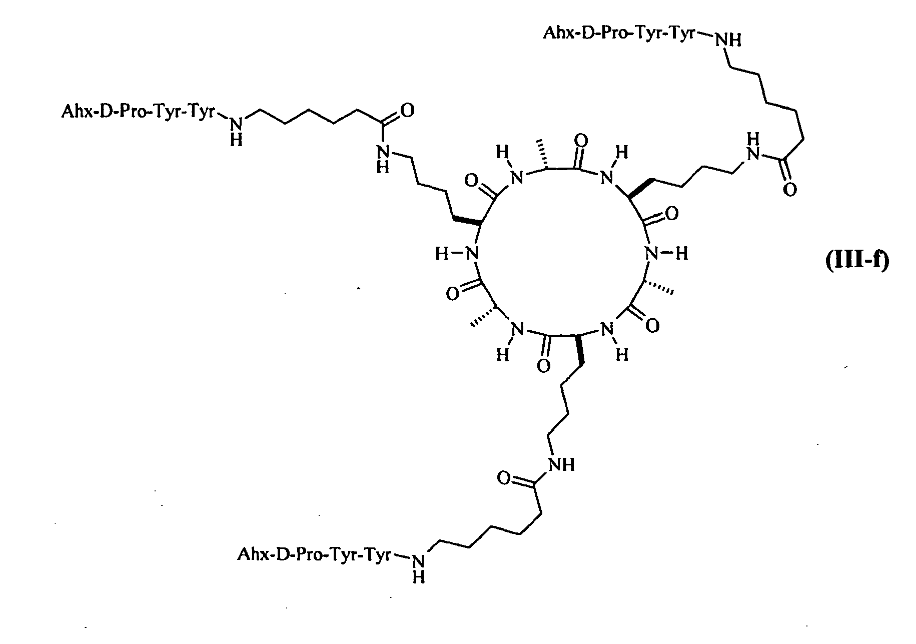

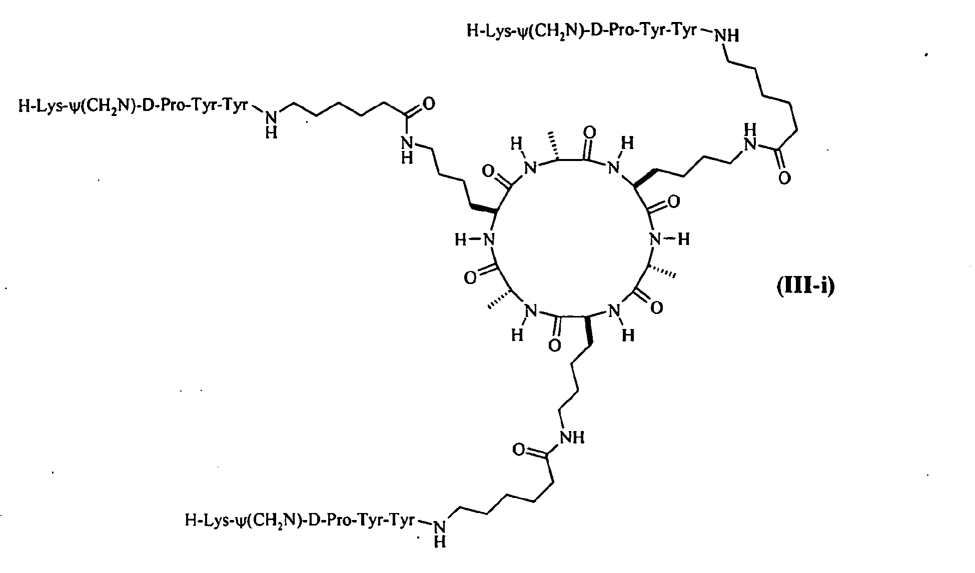

11. Composé selon l'une quelconque des revendications 1 à 5, 8 et 9, répondant à l'une des formules suivantes :

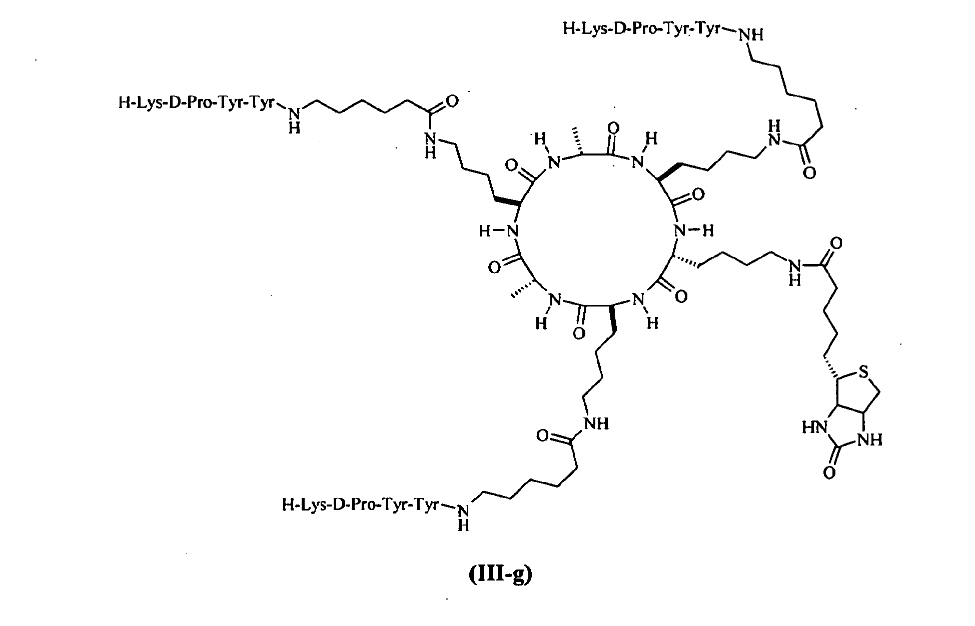

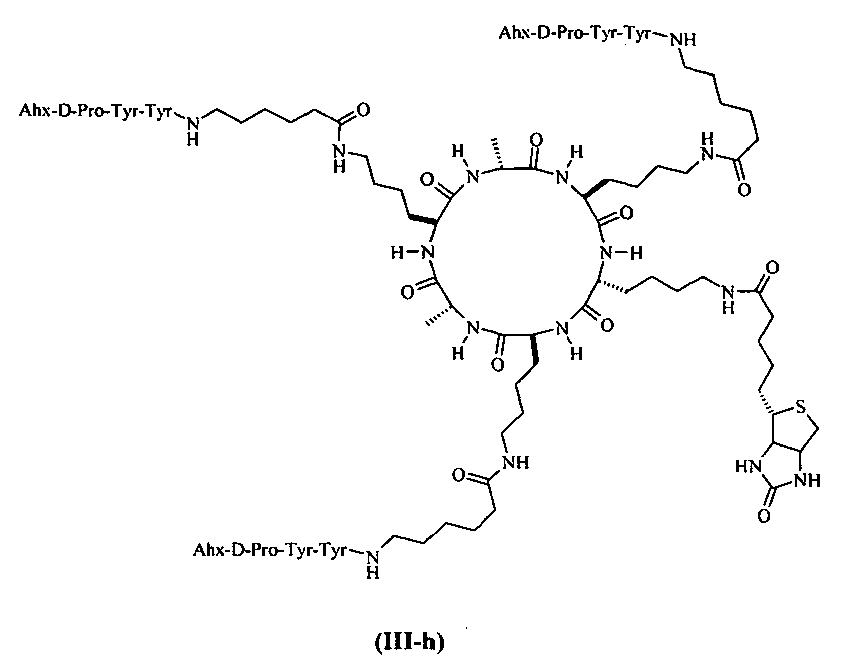

12. Composition pharmaceutique **caractérisée en ce qu'**elle comprend, à titre de substance active un composé selon l'une des revendications 1 à 11, en association avec un vecteur pharmaceutiquement acceptable.

13. Composition vaccinale, **caractérisée en ce qu'**elle comprend, à titre de substance active un composé selon l'une des revendications 1 à 11, en association avec un adjuvant pharmaceutiquement acceptable.

14. Utilisation de composés selon l'une des revendications 1 à 11, pour la préparation d'un médicament destiné au traitement de pathologies impliquant l'inhibition ou l'activation de la réponse immunitaire.

15. Utilisation selon la revendication 14, pour la préparation d'un médicament destiné au traitement de pathologies impliquant l'inhibition de la réponse immunitaire, telles que les rejets de greffes, les allergies ou les maladies auto-immunes.

16. Utilisation selon la revendication 14, pour la préparation d'un médicament destiné au traitement de pathologies impliquant l'augmentation de la réponse immunitaire, telles que les cancers ou les infections parasitaires, bactériennes, virales ou impliquant des agents infectieux non conventionnels tels que les prions.

17. Utilisation de composés selon la revendication 3, pour la préparation de ligands fonctionnalisés ou pour la purification de CD40.

18. Procédé de préparation sur support solide d'un composé, selon l'une des revendications 1 à 11, ledit procédé étant **caractérisé en ce qu'**il comprend les étapes suivantes :
- la formation d'un précurseur linéaire de Y tel que défini dans la revendication 1, lequel précurseur est constitué d'un enchaînement d'acides aminés formant une chaîne peptidique en croissance, synthétisée par des cycles successifs de couplage entre des résidus d'acides aminés N-protégés, dont trois portent un groupe Rₐ de type amine, Rₐ étant tel que défini dans la revendication 4, et la fonction amine de la chaîne peptidique en croissance, et de déprotection, le premier résidu d'acide aminé étant accroché sur un support solide,
- la cyclisation du précurseur linéaire de Y protégé susmentionné,
- le clivage des susdits groupes protecteurs, pour libérer les susdites fonctions amines protégées,
- le couplage des fonctions amines libérées susmentionné avec un peptide déjà formé ou formé in situ par l'assemblage séquentiel des résidus d'acides aminés correspondant au groupe R_{c} tel que défini dans la revendication 1, et
- le clivage de la molécule du support solide, après la suppression de tous les groupements protecteurs présents sur les chaînes latérales fonctionnalisées du groupe R_{c}, afin d'obtenir le composé selon l'une des revendications 1 à 11.

## Claims

1. Compound corresponding to the following formula (I): 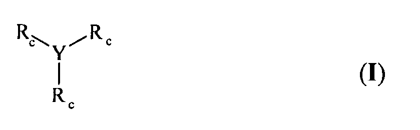 in which:
- Y represents a macrocycle the ring of which comprises 9 to 36 atoms, and is functionalized by three amine or COOH functions,
- R_{c} represents a group of formula H-Xₐ-X_{b}-X_{c}-X_{d}-Xₑ-(X_{f})ᵢ- or H-X'ₐ-L-X'_{b}-X_{c}-X_{d}-Xₑ-(X_{f})ᵢ-, in which:
• i represents 0 or 1,
• Xₐ is chosen from the following amino acid residues:
- lysine;
- arginine;
- ornithine;
- the β-amino acids corresponding to lysine, arginine or ornithine, carrying the substitution in α or β position;
- tranexamic acid;
- N-methyl-tranexamic acid;
- 8-amino-3,6-dioxaoctanoic acid;
- 4(piperidin-4-yl)butanoic acid;
- 3(piperidin-4-yl)propionic acid;
- N-(4-aminobutyl)-glycine;
- NH₂-(CH₂)ₙ-COOH, n varying from 1 to 10;
- NH₂-(CH₂-CH₂-O)ₘ-CH₂CH₂COOH, m varying from 3 to 6;
- 4-carboxymethyl-piperazine;
- 4-(4-aminophenyl)butanoic acid;
- 3-(4-aminophenyl)propanoic acid;
- 4-aminophenylacetic acid;
- 4-(2aminoethyl)-1-carboxymethyl-piperazine;
- trans-4-aminocyclohexanecarboxylic acid;
- cis-4-aniinocyclohexanecarboxylic acid;
- cis-4-aminocyclohexane acetic acid;
- trans-4-aminocyclohexane acetic acid;
- 4-amino-1-carboxymethyl piperidine;
- 4-aminobenzoic acid;
- 4(2-aminoethoxy)benzoic acid;
• X_{b} is chosen from the following amino acid residues:
- D-alanine;
- D-valine;
- L-proline substituted in β, γ or δ position;
- D-proline substituted or non-substituted in β, γ or δ position;
- N-alkylated natural amino acids, the alkyl group being a methyl, ethyl or benzyl group;
- acyclic dialkylated amino acids of the following formula: 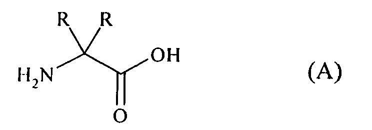 R representing H, Me, Et, Pr or Bu;
- cyclic dialkylated amino acids of the following formula: 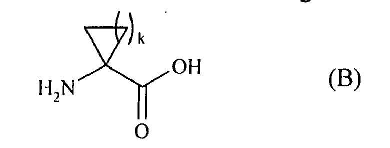 k representing 1, 2, 3 or 4;
• X_{c} is chosen from the following amino acid residues:
- tyrosine;
- phenylalanine;
- 3-nitro-tyrosine;
- 4-hydroxymethyl-phenylalanine;
- 3,5-dihydroxy-phenylalanine;
- 2,6-dimethyl-tyrosine;
- 3,4-dihydroxy-phenylalanine (DOPA);
• X_{d} is chosen from the following amino acid residues:
- tyrosine;
- phenylalanine;
- 3-nitro-tyrosine;
- 4-hydroxymethyl-phenylalanine;
- 3,5-dihydroxy-phenylalanine;
- 2,6-dimethyl-tyrosine;
- 3,4-dihydroxy-phenylalanine;
• X_{c} is chosen from the following amino acid residues:
- NH₂-(CH₂)ₙ-COOH, n varying from 2 to 10;
- NH₂-(CH₂-CH₂-O)ₘ-CH₂CH₂COOH, m varying from 3 to 6;
- 8-amino-3,6-dioxaoctanoic acid;
- tranexamic acid;
- N-methyl-tranexamic acid;
- 4(piperidin-4-yl)butanoic acid;
- 3(piperidin-4-yl)propionic acid;
- N-(4-aminobutyl)-glycine;
- 4-carboxymethyl-piperazine;
- 4-(4-aminophenyl)butanoic acid;
- 3-(4-aminophenyl)propanoic acid;
- 4-aminophenylacetic acid;
- 4-(2aminoethyl)-1-carboxymethyl-piperazine;
- trans-4-aminocyclohexanecarboxylic acid;
- cis-4-aminocyclohexanecarboxylic acid;
- cis-4-aminocyclohexane acetic acid;
- trans-4-aminocyclohexane acetic acid;
- 4-amino-1-carboxymethyl piperidine;
- 4-aminobenzoic acid;
- 4(2-aminoethoxy)benzoic acid;
• X_{f} is chosen from the following amino acid residues:
- NH₂-(CH₂)ₙ-COOH, n varying from 2 to 10;
- NH₂-(CH₂-CH₂-O)ₘ-CH₂CH₂COOH, m varying from 3 to 6;
- 8-amino-3,6-dioxaoctanoic acid;
- tranexamic acid;
- N-methyl-tranexamic acid;
- 4(piperidin-4-yl)butanoic acid;
- 3(piperidin-4-yl)propionic acid;
- N-(4-aminobutyl)-glycine;
- 4-carboxymethyl-piperazine;
- 4-(4-aminophenyl)butanoic acid;
- 3-(4-aminophenyl)propanoic acid;
- 4-aminophenylacetic acid;.
- 4-(2aminoethyl)-1-carboxymethyl-piperazine;
- trans-4-aminocyclohexanecarboxylic acid;
- cis-4-aminocyclohexanecarboxylic acid;
- cis-4-aminocyclohexane acetic acid;
- trans-4-aminocyclohexane acetic acid;
- 4-amino-1-carboxymethyl piperidine;
- 4-aminobenzoic acid;
- 4(2-aminoethoxy)benzoic acid;
• -L- represents a pseudopeptide-type bond between the X'ₐ and X'_{b} residues, chosen in particular from the list below:
- L- = -ψ(CH₂CH₂)-; -ψ(CH(Fₖ)=CH(Fₖ'))-; -ψ(CH₂NH)-; -ψ(NHCO)-; -ψ(NHCONH)-; -ψ(CO-O)-; -ψ(CS-NH)-; -ψ(CH(OH)-CH(OH) )-; -ψ(S-CH₂)-; -ψ(CH₂-S)-; -ψ(CH(CN)-CH₂)-; -ψ(CH(OH))-; -ψ(COCH₂)-; -ψ(CH(OH)CH₂)-; -ψ(CH(OH)CH₂NH)-; -ψ(CH₂)-; -ψ(CH(Fₖ))-; -ψ(CH₂O)-; -ψ(CH₂-NHCONH)-; -ψ(CH(Fₖ)NHCONFₖ)-; -ψ(CH₂-CONH)-; -ψ(CH(Fₖ)CONH)-; -ψ(CH(Fₖ)CH(Fₖ')CONH)-;
or -ψ(CH₂N)-; -ψ(NHCON)-; -ψ(CS-N)-; -ψ(CH(OH)CH₂N)-; -ψ(CH₂-NHCON)-; -ψ(CH₂-CON)-; -ψ(CH(Fₖ)CON)-; -ψ(CH(Fₖ)CH(Fₖ')CON)-when X'_{b} represents the side chain of a proline,
Fₖ and Fₖ' representing, independently of one another, a hydrogen, a halogen, an alkyl group of 1 to 20 carbon atoms, or an aryl group the ring structure of which contains from 5 to 20 carbon atoms,
• X'ₐ represents the side chain of lysine, arginine or ornithine; and
• X'_{b} represents the side chain of one of the following amino acid residues:
- glycine;
- asparagine;
- D-alanine;
- D-valine;
- L-proline substituted or non-substituted in β, γ or δ position;
- D-proline substituted or non-substituted in β, γ or δ position;
- N-alkylated natural amino acids, the alkyl group being a methyl, ethyl or benzyl group;
- acyclic dialkylated amino acids of the following formula: 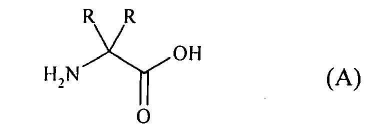 R representing H, Me, Et, Pr or Bu;
- cyclic dialkylated amino acids of the following formula: 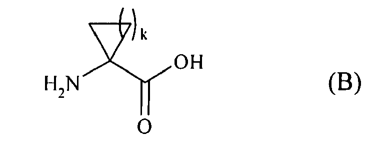 k representing 1, 2, 3 or 4.

2. Compound according to any one of claims 1, **characterized in that**:
- X is not a lysine residue.

3. Compound according to any one of claims 1 to 2, labelled with biotin.

4. Compound according to any one of claims 1 to 3, **characterized in that** Y corresponds to the following formula (II): 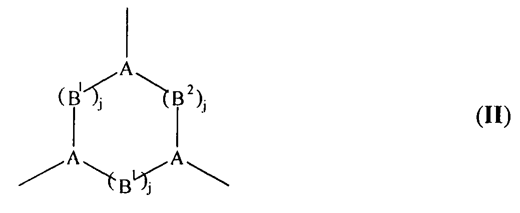 in which:
- j is 0 or 1;
- A represents an amino acid residue or an amino acid derivative chosen from any of: 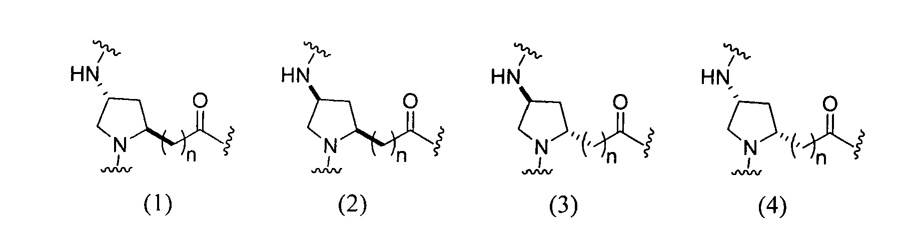 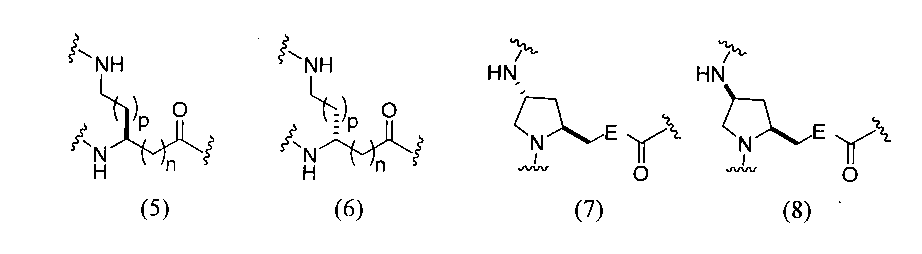 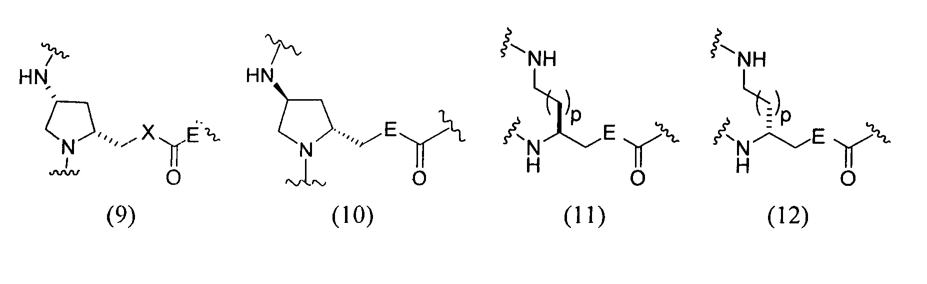
. n representing 0, 1, 2 or 3;
. p representing 0, 1, 2 or 3;
. E representing NH or O;
- B¹ is an amino acid residue or an amino acid derivative chosen independently from: 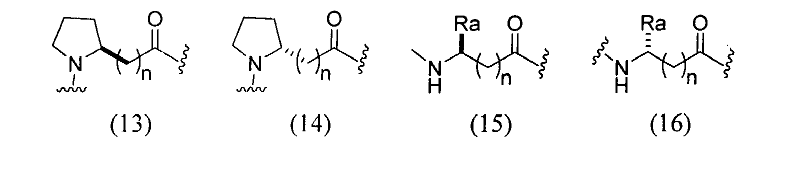 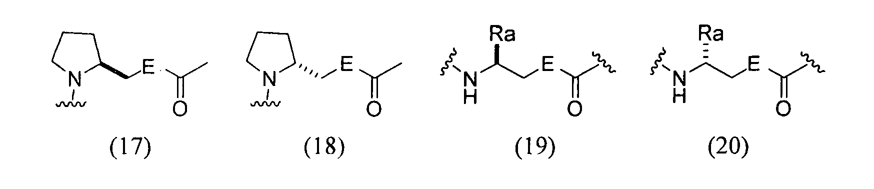
. n representing 0, 1, 2 or 3;
. p representing 0, 1, 2 or 3;
. E representing NH or O;
. Ra representing the chain of a proteinogenic amino acid, C1-C8 alkylated;
- B² can be identical to B¹ or chosen independently from the following groups: 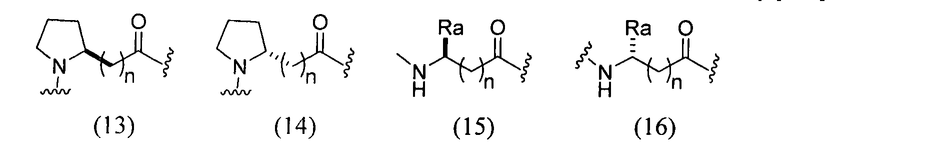 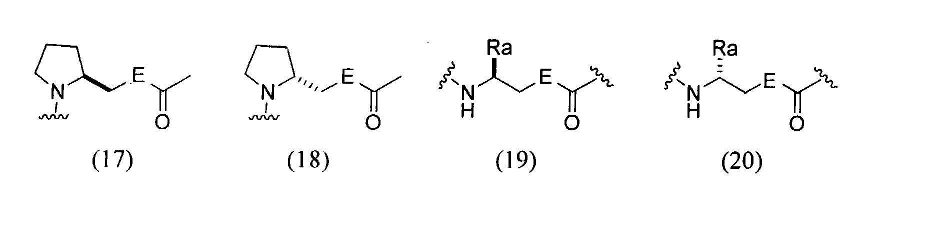 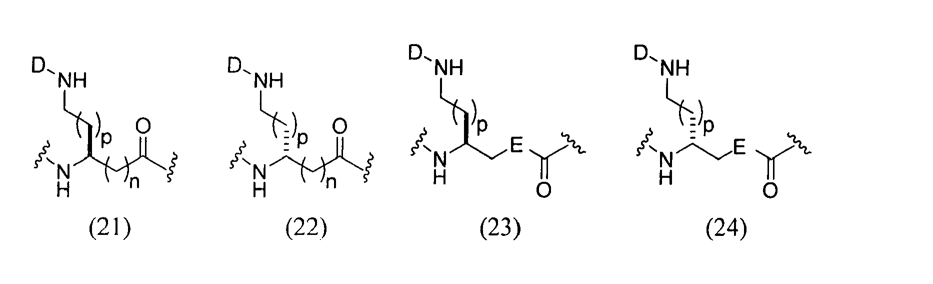
. n representing 0, 1, 2 or 3;
. p representing 0, 1, 2 or 3;
. E representing NH or O;
. Ra representing the chain of a proteinogenic amino acid, C1-C8 alkylated;
. D representing one of the following groups: (+)-Biotinyl-, (+)-Biotinyl-Xg-, HS-(CH₂)_{q}-CO-, Pys-S-(CH₂)_{q}-CO-, Npys-S-(CH₂)_{q}-CO-, HS-(CH₂)_{q}-CO-X,-, Pys-S-(CH₂)_{q}-CO-X_{g}-, Npys-S-(CH₂)_{q}-CO-X_{g}-
q representing an integer varying from 2 to 6;
X_{g} corresponding to the residue of one of the following groups:
-NH₂-(CH₂)ₙ-COOH, n varying from 1 to 10;
-NH₂-(CH₂-CH₂-O)ₘ-CH₂CH₂COOH, m varying from 3 to 6;
8-amino-3,6-dioxaoctanoic acid;
tranexamic acid;
N-methyl-tranexamic acid;
4(piperidin-4-yl)butanoic acid;
3(piperidin-4-yl)-propionic acid;
N-(4-aminobutyl)-glycine;
4-carboxymethyl-piperazine;
4-(4-aminophenyl)butanoic acid;
3-(4-aminophenyl)propanoic acid;
4-aminophenylacetic acid;
4-(2-aminoethyl)-1-carboxymethyl-piperazine;
trans-4-aminocyclohexane carboxylic acid;
cis-4-aminocyclohexane carboxylic acid;
cis-4-aminocyclohexane acetic acid;
trans-4-aminocyclohexane acetic acid;
4-amino-1-carboxymethyl piperidine;
4-aminobenzoic acid;
4(2-aminoethoxy)benzoic acid.

5. Compound according to any one of claims 1 to 4, corresponding to one of the following formulae: 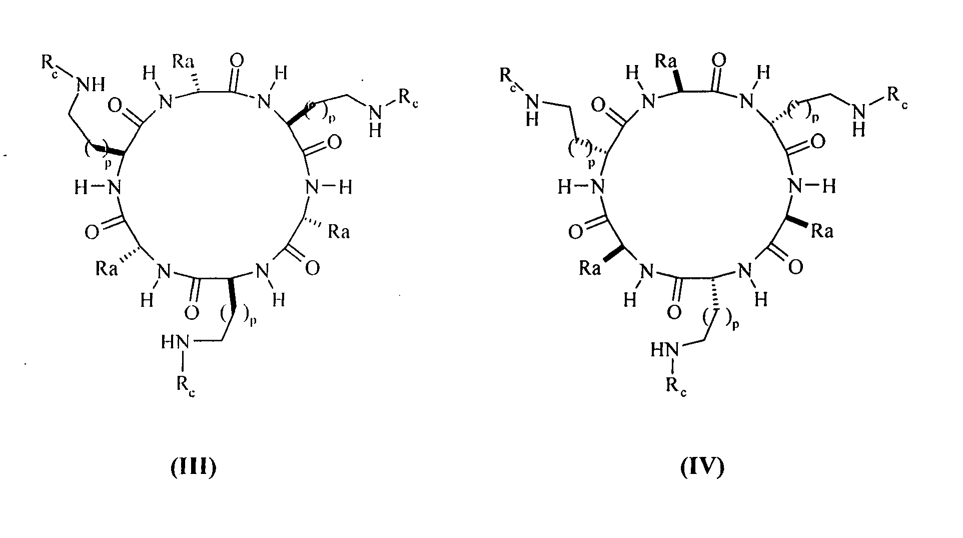 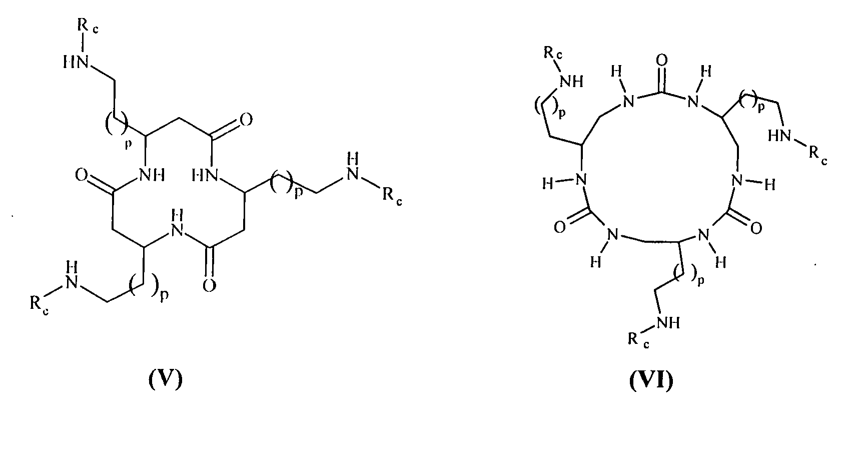 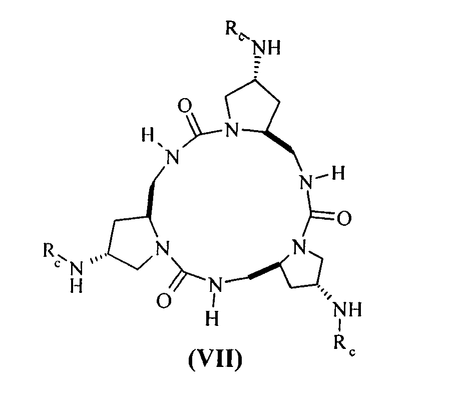 Ra, R_{c} and p being as defined in any one of claims 1 to 4.

6. Compound according to any one of claims 1 to 5, corresponding to the following formula (III-a):

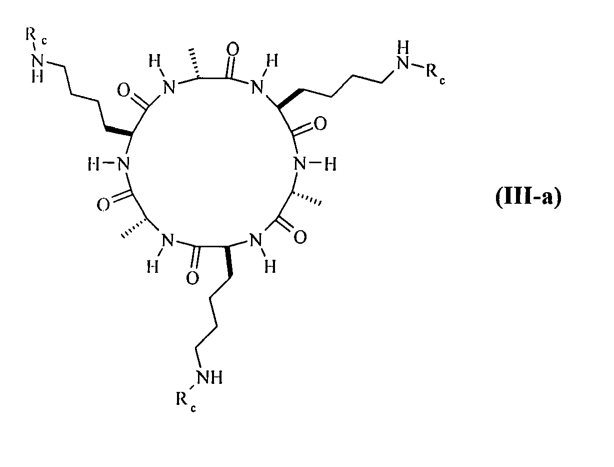

7. Compound according to any one of claims 1 to 5, corresponding to the following formula (V-a):

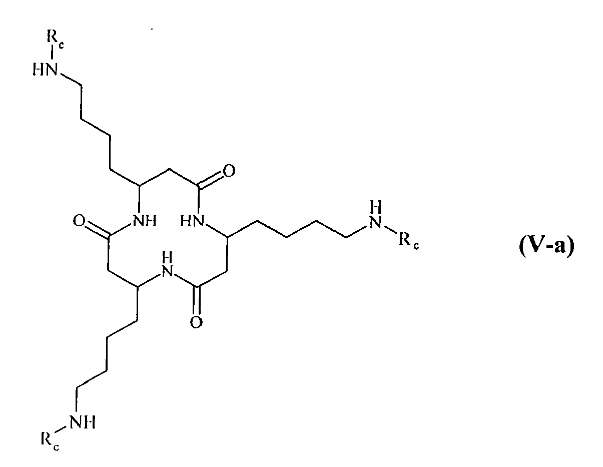

8. Compound according to any one of claims 1 to 5, corresponding to one of the following formulae: 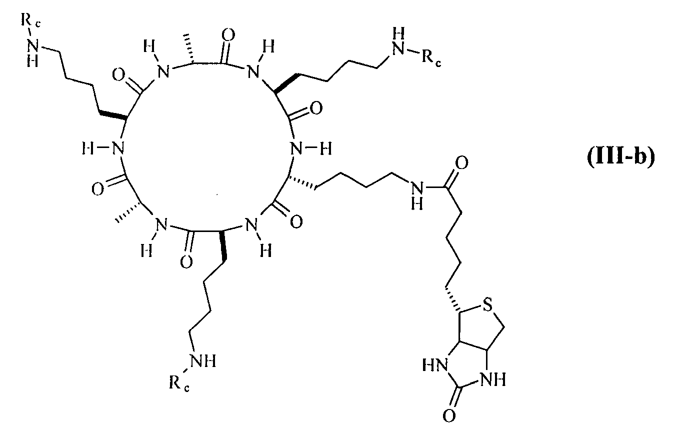 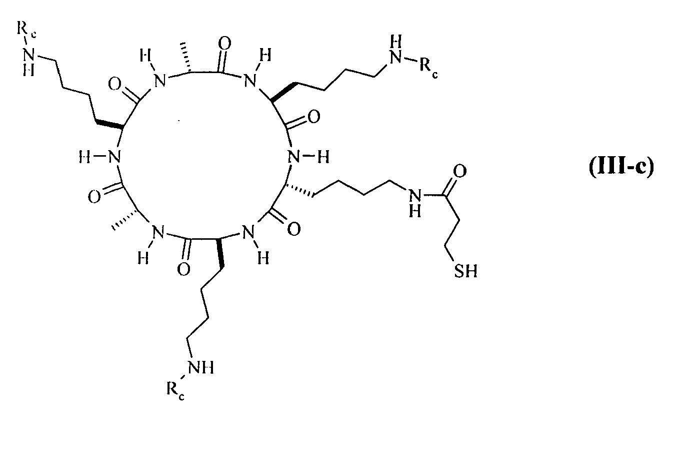 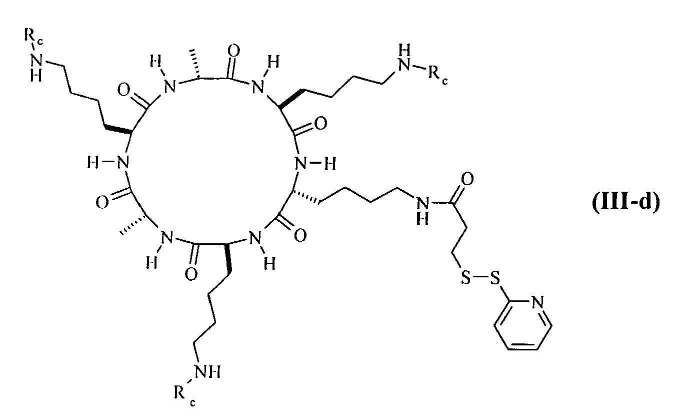 R_{c} being as defined in claim 1.

9. Compound according to any one of claims 1 to 8, in which R_{c} is:
- H-Lys-(D)Pro-Tyr-Tyr-NH-(CH₂)₅-CO- or
- Ahx-(D)Pro-Tyr-Tyr-NH-(CH₂)₅-CO- or
- H-Lys-ψ(CH₂N)-(D)Pro-Tyr-Tyr-NH-(CH₂)₅-CO-, -ψ(CH₂N)- corresponding to a pseudopeptide bond of methylene-amino type.

10. Compound according to any one of claims 1 to 7 and 9, corresponding to one of the following formulae:

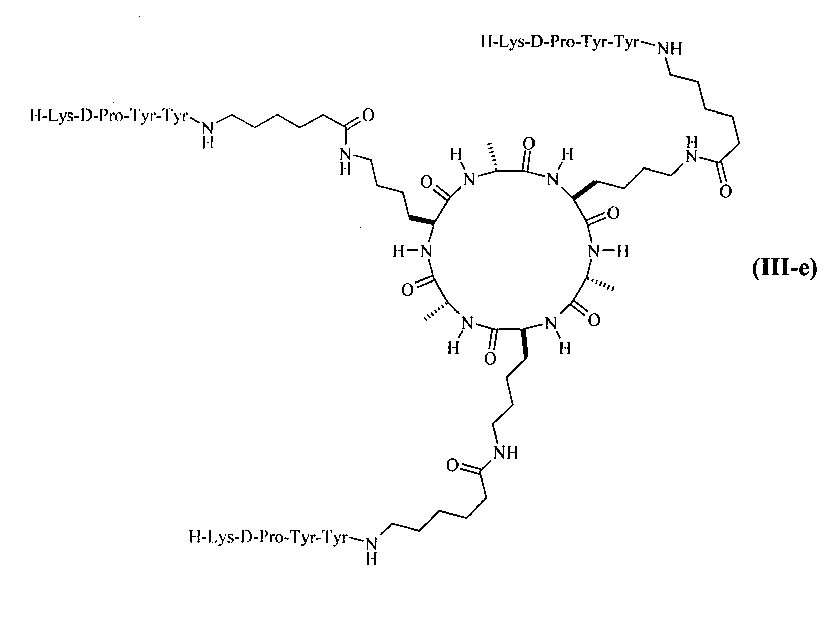

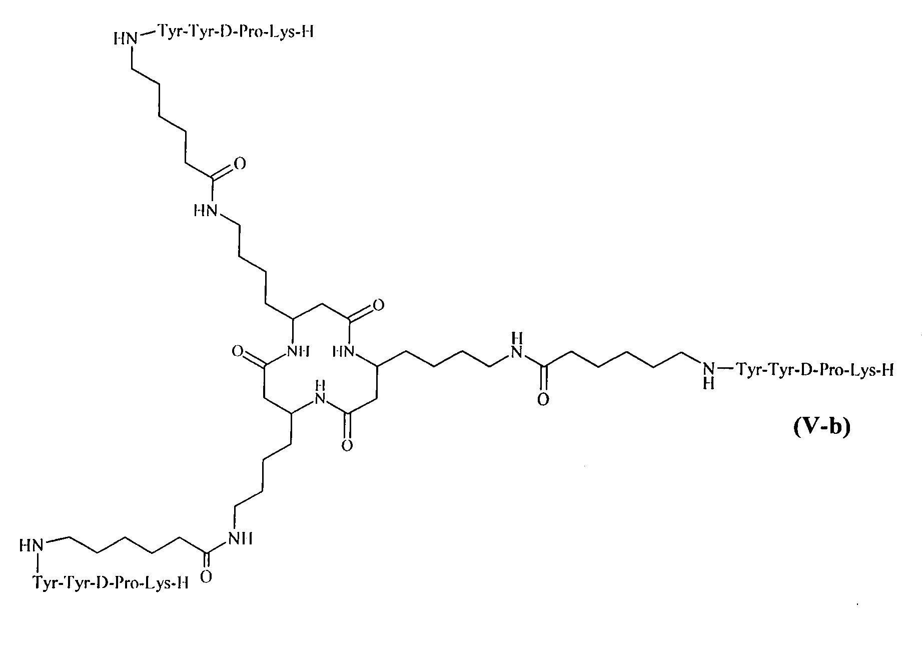

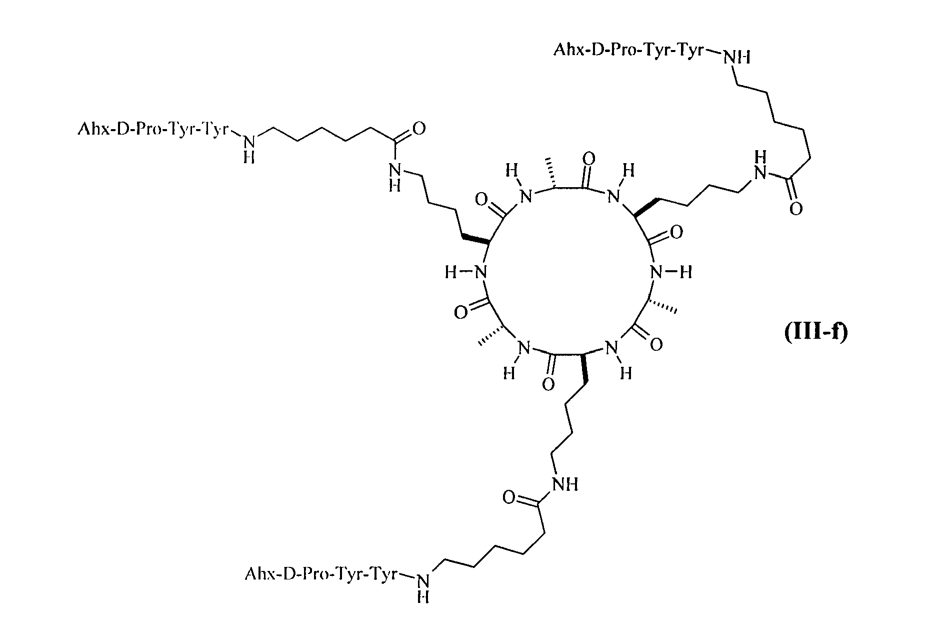

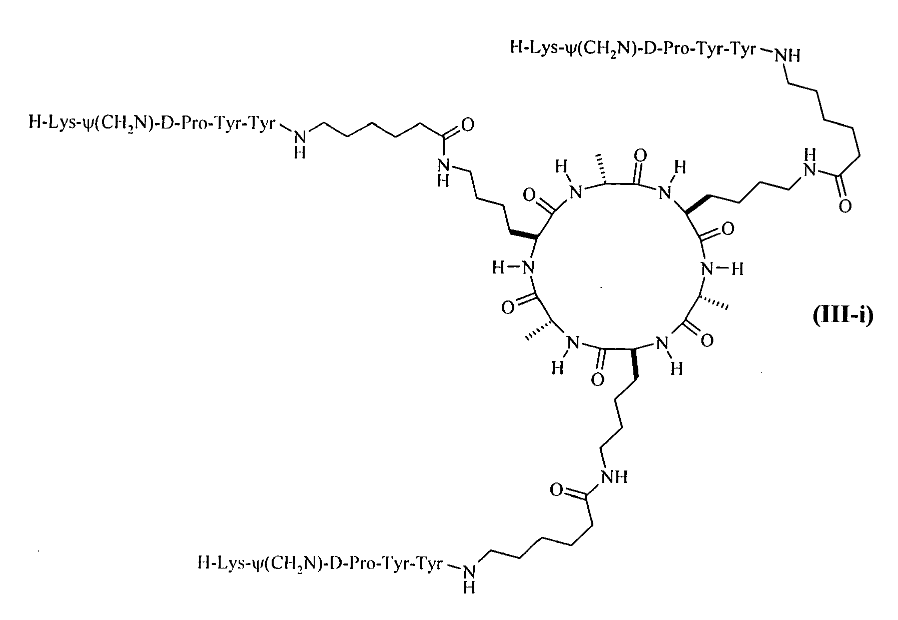

11. Compound according to any one of claims 1 to 5, 8 and 9, corresponding to one of the following formulae:

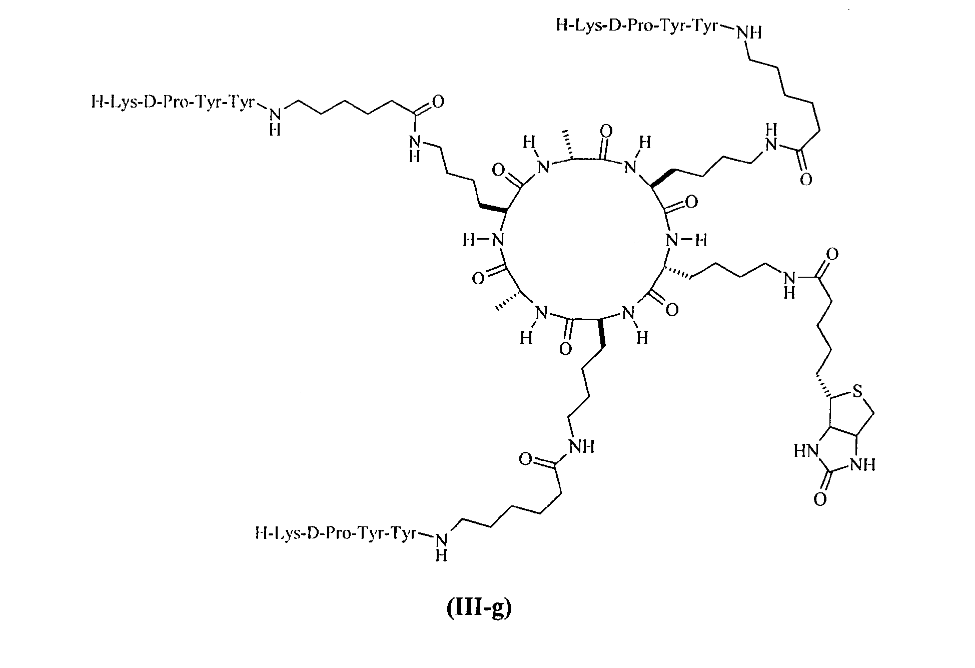

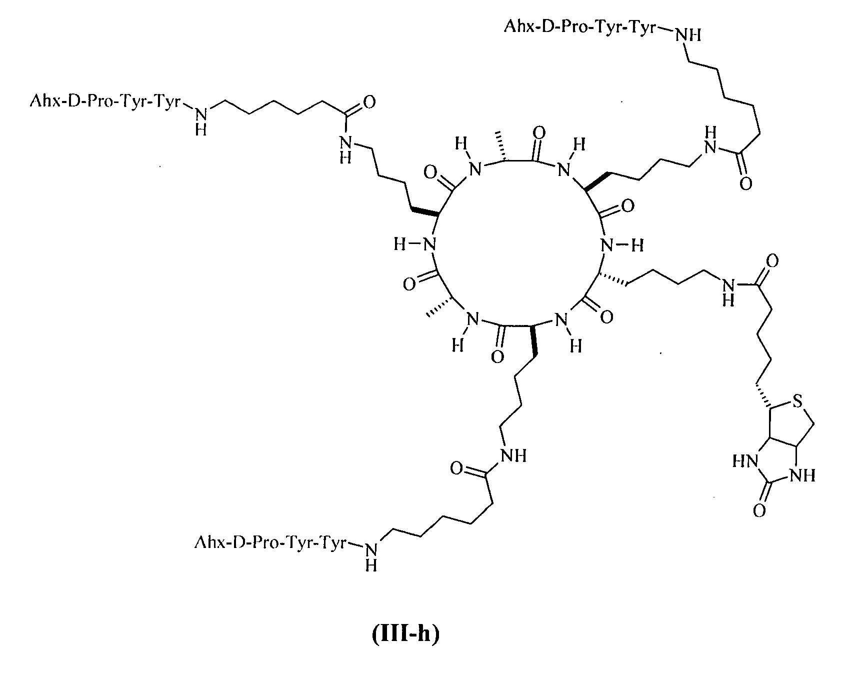

12. Pharmaceutical composition **characterized in that** it comprises, as active ingredient, a compound according to one of claims 1 to 11, in combination with a pharmaceutically acceptable vector.

13. Vaccine composition **characterized in that** it comprises, as active ingredient, a compound according to one of claims 1 to 11, in combination with a pharmaceutically acceptable adjuvant.

14. Use of compounds according to one of claims 1 to 11, for the preparation of a medicament intended for the treatment of pathologies involving the inhibition or the activation of the immune response.

15. Use according to claim 14, for the preparation of a medicament intended for the treatment of pathologies involving the inhibition of the immune response, such as graft rejections, allergies or auto-immune diseases.

16. Use according to claim 14, for the preparation of a medicament intended for the treatment of pathologies involving the boosting of the immune response, such as cancers or parasitic, bacterial, viral infections or infections involving non-conventional infectious agents such as prions.

17. Use of compounds according to claim 3, for the preparation of functionalized ligands or for the purification of CD40.

18. Process for the preparation on solid support of a compound, according to one of claims 1 to 11, said process being **characterized in that** it comprises the following stages:
- the formation of a linear precursor of Y as defined in claim 1, which precursor is constituted by a chaining of amino acids forming a growing peptide chain, synthesized by successive cycles of coupling between N-protected amino acid residues, three of which carry an Rₐ group of amine type, Rₐ being as defined in claim 4, and the amine function of the growing peptide chain, and of deprotection, the first amino acid residue being attached to a solid support,
- the cyclization of the abovementioned protected linear precursor of Y,
- the cleavage of said protective groups, in order to release said protected amine functions,
- the coupling of the abovementioned released amine functions with a peptide which is already formed or formed in situ by the sequential assembly of the amino acid residues corresponding to the R_{c} group as defined in claim 1, and
- the cleavage of the molecule from the solid support, after the deletion of all the protective groups present on the functionalized side chains of the R_{c} group, in order to obtain the compound according to one of claims 1 to 11.

## Patentansprüche

1. Verbindung mit der folgenden Formel (I) : 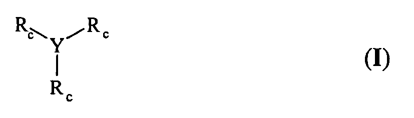 worin :
- Y einen Makrozyclus mit 9 - 36 Atomen im Zyklus bedeutet, und mit drei Aminfunktionen oder COOH funktionalisiert wird,
- R_{c} eine Gruppe der Formel H-Xₐ-X_{b}-X_{c}-X_{d}-Xₑ-(X_{f})ᵢ- oder H-X'ₐ-L-X'_{b}-X_{c}-X_{d}-Xₑ-(X_{f})ᵢ- bedeutet, worin :
• i 0 oder 1 bedeutet,
• Xₐ zwischen den folgenden Aminosäureresten gewählt wird :
- Lysin;
- Arginin ;
- Ornithin;
- die der Lysin, der Arginin oder der Omithin entsprechenden β-Aminosäuren mit der Substitution in der α oder β Position ;
- Tranexamische Säure ;
- N-methyl-tranexamische Säure ;
- 8-amino-3,6-dioxaoctansäure ;
- 4(piperidin-4-yl)butansäure ;
- 3(piperidin-4-yl)propionsäure ;
- N-(4-aminobutyl)-Glycin ;
- NH₂-(CH₂)ₙ-COOH, worin n zwischen 1 - 10 liegt;
- NH₂-(CH₂-CH₂-O)ₘ-CH₂CH₂COOH, worin m zwischen 3 - 6 liegt ;
- 4-carboxymethyl-Piperazin ;
- 4-(4-aminophenyl)Butansäure ;
- 3-(4-aminophenyl)Propansäure ;
- 4-aminophenylessigsäure ;
- 4-(2aminoethyl)-1-carboxymethyl-Piperazin ;
- Trans-4-aminocyclohexancarbonsäure ;
- Cis-4-aminocyclohexancarbonsäure ;
- Cis-4-aminocyclohexanessigsäure ;
- Trans-4-aminocyclohexanessigsäure ;
- 4-amino-1-carboxymethyl Piperidin;
- 4-aminobenzoesäure ;
- 4(2-aminoethoxy)Benzoesäure ;
• X_{b} wird zwischen den folgenden Aminosäureresten gewählt :
- D-Alanin ;
- D-Valin ;
- L-Prolin in der β, γ oder δ Position substituiert ;
- D-Prolin, ggf. in der β, γ oder δ Position substituiert ;
- N-alkylierte natürliche Aminosäuren, worin die Alkylgruppe eine Methyl-, Äthyl- oder Benzylgruppe ist;
- Azyklische dialkylierte Aminosäuren mit der folgenden Formel : 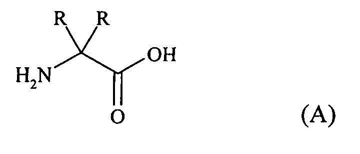 worin R H, Me, Et, Pr oder Bu bedeutet;
- Zyklische dialkylierte Aminosäuren mit der folgenden Formel : 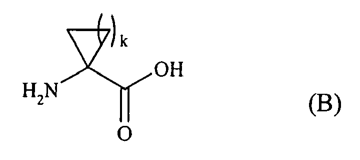 worin k 1, 2, 3 oder 4 bedeutet ;
• X_{c} wird zwischen den folgenden Aminosäureresten gewählt :
- Tyrosin ;
- Phenylalanin ;
- 3-nitro-Tyrosin ;
- 4-hydroxymethyl-Phenylalanin ;
- 3,5-dihydroxy-Phenylalanin ;
- 2,6-dimethyl-Tyrosin ;
- 3,4-dihydroxy-Phenylalanin (DOPA) ;
• X_{d} wird zwischen den folgenden Aminosäureresten gewählt :
- Tyrosin ;
- Phenylalanin ;
- 3-nitro-Tyrosin ;
- 4-hydroxymethyl-Phenylalanin ;
- 3,5-dihydroxy-Phenylalanin ;
- 2,6-dimethyl-Tyrosin ;
- 3,4-dihydroxy-Phenylalanin ;
• Xₑ wird zwischen den folgenden Aminosäureresten gewählt :
- NH₂-(CH₂)ₙ-COOH, worin n zwischen 1 und 10 liegt;
- NH₂-(CH₂-CH₂-O)ₘ-CH₂CH₂COOH, worin m zwischen 3 und 6 liegt ;
- 8-amino-3,6-Dioxaoctansäure ;
- Tranexamische Säure ;
- N-methyl-tranexamische Säure ;
- 4(piperidin-4-yl)Butansäure ;
- 3(piperidin-4-yl)Propionsäure ;
- N-(4-aminobutyl)-Glycin ;
- 4-carboxymethyl-Piperazin ;
- 4-(4-aminophenyl)Butansäure ;
- 3-(4-aminophenyl)Propansäure ;
- 4-aminophenylessigsäure ;
- 4-(2aminoethyl)-1-carboxymethyl-Piperazin ;
- Trans-4-aminocyclohexancarbonsäure ;
- Cis-4-aminocyclohexancarbonsäure ;
- Cis-4-aminocyclohexanessigsäure ;
- Trans-4-aminocyclohexanessigsäure ;
- 4-amino-1-carboxymethyl Piperidin;
- 4-aminobenzoesäure ;
- 4(2-aminoethoxy)Benzoesäure ;
• X_{f} wird zwischen den folgenden Aminosäureresten gewählt :
- NH₂-(CH₂)ₙ-COOH, worin n zwischen 1 und 10 liegt ;
- NH₂-(CH₂-CH₂-O)ₘ-CH₂CH₂COOH, worin m zwischen 3 und 6 liegt ;
- 8-amino-3,6-Dioxaoctansäure ;
- Tranexamische Säure ;
- N-methyl-tranexamische Säure ;
- 4(piperidin-4-yl)Butansäure ;
- 3(piperidin-4-yl)Propionsäure ;
- N-(4-aminobutyl)-Glycin ;
- 4-carboxymethyl-Piperazin ;
- 4-(4-aminophenyl)Butansäure ;
- 3-(4-aminophenyl)Propansäure ;
- acide 4-aminophenylessigsäure ;
- 4-(2aminoethyl)-1-carboxymethyl-Piperazin ;
- Trans-4-aminocyclohexancarbonsäure ;
- Cis-4-aminocyclohexancarbosäure ;
- Cis-4-aminocyclohexanessigsäure ;
- Trans-4-aminocyclohexanessigsäure ;
- 4-amino-1-carboxymethyl Piperidin ;
- 4-aminobenzoesäure ;
- 4(2-aminoethoxy)Benzoesäure ;
• -L- eine pseudopeptidische Bindung zwischen den Resten X'ₐ und X'_{b} bedeutet, und zwar insbesondere unter der folgenden Auflistung :
- L- = -ψ(CH₂CH₂)- -ψ(CH(Fₖ)=CH(Fₖ'))- ; -ψ(CH₂NH)- ; -ψ(NHCO)-; -ψ(NHCONH)- ; -ψ(CO-O)- ; -ψ(CS-NH)- ; -ψ(CH(OH)-CH(OH) )- ; -ψ(S-CH₂)- ; -ψ(CH₂-S)- ; -ψ(CH(CN)-CH₂)- ; -ψ(CH(OH))- ; -ψ(COCH₂)- ; -ψ(CH(OH)CH₂)- ; -ψ(CH(OH)CH₂NH)- ; -ψ(CH₂)- ; -ψ(CH(Fₖ))- ; -ψ(CH₂O)- ; -ψCH₂-NHCONH)- ; -ψCH(Fₖ)NHCONFₖ')- ; -ψCH₂-CONH)- ; -ψCH(Fₖ)CONH)- ; -ψCH(Fₖ)CH(Fₖ')CONH)- ;
oder -ψ(H₂N)- -ψ(HCON)- ; -ψ(CS-N)- ; -ψ(CH(OH)CH₂N)- ; -ψ(H₂-NHCON)- -ψ(CH₂-CON)-; -ψ(CH(Fₖ)CON)- ; -ψ(CH(Fₖ)CH(Fₖ')CON)-wenn X'_{b} eine Prolin-Seitenkette bedeutet,
Fₖ und Fₖ' unabhängig ein Wasserstoff, ein Halogen, eine Alkylgruppe mit 1 - 20 Kohlenstoffatomen, oder eine Arylgruppe mit 5 - 20 Kohlenstoffatomen im Zyklus bedeuten,
• X'ₐ die Seitenkette der Lysin, der Arginin oder der Omithin bedeutet ; und
• X'_{b} die Seitenkette von einem Aminosäurerest bedeutet, und zwar unter den folgenden :
- Glycin ;
- Asparagin ;
- D-Alanin ;
- D-Valin ;
- L-Prolin in der β, γ oder δ Position substituiert ;
- D-Prolin, ggf. in der β, γ oder δ Position substituiert ;
- N-alkylierte natürliche Aminosäuren, worin die Alkylgruppe eine Methyl-, Äthyl- oder Benzylgruppe ist;
- Azyklische dialkylierte Aminosäuren mit der folgenden Formel : 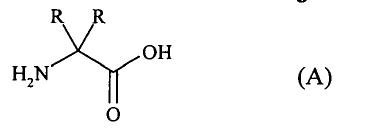 worin R H, Me, Et, Pr oder Bu bedeutet;
- Zyklische dialkylierte Aminosäuren mit der folgenden Formel : 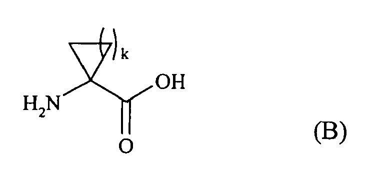 worin k 1, 2, 3 oder 4 bedeutet;

2. Verbindung nach Anspruch 1, **dadurch gekennzeichnet, daß** :
- Xₐ nicht ein Lysinrest ist.

3. Verbindung nach irgendwelcher Anspruch 1 - 5, die mit Biotin markiert wird.

4. Verbindung nach irgendwelcher Anspruch 1 - 3, **dadurch gekennzeichnet, daß** Y die folgende Formel (II) hat: 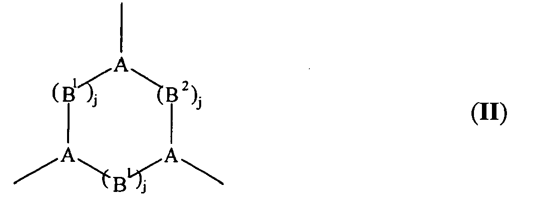 worin :
- j 0 oder 1 ist ;
- A ein Aminosäurerest oder ein Derivat eines Aminosäurerest bedeutet, der zwischen den folgenden in beliebiger Weise gewählt wird : 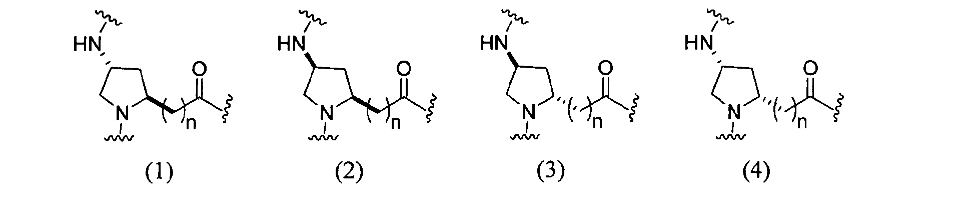 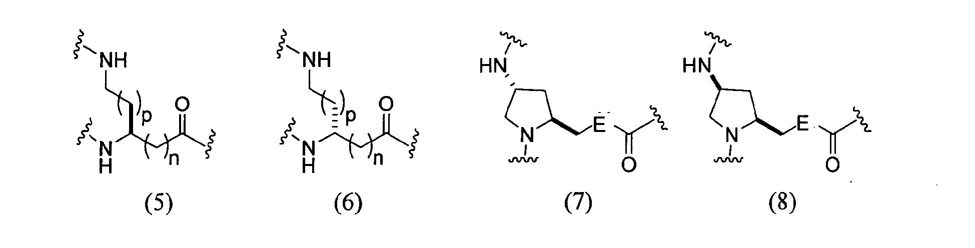 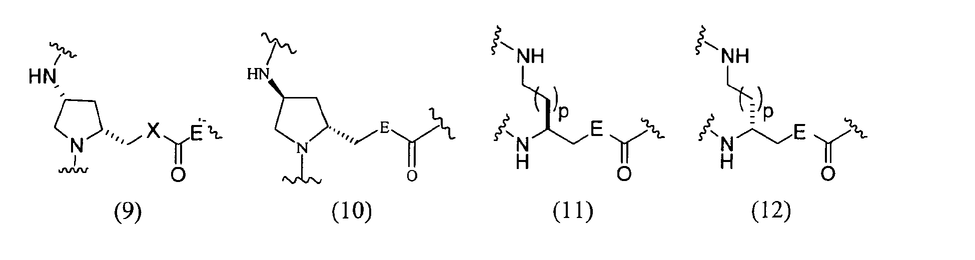
. worin n 0, 1, 2 oder 3 ist ;
. p 0, 1, 2 oder 3 ist;
. E NH oder O bedeutet ;
- B¹ ist ein Aminosäurerest oder ein Derivat eines Aminosäurerest, der zwischen den folgenden in beliebiger Weise gewählt wird : 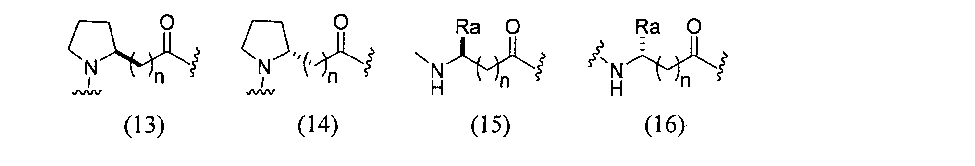 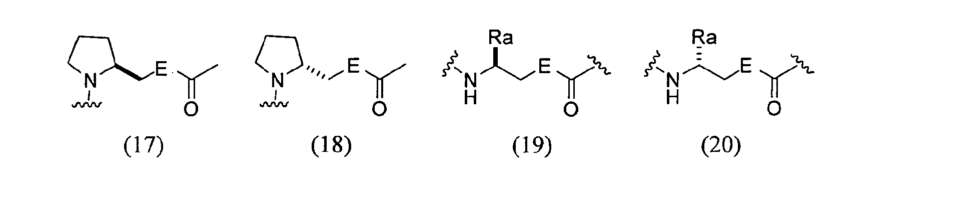
. worin n 0, 1, 2 oder 3 ist ;
. p 0, 1, 2 oder 3 ist ;
. E NH oder O bedeutet;
. Ra die Kette einer proteinogenischen, C1-C8 alkylierten Aminosäure ;
- B² kann gleich B¹ sein, oder kann in beliebiger Weise zwischen den folgenden Gruppen gewählt werden : 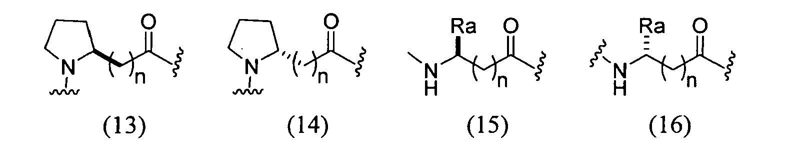 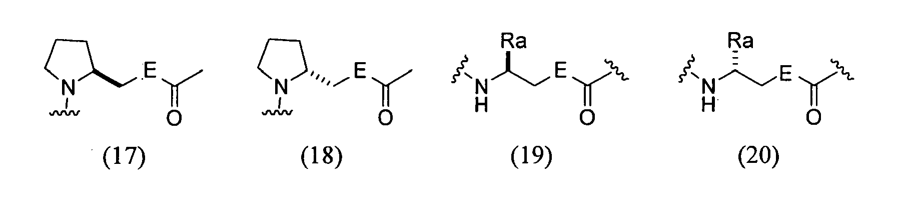 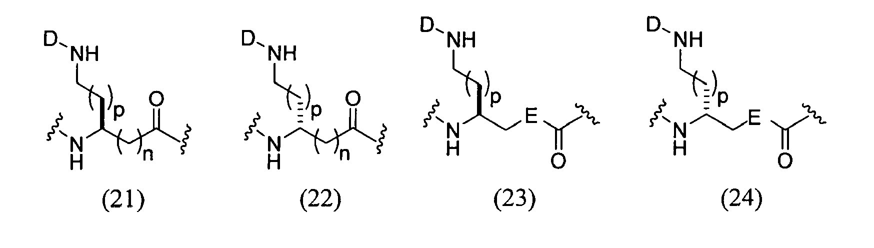
. worin n 0, 1, 2 oder 3 ist;
. p 0, 1, 2 oder 3 ist ;
. E NH oder O bedeutet;
. Ra die Kette einer proteinogenischen, C1-C8 alkylierten Aminosäure bedeutet ;
. D eine von den folgenden Gruppen bedeutet: (+)-Biotinyl-, (+)-Biotinyl-X_{g}-, HS-(CH₂)_{q}-CO-, Pys-S-(CH₂)_{q}-CO-, Npys-S-(CH₂)_{q}-CO-, HS-(CH₂)_{q}-CO-X_{g}-, Pys-S-(CH₂)_{q}-CO-X_{g}-, Npys-S-(CH₂)_{q}-CO-X_{g}-
q eine ganze Zahl zwischen 2 - 6 bedeutet ;
X_{g} dem Rest einer der folgenden Gruppen entspricht :
-NH₂-(CH₂)ₙ-COOH, worin n zwischen 1 - 10 liegt ;
-NH₂-(CH₂-CH₂-O)ₘ-CH₂CH₂COOH, worin m zwischen 3 - 6 liegt ;
8-amino-3,6-Dioxaoctansäure ;
Tranexamische Säure ;
N-methyl-tranexamische Säure ;
4(piperidin-4-yl)Butansäure ;
3(piperidin-4-yl)-Propionsäure ;
N-(4-aminobutyl)-Glycin ;
4-carboxymethyl-Piperazin ;
4-(4-aminophenyl)Butansäure ;
3-(4-aminophenyl)Propansäure ;
4-aminophenylessigsäure ;
4-(2-aminoethyl)-1-carboxymethyl-Piperazin ;
Trans-4-aminocyclohexancarbonsäure ;
Cis-4-aminocyclohexancarbonsäure ;
Cis-4-aminocyclohexanessigsäure ;
Trans-4-aminocyclohexanessigsäure ;
4-amino-1-carboxymethyl Piperidin ;
4-aminobenzoesäure ;
acide 4(2-aminoethoxy)Benzoesäure.

5. Verbindung nach irgendwelcher Anspruch 1 - 4, mit einer der folgenden Formeln : 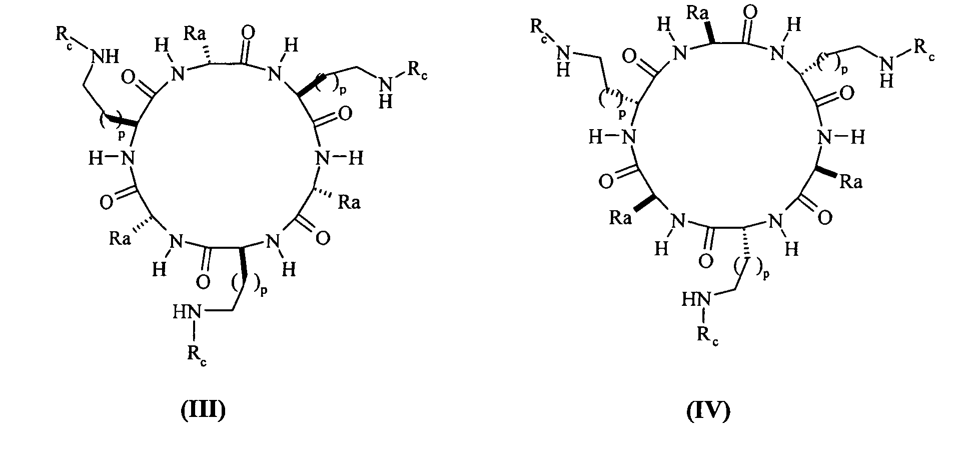 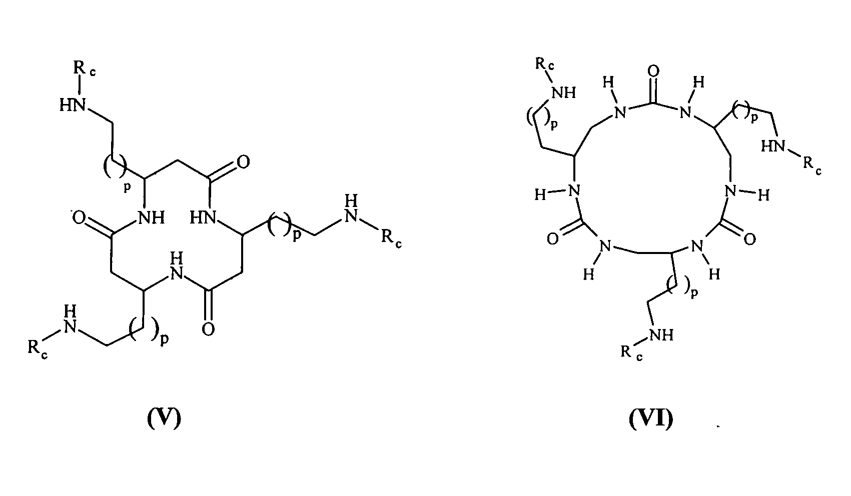 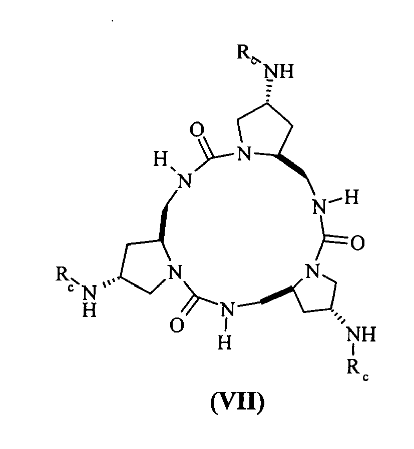 worin Ra, R_{c} und p wie in irgendwelcher Anspruch 1 - 4 definiert sind.

6. Verbindung nach irgendwelcher Anspruch 1 - 5, mit der folgenden Formel (III-a) :

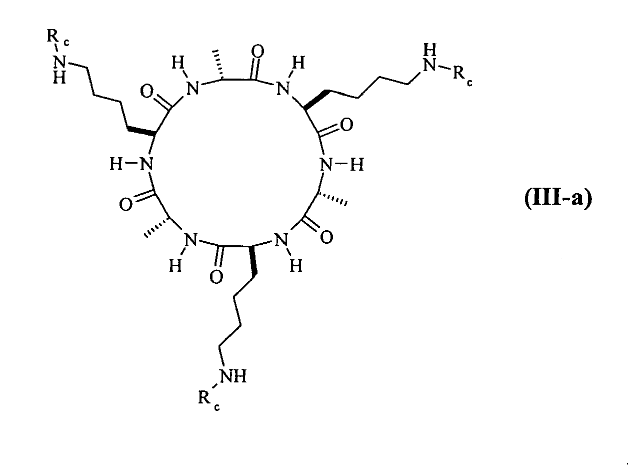

7. Verbindung nach irgendwelcher Anspruch 1 - 5, mit der folgenden Formel (V-a) :

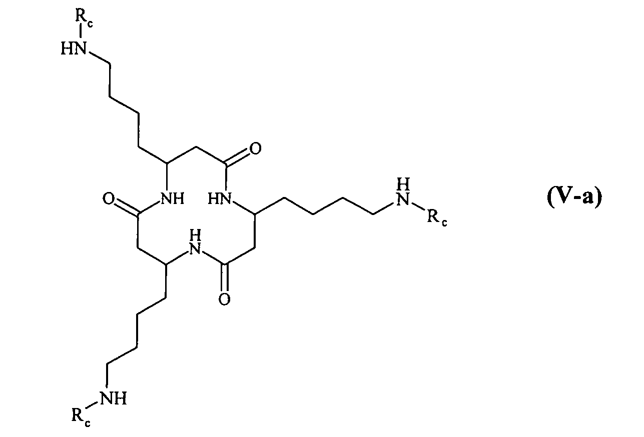

8. Verbindung nach irgendwelcher Anspruch 1 - 5, mit einer der folgenden Formeln : 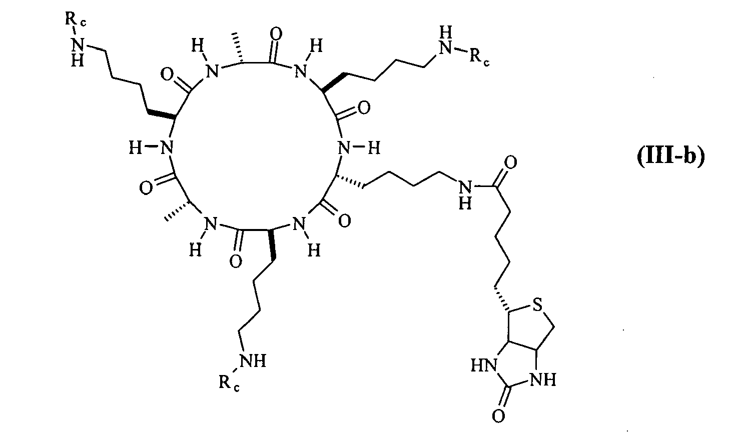 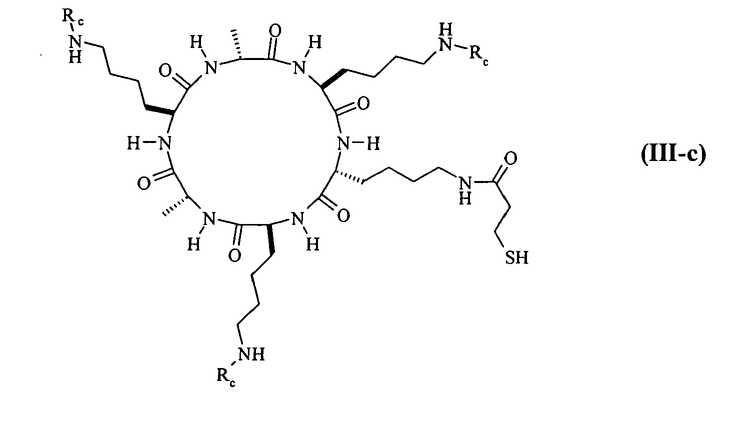 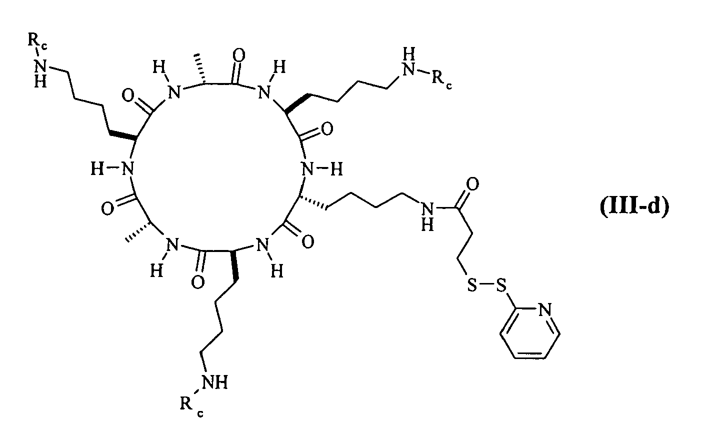 worin R_{c} wie in Anspruch 1 definiert ist.

9. Verbindung nach irgendwelcher Anspruch 1 - 8, worin R_{c} folgende Bedeutungen hat :
- H-Lys-(D)Pro-Tyr-Tyr-NH-(CH₂)₅-CO- oder
- Ahx-(D)Pro-Tyr-Tyr-NH-(CH₂)₅-CO- oder
- H-Lys-ψ(CH₂N)-(D)Pro-Tyr-Tyr-NH-(CH₂)₅-CO-, -ψ(CH₂N)-entsprechend einer methylene-amino-ähnliche pseudopeptidische Bindung.

10. Verbindung nach irgendwelcher Anspruch 1 - 7 und 9, mit einer der

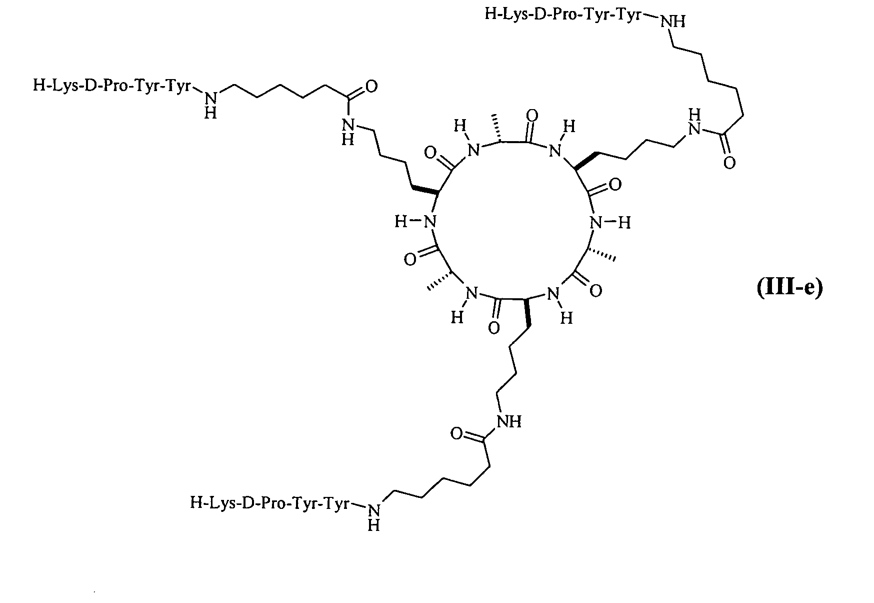

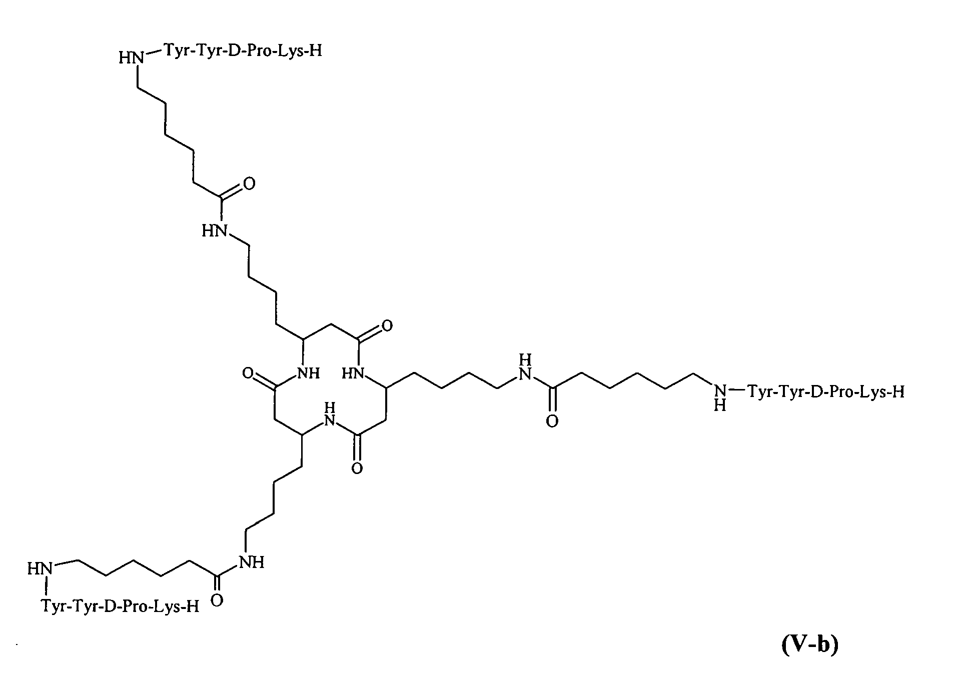

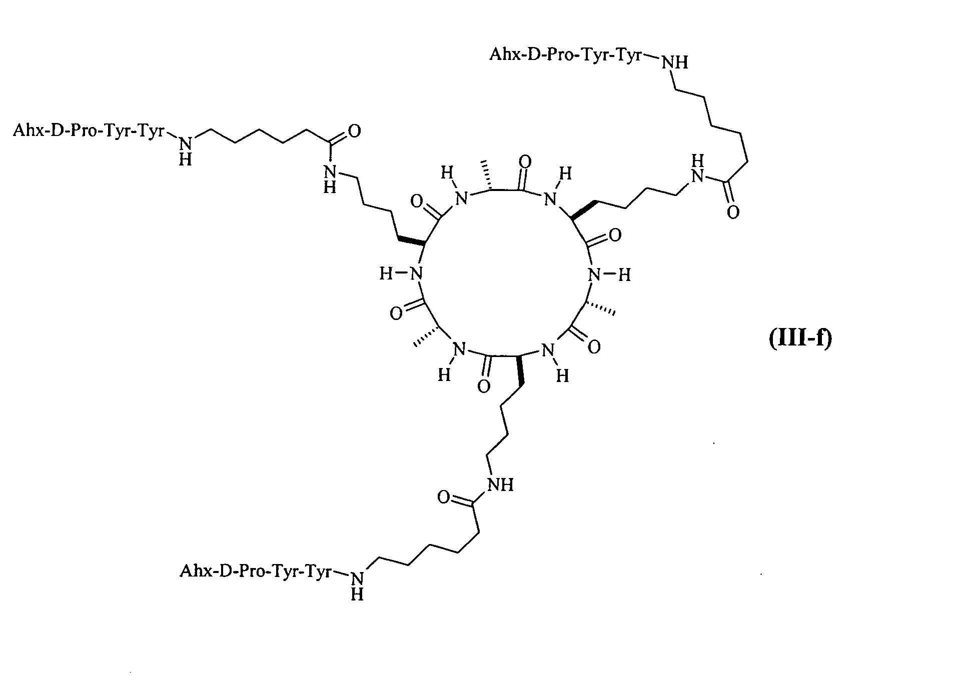

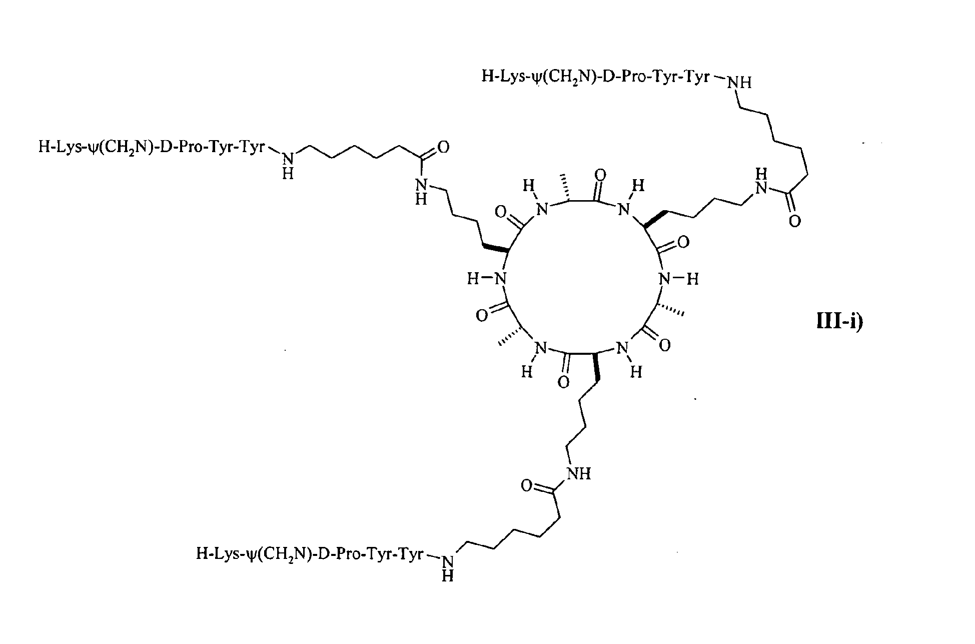

11. Verbindung nach irgendwelcher Anspruch 1 - 5, 8 und 9, mit einer der folgenden Formeln :

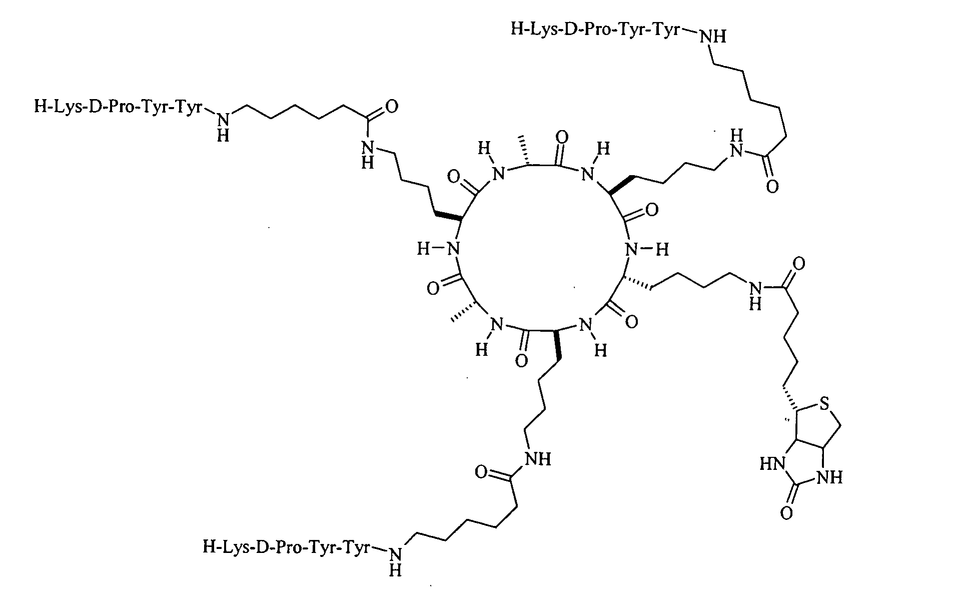

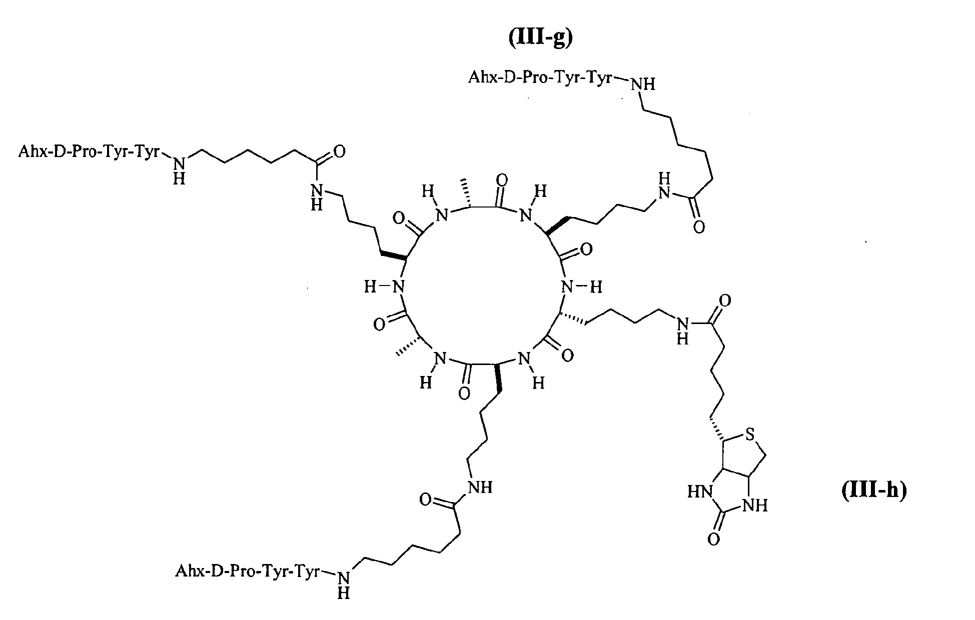

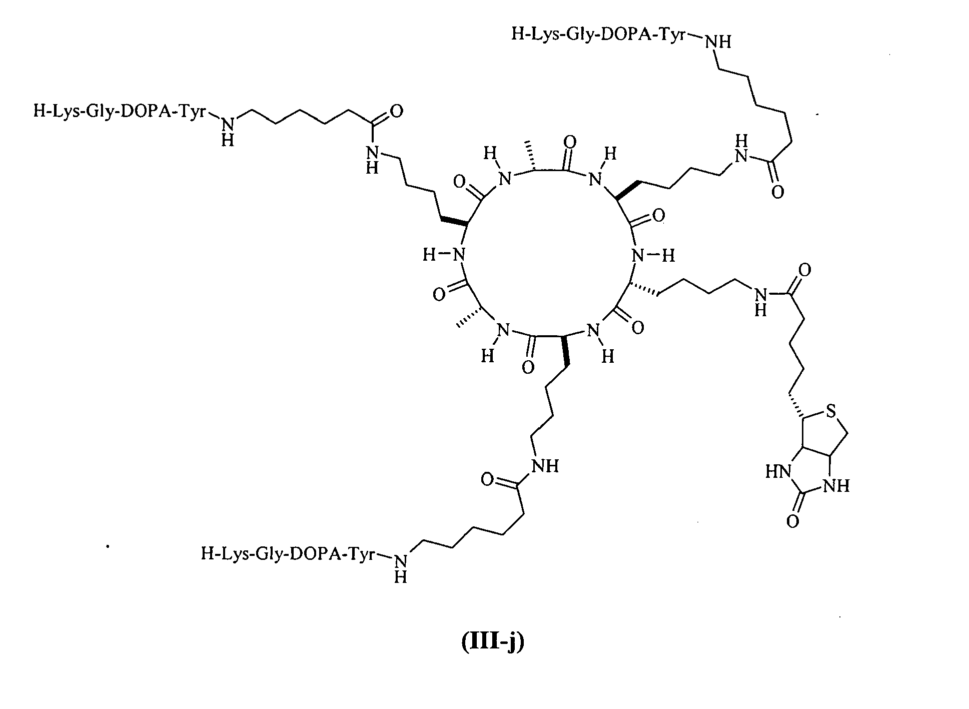

12. Pharmazeutische Zusammenstellung, **dadurch gekennzeichnet, daß** sie als Aktivsubstanz eine Verbindung nach irgendwelcher Anspruch 1 - 11, zusammen mit einem pharmazeutisch annehmbaren Träger enthält.

13. Vaccinale Zusammenstellung, **dadurch gekennzeichnet, daß** sie als Aktivsubstanz eine Verbindung nach irgendwelcher Anspruch 1 - 11, zusammen mit einem pharmazeutisch annehmbaren Adjuvans enthält.

14. Verwendung von Verbindungen nach irgendwelcher Anspruch 1 - 11, für die Herstellung eines Arzneimittel für die Behandlung von Pathologien, worin die Inhibition oder die Aktivierung der Immunantwort eine Rolle spielen.

15. Verwendung nach Anspruch 14, für die Herstellung eines Arzmittels für die Behandlung von Pathologien, worin die Inhibition der Immunantwort eine Rolle spielt, wie etwa in der Abstoßung eines Transplantats, in Allergien oder in autoimmune Krankheiten.

16. Verwendung nach Anspruch 14, für die Herstellung eines Arzmittels für die Behandlung von Pathologien, worin die Immunantwort ansteigt, wie etwa Krebse oder Infektionen durch Parasite, Bakterien, Viren oder unkonventionnelle infektiöse Agenzien wie etwa Prionen.

17. Verwendung von Verbindungen nach Anspruch 3, für die Herstellung von funktionalisierten Liganden, oder für die Reinigung von CD40.

18. Verfahren für die Herstellung auf festem Träger von einer Verbindung nach irgendwelcher Anspruch 1 - 11, worin dieses Verfahren **dadurch gekennzeichnet ist, daß** es die folgenden Schritte beinhaltet :
- die Bildung eines linearen Vorläufer von Y wie in Anspruch 1 definiert, worin dieser Vorläufer aus einer Verkettung von Aminosäuren, die eine wachsende Peptidkette bilden, besteht, worin diese Kette durch aufeinanderfolgende Kupplungszyklen zwischen N-geschützte Aminosäureresten und die Aminfunktion der wachsenden Peptidkette synthetisiert wird, und worin 3 Aminosäuren eine aminähnliche Gruppe Rₐ trägen, Rₐ wie in Anspruch 4 definiert ist, und dazwischen durch Deprotektion, worin der erste Aminosäurerest auf einem festen Träger aufhängt,
- der Ringschluß des obengenannten geschützten linearen Vorläufer von Y,
- die Spaltung der obengenannten Schutzgruppen, um die genannten geschützten Aminfunktionen zu befreien,
- die Kupplung der obengenannten befreiten Aminfunktionen mit einem Peptid, welches schon gebildet ist, oder in situ durch den sequenziellen Aufbau von den Aminosäureresten, die der wie in Anspruch 1 definierten R_{c}-Gruppe entsprechen, gebildet wird, und
- die Spaltung des Festträgermoleküls, und zwar nach der Beseitigung von allen anwesenden Schutzgruppen auf die funktionalisierten Seitenketten der R_{c}-Gruppe, um die Verbindung nach irgendwelcher Anspruch 1 -11 zu erhalten.
